# EUROPEAN PATENT APPLICATION

(11) **EP 4 321 531 A1**
(43) Date of publication of application: **14.02.2024**
(21) Application number: 21936107.8
(22) Date of filing: 05.04.2021
(51) Int. Cl.: C07K 16/08, A61P 35/00, A61K 39/00

(54) **VIRAL VECTOR-DERIVED TARGET PROTEIN FOR ANTICANCER THERAPY AND BINDING MOLECULE OR FRAGMENT THEREOF SPECIFICALLY BINDING THERETO**

(71) Applicant: Bionoxx Inc., Seongnam-si, Gyeonggi-do 13554 (KR)
(72) Inventor: HWANG, Tae-Ho, Yangsan-si, Gyeongsangnam-do 50612 (KR); CHO, Mong, Yangsan-si, Gyeongsangnam-do 50612 (KR)
(74) Representative: Dehns
(86) International application number: PCT/KR2021/004225
(87) International publication number: WO 2022/215762

(57) **Abstract**

The present invention relates to a target protein for viral vector-based anticancer therapy, and a binding molecule or a fragment thereof which specifically binds thereto. More specifically, the present invention relates to a conformational epitope of protein A56, and a binding molecule or a fragment thereof which specifically binds thereto. The A56-binding molecule or a fragment thereof, which binds to a conformational epitope of A56, according to the present invention forms a specific structural bond with A56 and shows high affinity thereto. The present inventors have found that in a case where a vaccinia virus-based oncolytic virus is administered, A56 is expressed on the cancer cell surface in various carcinomas. The A56-binding molecule or a fragment thereof according to the present invention effectively targets A56 that is specifically expressed on the cancer cell surface, which enables targeted therapy for cancer cells that have survived even infection with an oncolytic virus, thereby providing effective anticancer therapy.

## Description

### Technical Field

The present invention relates to a target protein for viral vector-based anticancer therapy, and a binding molecule or a fragment thereof which specifically binds thereto. More specifically, the present invention relates to a conformational epitope of protein A56, and a binding molecule or a fragment thereof which specifically binds thereto.

### Background Art

Cancer is also called a tumor, and refers to cells that have grown abnormally due to autonomous overgrowth of body tissue. Cancer incidence continues to increase due to aging population, increased smoking population, increase in alcohol consumption, westernized eating habits, and environmental pollution in modern society.

Methods for treating cancer include surgery, radiation therapy, chemotherapy, and the like. Specifically, surgery is a therapeutic method that removes cancerous tissue from the body, and is very effective for early cancer or cancer in which lesions are restricted to a certain location. However, it is difficult to remove cancer that has invaded tissue around the lesion or has metastasized to the lymph node, and such cancer has a high probability of recurrence. In this case, radiation therapy or chemotherapy is used in combination with surgery. Radiation therapy or chemotherapy is used mainly for the treatment of advanced or terminal cancer; however, this therapy also affects normal cells, and thus causes serious adverse effects.

Meanwhile, with full-scale use of gene recombination techniques, clinical studies using oncolytic viruses with increased tumor selectivity and anticancer efficacy have been initiated. The first recombinant oncolytic virus reported in literature was a herpes simplex virus. Since then, studies on oncolysis using other viruses have been actively conducted.

Among them, studies on thymidine kinase (TK) gene-deficient vaccinia viruses obtained by using a vaccinia virus have been conducted; however, despite clinical usefulness, the viruses have a limit in maximizing their clinical effect due to a narrow therapeutic window. For the TK-deficient vaccinia virus, the narrow therapeutic window means that a high viral dose has great clinical efficacy but may entail clinical risks due to toxicity of the virus. In order to overcome this problem, studies have been conducted to cause increased therapeutic efficacy and decreased toxicity in such viruses by deleting a gene region encoding a non-essential protein such as protein A56 along with deletion of TK gene (Izmailyan R, Chang W. Journal of virology. 2008 Oct;82(20): 10079-87).

Recently, immune cell therapy has been actively studied as a cancer therapeutic method. The immune cell therapy is different from existing therapeutic methods in that it uses the patient's immune cells to kill cancer cells. Specifically, the immune cell therapy is a method in which immune cells are obtained from a patient, activated to specifically attack proliferating cells or cancer cells, and then returned back to the patient's body; and this method maximizes an anticancer effect while minimizing drug-induced adverse effects.

Over the past 5 years, immune checkpoint inhibitors have been actively studied for immunotherapy for treating cancer. In particular, inhibitors against immune checkpoints, such as cytotoxic T-lymphocyte-associated protein 4 (CTLA-4), programmed cell death protein 1 (PD-1), and PD-L1, have been studied. Immune checkpoint inhibitors, such as ipilimumab (anti-CTLA-4), nivolumab (anti-PD-1), and pembrolizumab (anti-PD-1), have been approved by regulatory agencies for the treatment of several types of cancer. However, the immune checkpoint inhibitors are limitedly used for the treatment of patients with certain types of cancer, such as melanoma, lung cancer, head and neck cancer, kidney cancer, and bladder cancer.

In addition, chimeric antigen receptor-expressing T cells (hereinafter, CAR-T cells) showed excellent therapeutic efficacy against various types of blood cancer. However, for solid cancer, almost no clinically successful cases with CAR-T cells have been reported. The reasons related thereto include difficulty in trafficking and infiltration of CAR-T cells into a tumor, persistence and proliferation issues of CAR-T cells, and a limitation of CAR-T cells whose therapeutic effect is not exerted due to hostile tumor microenvironment; however, the greatest limitation is that serious safety problems arise due to the absence of a suitable target antigen that is specifically expressed in tumors. In fact, most of the target antigens used in clinical development of CAR-T cells for solid cancer are overexpressed in tumors and also expressed at low levels in normal cells. For this reason, cases have been reported that CAR-T cells also bind to normal cells and cause serious toxicity problems. To overcome this on-target off-tumor toxicity problem, a target antigen is required which satisfies conditions of tumor specificity, high and uniform coverage, and expression stability. However, there is currently almost no solid cancer target antigen that satisfies all three conditions. Therefore, there is a need for continuous research and development on cancer cell-targeting methods capable of targeting solid cancer in a safe and effective manner.

### [Prior Art Document]

### [Non-Patent Document]

(Non-Patent Document 1) Izmailyan R, Chang W. Journal of virology. 2008 Oct;82(20): 10079-87

### Disclosure of Invention

### Technical Problem

Accordingly, the present inventors have studied to develop a method for safely and effectively targeting or treating cancer, in particular, solid cancer. As a result, the present inventors have found that for various carcinomas, in a case where cancer cells are treated by injection of a vector that contains a nucleic acid encoding vaccinia virus protein A56 or a fragment thereof, the protein A56 is expressed on the cancer cell surface in a tumor-specific and stable manner. In addition, for a plurality of antibodies binding to the protein A56 or a fragment thereof, the present inventors have analyzed their binding to A56. As a result, the present inventors have found that the antibodies cross-inhibit binding to A56 despite their epitope sequences being not completely identical, which is unique. Then, the present inventors have identified a conformational epitope that determines binding to A56, and have found that binding molecules binding to such a conformational epitope exert an excellent effect in decreasing burden of cancer cells, thereby completing the present invention.

### Solution to Problem

In an aspect of the present invention, there is provided is a binding molecule or a fragment thereof, which specifically binds to a conformational epitope of A56 or a fragment thereof, wherein the conformational epitope includes a basic or nucleophilic amino acid present in a region from amino acids at positions 60 to 63 in an amino acid sequence of protein A56 represented by SEQ ID NO: 1, and further includes (i) a nucleophilic amino acid present in a region from amino acids at positions 44 to 50 in the amino acid sequence of protein A56, (ii) a nucleophilic amino acid present in a region from amino acids at positions 53 to 59 in the amino acid sequence of protein A56, (iii) a nucleophilic amino acid present in a region from amino acids at positions 85 to 90 in the amino acid sequence of protein A56, or (iv) a basic or nucleophilic amino acid present in a region from amino acids at positions 91 to 94 in the amino acid sequence of protein A56.

In another aspect of the invention, there is provided a method for decreasing burden of cancer cells in a subject, comprising a step of administering, to the subject, the binding molecule or a fragment thereof.

In yet another aspect of the present invention, there is provided a pharmaceutical composition for preventing or treating cancer, comprising the binding molecule or a fragment thereof.

In still yet another aspect of the present invention, there is provided a kit for preventing or treating cancer, comprising the binding molecule or a fragment thereof and an oncolytic virus.

In still yet another aspect of the invention, there is provided a use of the binding molecule or fragment thereof for the treatment of cancer.

### Advantageous Effects of Invention

The A56-binding molecule or a fragment thereof, which binds to a conformational epitope of A56, according to the present invention forms a specific structural bond with A56 and shows high affinity thereto. The present inventors have found that in a case where a vaccinia virus-based oncolytic virus is administered, A56 is expressed on the cancer cell surface in various carcinomas. The A56-binding molecule or a fragment thereof according to the present invention effectively targets A56 that is specifically expressed on the cancer cell surface, which enables targeted therapy for cancer cells that have survived even infection with an oncolytic virus, thereby providing effective anticancer therapy. For example, the A56-binding molecule according to the present invention itself enables effective targeting of cancer cells that have survived even infection with an oncolytic virus. In addition, in a case of being used in cancer immunotherapy such as CAR-T cell therapy, the A56-binding molecule exhibits increased activation and proliferation capacity specifically for cancer cells expressing A56 and exerts an excellent cytotoxic effect thereon. Thus, the A56-binding molecule can provide effective anticancer therapy against cancer cells expressing A56. Cancer immunotherapy using the A56-binding molecule according to the present invention is preferably used in combination with an oncolytic virus, and may be additionally used in combination with a drug capable of enhancing an anticancer effect of the oncolytic virus (for example, hydroxyurea, chemotherapeutic agents for regulating lymphocyte removal (for example, cyclophosphamide and fludarabine), or immunotherapeutic agents). As used herein, unless otherwise indicated, the expression "in combination with" includes both the administration of therapies/agents in question which is performed at time intervals in any order and the administration thereof which is performed at the same time.

### Brief Description of Drawings

FIG. 1 illustrates results obtained by subjecting a human lung cancer cell line (A549), a human colorectal cancer cell line (HCT-116), or a human melanoma cell line (SK-MEL-5) to infection with an oncolytic virus that contains a nucleic acid encoding protein A56, and then identifying, with immunofluorescence staining, presence or absence of expression of the protein A56 on the cell surface of each cell line.
FIG. 2 illustrates results obtained by subjecting a human colorectal cancer cell line (HCT-116) to infection with an oncolytic virus that contains a nucleic acid encoding protein A56, and then identifying, with immunofluorescence staining, presence or absence of expression of the protein A56 on the cell surface of the infected HCT-116 cell line.
FIG. 3 illustrates results obtained by subjecting human colorectal cancer cell line (HCT-116)-transplanted mice to intraperitoneal administration of an oncolytic virus that contains a nucleic acid encoding protein A56, and then identifying expression of the protein A56 on the surface of each tissue.
FIG. 4 illustrates results obtained by subjecting normal rabbits to intravascular administration of an oncolytic virus that contains a nucleic acid encoding protein A56, and then identifying expression of the protein A56 on the surface of each tissue.
FIG. 5 illustrates results obtained by subjecting mouse renal cancer cell line (Renca)-transplanted mice to intratumoral administration of an oncolytic virus that contains a nucleic acid encoding protein A56, and then identifying expression of the protein A56 on the tumor tissue surface on days 7, 10, and 14.
FIG. 6 illustrates results obtained by subjecting mouse renal cancer cell line (Renca)-transplanted mice to intratumoral administration of hydroxyurea and an oncolytic virus that contains a nucleic acid encoding protein A56, and then identifying expression of the protein A56 on the tumor tissue surface on days 7, 10, and 14.
FIG. 7 illustrates results obtained by subjecting the mouse renal cancer cell line (Renca)-transplanted mice to secondary administration of the hydroxyurea and the oncolytic virus that contains a nucleic acid encoding protein A56, and then identifying expression of the protein A56 on the tumor tissue surface on days 21, 24, and 28.
FIG. 8 illustrates a diagram showing an embodiment for protein A56 and fragments thereof.
FIG. 9 illustrates results obtained by identifying whether the protein A56 and fragments thereof are expressed in cells and on the cell surface.
FIG. 10 illustrates results obtained by identifying expression levels of Fc-fused A56 fusion protein.
FIGS. 11 to 16 illustrate results obtained by measuring affinity between anti-A56 antibodies produced in an embodiment of the present invention and A56.
FIG. 17A illustrates results obtained by identifying productivity of the antibodies A56-01A02 to A56-02B06.
FIG. 17B illustrates a graph showing productivity of the antibodies A56-01A02 to A56-02B06.
FIG. 17C illustrates results obtained by identifying productivity of the antibodies A56-02D04 to A56-59E12.
FIGS. 18 to 34 illustrate results obtained by purifying the anti-A56 antibodies produced in an embodiment of the present invention, and then identifying these antibodies through SDS-PAGE.
FIG. 35 illustrates results obtained by comparing homology of amino acid sequences of protein A56s depending on vaccinia virus strains.
FIG. 36 illustrates results obtained by measuring binding capacity of ten anti-A56 antibodies (Ab18, Ab19, Ab01, Ab13, Ab14, Ab08, Ab03, Ab51, Ab55, Ab16) to protein A56.
FIG. 37 illustrates a photograph taken after reacting protein A56 with a commercial anti-A56 antibody, and then performing Western blotting, which was used as a control to determine whether the anti-A56 antibodies have a conformational epitope.
FIG. 38 illustrates photographs taken after reacting protein A56 with each of ten anti-A56 antibodies (Ab18, Ab19, Ab01, Ab13, Ab14, Ab08, Ab03, Ab51, Ab55, Ab16), and then performing Western blotting.
FIG. 39 illustrates results obtained by analyzing an antigen-antibody binding complex (A56-C-His/Ab13) with high-mass MALDI.
FIG. 40 illustrates results obtained by analyzing an antigen-antibody binding complex (A56-C-His/Ab16) with high-mass MALDI.
FIG. 41 illustrates results obtained by analyzing an antigen-antibody binding complex (A56-C-His/Ab18) with high-mass MALDI.
FIG. 42 illustrates results obtained by analyzing an antigen-antibody binding complex (A56-C-His/Ab01) with high-mass MALDI.
FIG. 43 illustrates results obtained by analyzing an antigen-antibody binding complex (A56-C-His/Ab19) with high-mass MALDI.
FIG. 44 illustrates results obtained by treating protein A56 with five proteolytic enzymes (trypsin, chymotrypsin, ASP-N, elastase, and thermolysin), and then performing LTQ-Orbitrap MS (mass spectrometry) analysis.
FIG. 45 illustrates diagrams showing epitope positions on A56-C-His, to which an anti-A56 antibody (Ab13) binds, and modeled structures of the protein A56-C-His. The amino acid numbering in A56 as shown here is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).
FIG. 46 illustrates diagrams showing epitope positions on A56-C-His, to which an anti-A56 antibody (Ab16) binds, and modeled structures of the protein A56-C-His. The amino acid numbering in A56 as shown here is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).
FIG. 47 illustrates diagrams showing epitope positions on A56-C-His, to which an anti-A56 antibody (Ab18) binds, and modeled structures of the protein A56-C-His. The amino acid numbering in A56 as shown here is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).
FIG. 48 illustrates diagrams showing epitope positions on A56-C-His, to which an anti-A56 antibody (Ab01) binds, and modeled structures of the protein A56-C-His. The amino acid numbering in A56 as shown here is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).
FIG. 49 illustrates diagrams showing an epitope position on A56-C-His, to which an anti-A56 antibody (Ab19) binds, and modeled structures of the protein A56-C-His. The amino acid numbering in A56 as shown here is made based on a sequence obtained by excluding N-terminal amino acids 1-16 from the amino acid sequence represented by SEQ ID NO: 1 (for example, isoleucine at position 40 corresponds to isoleucine at position 56 in the amino acid sequence represented by SEQ ID NO: 1).
FIG. 50 illustrates a diagram obtained by summarizing results of epitope mapping of five antibodies (Ab13, Ab16, Ab18, Ab01, Ab19) to protein A56. The amino acid numbering in A56 as shown here is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).
FIG. 51 illustrates a diagram obtained by modeling protein A56 into a 3D protein structure, and then analyzing the structure with iCn3D.
FIG. 52 illustrates diagrams showing paratope positions on an anti-A56 antibody (Ab13) and a modeled structure formed by binding between the antibody and protein A56-C-His.
FIG. 53 illustrates diagrams showing paratope positions on an anti-A56 antibody (Ab16) and a modeled structure formed by binding between the antibody and protein A56-C-His.
FIG. 54 illustrates diagrams showing paratope positions on an anti-A56 antibody (Ab18) and a modeled structure formed by binding between the antibody and protein A56-C-His.
FIG. 55 illustrates diagrams showing paratope positions on an anti-A56 antibody (Ab01) and a modeled structure formed by binding between the antibody and protein A56-C-His.
FIG. 56 illustrates diagrams showing paratope positions on an anti-A56 antibody (Ab19) and a modeled structure formed by binding between the antibody and protein A56-C-His.
FIG. 57A illustrates a view showing the formation of a hydrogen bond between K91 in protein A56 and T52 of the heavy chain in Ab13, and the measured binding distance.
FIG. 57B illustrates a view showing the formation of a hydrogen bond between S62 in protein A56 and K57 of the heavy chain in Ab13, and the measured binding distance.
FIG. 58A illustrates results obtained by allowing primary antibodies (SA2038, Ab13, A56-02A02) to be bound to an A56-C-His antigen-immobilized biosensor (NTA) to a saturated state, performing treatment with nine antibodies, and then analyzing, with a surface plasmon resonance (SPR) method, whether additional binding of such antibodies thereto occurs.
FIG. 58B illustrates results obtained by measuring binding affinity (K_{D}) between protein A56 and Ab13 with OCTET (SPR) and ELISA.
FIG. 59 illustrates a view showing the binding between protein A56 and Ab16, and the measured distance thereof.
FIG. 60A illustrates results obtained by allowing primary antibodies (SA2041, Ab16, A56-02B08) to be bound to an A56-C-His antigen-immobilized biosensor (NTA) to a saturated state, performing treatment with nine antibodies, and then analyzing, with an SPR method, whether additional binding of such antibodies thereto occurs.
FIG. 60B illustrates results obtained by measuring binding affinity (K_{D}) between protein A56 and Ab16 with OCTET (SPR) and ELISA.
FIG. 61A illustrates a view showing the formation of a hydrogen bond between S62 in protein A56 and R98 of the heavy chain in Ab18, and the measured binding distance.
FIG. 61B illustrates a view showing the formation of a hydrogen bond between K91 in protein A56 and Y32 of the light chain in Ab18, and the measured binding distance.
FIG. 62A illustrates results obtained by allowing primary antibodies (SA2043, Ab18, A56-02C06) to be bound to an A56-C-His antigen-immobilized biosensor (NTA) to a saturated state, performing treatment with nine antibodies, and then analyzing, with an SPR method, whether additional binding of such antibodies thereto occurs.
FIG. 62B illustrates results obtained by measuring binding affinity (K_{D}) between protein A56 and Ab18 with OCTET (SPR) and ELISA.
FIG. 63 illustrates a view showing the binding between protein A56 and Ab01, and the measured distance thereof.
FIG. 64A illustrates results obtained by allowing primary antibodies (SA2026, Ab01, A56-01A02) to be bound to an A56-C-His antigen-immobilized biosensor (NTA) to a saturated state, performing treatment with nine antibodies, and then analyzing, with an SPR method, whether additional binding of such antibodies thereto occurs.
FIG. 64B illustrates results obtained by measuring binding affinity (K_{D}) between protein A56 and Ab01 with OCTET (SPR) and ELISA.
FIG. 65 illustrates a view showing the binding between protein A56 and Ab19, and the measured distance thereof.
FIG. 66A illustrates results obtained by allowing primary antibodies (SA2044, Ab19, A56-02C07) to be bound to an A56-C-His antigen-immobilized biosensor (NTA) to a saturated state, performing treatment with nine antibodies, and then analyzing, with an SPR method, whether additional binding of such antibodies thereto occurs.
FIG. 66B illustrates results obtained by measuring binding affinity (K_{D}) between protein A56 and Ab19 with OCTET (SPR) and ELISA.
FIG. 67 illustrates a schematic diagram of a structure of the chimeric antigen receptor constructed in an embodiment of the present invention.
FIG. 68 illustrates results obtained by comparing the cytotoxicity observed in a case where HeLa cell line is subjected to administration of five types of CAR-T cells alone or in combination with an oncolytic virus (OTS-412).
FIG. 69 illustrates results obtained by comparing the cytotoxicity observed in a case where NCI-H522 cell line is subjected to administration of five types of CAR-T cells alone or in combination with an oncolytic virus (OTS-412).
FIG. 70 illustrates results obtained by comparing the cytotoxicity observed in a case where HCT-116 cell line is subjected to administration of five types of CAR-T cells alone or in combination with an oncolytic virus (OTS-412).
FIG. 71 illustrates results obtained by measuring, with flow cytometry (FACS), the transduction efficiency of five types of CAR-T cells.
FIG. 72 illustrates results showing the cytotoxicity observed in a case where A549 cell line or HCT-116 cell line is subjected to administration of UTD, Ab16 CAR-T cells, or Ab18 CAR-T cells alone or in combination with an oncolytic virus (OTS-412).
FIG. 73 illustrates photographs taken after subjecting HCT-116 cell line infected with an oncolytic virus (OTS-412) to administration of UTD and five types of CAR-T cells (Ab13 CAR-T, Ab16 CAR-T, Ab18 CAR-T, Ab01 CAR-T, Ab19 CAR-T), and then performing staining of the dead cells.
FIG. 74 illustrates photographs taken after subjecting HCT-116 cell line infected with an oncolytic virus (WOTS-418) to administration of UTD and five types of CAR-T cells (Ab13 CAR-T, Ab16 CAR-T, Ab18 CAR-T, Ab01 CAR-T, Ab19 CAR-T), and then performing staining of the dead cells.

### Best Mode for Carrying out the Invention

Hereinafter, the present invention will be described in detail.

In an aspect of the present invention, there is provided is a binding molecule or a fragment thereof, which specifically binds to a conformational epitope of A56 or a fragment thereof, wherein the conformational epitope includes a basic or nucleophilic amino acid present in a region from amino acids at positions 60 to 63 in an amino acid sequence of protein A56 represented by SEQ ID NO: 1, and further includes (i) a nucleophilic amino acid present in a region from amino acids at positions 44 to 50 in the amino acid sequence of protein A56, (ii) a nucleophilic amino acid present in a region from amino acids at positions 53 to 59 in the amino acid sequence of protein A56, (iii) a nucleophilic amino acid present in a region from amino acids at positions 85 to 90 in the amino acid sequence of protein A56, or (iv) a basic or nucleophilic amino acid present in a region from amino acids at positions 91 to 94 in the amino acid sequence of protein A56.

As used herein, the term "A56" refers to a protein translated from a gene (for example, GeneID: 3707652) represented by *A56, A56R,* or *HA,* which is encoded in the gene of a poxviridae family virus after a host cell is infected with the virus. The protein A56 or fragment thereof may include any one amino acid sequence selected from SEQ ID NOs: 1 to 15. The nucleic acid encoding the protein A56 or a fragment thereof may include any one nucleotide sequence selected from SEQ ID NOs: 16 to 31. In the present invention, the protein A56 may be used interchangeably with "UTTA".

Specifically, the protein A56 may be wild-type protein A56 or a variant thereof. The wild-type protein A56 may have an amino acid sequence represented by SEQ ID NO: 1 or 12. In addition, the nucleic acid encoding the wild-type protein A56 may be a nucleotide sequence represented by SEQ ID NO: 16 or 28.

In addition, the protein A56 variant may have undergone substitution, deletion, or addition of one or more amino acids as long as the variant can be located on the cancer cell surface like the protein A56. The nucleotide sequence encoding the protein A56 variant may be a nucleotide sequence that encodes an amino acid sequence having a sequence homology of at least 60%, at least 70%, at least 80%, or at least 90% to an amino acid sequence represented by SEQ ID NO: 1 or 15, and may be most preferably a nucleotide sequence that encodes an amino acid sequence having a sequence homology of at least 95% thereto. Specifically, the protein A56 variant may include an amino acid sequence represented by SEQ ID NO: 13 or 15. The nucleic acid encoding the protein A56 variant may include a nucleotide sequence represented by SEQ ID NO: 29 or 31.

The protein A56 fragment may be a polypeptide including any one amino acid sequence selected from SEQ ID NOs: 2 to 14. In addition, the nucleic acid encoding the fragment may be a nucleotide sequence encoding a polypeptide including any one amino acid sequence selected from SEQ ID NOs: 2 to 14. Specifically, the nucleotide sequence encoding the polypeptide including any one amino acid sequence selected from SEQ ID NOs: 2 to 14 may be represented by SEQ ID NOs: 17 to 31, respectively, in the order mentioned.

In addition, the nucleotide sequence encoding the protein A56 fragment may be a nucleotide sequence that encodes an amino acid sequence having a sequence homology of at least 60%, at least 70%, at least 80%, or at least 90% to any one amino acid sequence selected from SEQ ID Nos: 2 to 14, and may be most preferably a nucleotide sequence that encodes an amino acid sequence having a sequence homology of at least 95% thereto.

The binding molecule according to the present invention refers to a biological molecule having capacity to specifically bind to A56, and representative examples thereof may include an antibody or a chimeric antigen receptor. More specifically, the binding molecule according to the present invention can bind specifically to A56 exposed on the cancer cell surface, and this capacity enables anticancer therapy of cancer cells, in particular, effective targeting of cancer cells, which have survived even infection with an oncolytic virus, for secondary anticancer therapy. In addition, the binding molecule itself exerts an excellent cytotoxic effect in a case of being used in cancer immunotherapy such as CAR-T cell therapy, and thus enables effective treatment of cancer.

The conformational epitope may include adjacent amino acids or amino acids that are not adjacent due to three-dimensional folding of a protein. Specifically, the conformational epitope may include at least 2, 5, 8, 10, or 11 amino acids in a three-dimensional structure of an independent space.

Some amino acid residues of an antigen involved in antigen/antibody binding are called an epitope, and a specific part of an antibody, which recognizes an epitope, is called a paratope. Depending on how to bind a paratope, the epitope is divided largely into a linear epitope consisting of a sequence of about 4 to about 12 consecutive amino acids and a conformational epitope consisting of a sequence of non-consecutive amino acids. For the conformational epitope, antigen/antibody binding occurs three-dimensionally in a folding structure. Thus, the conformational epitope has a relatively wide and complex range of amino acid residues involved in the binding. Therefore, even if a structure of an antigen or a partial sequence thereof is known, it is difficult to predict or mimic a monoclonal antibody that three-dimensionally binds to the antigen. Whether an epitope of an antigen is linear or conformational can be identified, through Western blot analysis, by subjecting the antigen to denaturation, and then allowing an antibody to bind thereto. For the linear epitope, an antibody can bind thereto even if a sample (antigen) is denatured; however, for the conformational epitope, it is difficult or impossible for an antibody to three-dimensionally bind thereto in a case where an antigen is denatured, and thus a band corresponding to the expected molecular weight is not detected.

The conformational epitope may include a basic or nucleophilic amino acid present in a region from amino acids at positions 60 to 63 in an amino acid sequence of protein A56 represented by SEQ ID NO: 1, and may further include (i) a nucleophilic amino acid present in a region from amino acids at positions 44 to 50 in the amino acid sequence of protein A56, (ii) a nucleophilic amino acid present in a region from amino acids at positions 53 to 59 in the amino acid sequence of protein A56, (iii) a nucleophilic amino acid present in a region from amino acids at positions 85 to 90 in the amino acid sequence of protein A56, or (iv) a basic or nucleophilic amino acid present in a region from amino acids at positions 91 to 94 in the amino acid sequence of protein A56.

The protein A56 represented by SEQ ID NO: 1 may preferably take the following secondary structural configuration consisting of a total of 8 sheets (green), 4 helices (red), and 7 loops (blue) (however, the structural configuration does not have to be like this):
a region from amino acid at position 41 to amino acid at position 43: Helices 1 and 2
a region from amino acid at position 44 to amino acid at position 50: Sheet 3
a region from amino acid at position 51 to amino acid at position 52: loop
a region from amino acid at position 53 to amino acid at position 59: Sheet 4
a region from amino acid at position 60 to amino acid at position 63: loop
a region from amino acid at position 64 to amino acid at position 66: Sheet 5
a region from amino acid at position 67 to amino acid at position 74: loop
a region from amino acid at position 75 to amino acid at position 78: Sheet 6
a region from amino acid at position79 to amino acid at position 80: loop
a region from amino acid at position 81 to amino acid at position 83: Helix 3
an amino acid at position 84: loop
a region from amino acid at position 85 to amino acid at position 90: Sheet 7
a region from amino acid at position 91 to amino acid at position 94: loop
a region from amino acid at position 95 to amino acid at position 97: Helix 4
an amino acid at position 98: loop
a region from amino acid at position 99 to amino acid at position105: Sheet 8

### (Sheets 1 and 2 not shown)

The nucleophilic amino acid refers to an amino acid having a nucleophilic side chain susceptible to a covalent reaction with an electrophilic side chain, and may be, for example, cysteine (C), lysine (K), serine (S), threonine (T), or tyrosine (Y). The basic amino acid refers to an amino acid whose side chain shows basicity and whose side chain has (+) charge in a case where the amino acid is dissociated in a neutral pH region, and may be, for example, histidine (H), arginine (R), or lysine (K).

The conformational epitope of A56 according to the present invention may include a basic or nucleophilic amino acid present in a region from amino acids at positions 60 to 63 in an amino acid sequence of protein A56 represented by SEQ ID NO: 1, and may include (iii) a nucleophilic amino acid present in a region from amino acids at positions 85 to 90 in the amino acid sequence of protein A56, and (iv) a basic or nucleophilic amino acid present in a region from amino acids at positions 91 to 94 in the amino acid sequence of protein A56.

Specifically, the conformational epitope may include serine at position 62 (S62), which is a nucleophilic amino acid, tyrosine at position 66 (Y66), threonine at position 87 (T87), lysine at position 91 (K91), and serine at position 92 (S92), in an amino acid sequence of protein A56 represented by SEQ ID NO: 1. More specifically, the conformational epitope may include amino acids at positions 54 (S54), 62 (S62), 66 (Y66), 86 (T86), 87 (T87), 91 (K91), 92 (S92), 94 (T94), 96 (arginine, R96), 100 (T100), and 101 (Y101) in an amino acid sequence of protein A56 represented by SEQ ID NO: 1. In addition, the conformational epitope may include amino acids at positions 62 (S62), 66 (Y66), 71 (T71), 72 (K72), 76 (S76), 87 (T87), 91 (K91), and 92 (S92) in an amino acid sequence of protein A56 represented by SEQ ID NO: 1.

In addition, the conformational epitope of A56 according to the present invention may include a basic or nucleophilic amino acid present in a region from amino acids at positions 60 to 63 in an amino acid sequence of protein A56 represented by SEQ ID NO: 1, and may include (i) a nucleophilic amino acid present in a region from amino acids at positions 44 to 50 in the amino acid sequence of protein A56 and (ii) a nucleophilic amino acid present in a region from amino acids at positions 53 to 59 in the amino acid sequence of protein A56.

Specifically, the conformational epitope may include amino acids at positions 46 (S46), which is a nucleophilic amino acid, 54 (S54), and 61 (K61), in an amino acid sequence of protein A56 represented by SEQ ID NO: 1. More specifically, the conformational epitope may include amino acids at positions 46 (S46), 54 (S54), 61 (K61), 91 (K91), and 100 (T100) in an amino acid sequence of protein A56 represented by SEQ ID NO: 1. In addition, the conformational epitope may include amino acids at positions 46 (S46), 49 (Y49), 54 (S54), 61 (K61), 62 (K62), and 71 (T71) in an amino acid sequence of protein A56 represented by SEQ ID NO: 1.

In addition, the conformational epitope may include amino acids at positions 61 (K61) and 62 (S62) in an amino acid sequence of protein A56 represented by SEQ ID NO: 1.

Analysis was performed on binding characteristics of a plurality of different antibodies that bind to A56 with high affinity. As a result, it was found that the basic amino acid (K61, K91) at position 61 or 91 of A56 plays, in common, an important role in the binding. Specifically, K61 or K91 forms a strong hydrogen bond with a nucleophilic amino acid present at a paratope of each A56 antibody, and as such binding progresses, heavy and light chains of the antibody are folded to cause shrink folding, which allows the remaining antigen-antibody binding to occur. Separately, K61 or K91 may also contribute to antigen-antibody binding by affecting serine (S62, S92), which is a nucleophilic amino acid located right next thereto. Specifically, it is believed that K61 or K91 excites a hydroxyl group of S62 or S92, which is located right next thereto, to generate an electrostatic force, and thus S62 or S92 also actively contributes to strong binding with the antibody.

The binding molecule or a fragment thereof may compete for binding to A56 by preventing one or more of the following antigen-binding molecules from binding to A56. Such competitive binding means that they share a binding site (epitope) for A56. The binding molecule or a fragment thereof may compete for binding to A56 with one or more of the following antibodies:
an antibody (for example, Ab13) that includes a heavy chain CDR1 having the amino acid sequence of SEQ ID NO: 32, a heavy chain CDR2 having the amino acid sequence of SEQ ID NO: 33, a heavy chain CDR3 having the amino acid sequence of SEQ ID NO: 34, a light chain CDR1 having the amino acid sequence of SEQ ID NO: 35, a light chain CDR2 having the amino acid sequence of SEQ ID NO: 36, and a light chain CDR3 having the amino acid sequence of SEQ ID NO: 37;
an antibody (for example, Ab16) that includes a heavy chain CDR1 having the amino acid sequence of SEQ ID NO: 38, a heavy chain CDR2 having the amino acid sequence of SEQ ID NO: 39, a heavy chain CDR3 having the amino acid sequence of SEQ ID NO: 40, a light chain CDR1 having the amino acid sequence of SEQ ID NO: 41, a light chain CDR2 having the amino acid sequence of SEQ ID NO: 42, and a light chain CDR3 having the amino acid sequence of SEQ ID NO: 43;
an antibody (for example, Ab18) that includes a heavy chain CDR1 having the amino acid sequence of SEQ ID NO: 44, a heavy chain CDR2 having the amino acid sequence of SEQ ID NO: 45, a heavy chain CDR3 having the amino acid sequence of SEQ ID NO: 46, a light chain CDR1 having the amino acid sequence of SEQ ID NO: 47, a light chain CDR2 having the amino acid sequence of SEQ ID NO: 48, and a light chain CDR3 having the amino acid sequence of SEQ ID NO: 49;
an antibody (for example, Ab01) that includes a heavy chain CDR1 having the amino acid sequence of SEQ ID NO: 50, a heavy chain CDR2 having the amino acid sequence of SEQ ID NO: 51, a heavy chain CDR3 having the amino acid sequence of SEQ ID NO: 52, a light chain CDR1 having the amino acid sequence of SEQ ID NO: 53, a light chain CDR2 having the amino acid sequence of SEQ ID NO: 54, and a light chain CDR3 having the amino acid sequence of SEQ ID NO: 55;
an antibody (for example, Ab19) that includes a heavy chain CDR1 having the amino acid sequence of SEQ ID NO: 56, a heavy chain CDR2 having the amino acid sequence of SEQ ID NO: 57, a heavy chain CDR3 having the amino acid sequence of SEQ ID NO: 58, a light chain CDR1 having the amino acid sequence of SEQ ID NO: 59, a light chain CDR2 having the amino acid sequence of SEQ ID NO: 60, and a light chain CDR3 having the amino acid sequence of SEQ ID NO: 61;
an antibody (for example, Ab03) that includes a heavy chain CDR1 having the amino acid sequence of SEQ ID NO: 62, a heavy chain CDR2 having the amino acid sequence of SEQ ID NO: 63, a heavy chain CDR3 having the amino acid sequence of SEQ ID NO: 64, a light chain CDR1 having the amino acid sequence of SEQ ID NO: 65, a light chain CDR2 having the amino acid sequence of SEQ ID NO: 66, and a light chain CDR3 having the amino acid sequence of SEQ ID NO: 67;
an antibody (for example, Ab08) that includes a heavy chain CDR1 having the amino acid sequence of SEQ ID NO: 68, a heavy chain CDR2 having the amino acid sequence of SEQ ID NO: 69, a heavy chain CDR3 having the amino acid sequence of SEQ ID NO: 70, a light chain CDR1 having the amino acid sequence of SEQ ID NO: 71, a light chain CDR2 having the amino acid sequence of SEQ ID NO: 72, and a light chain CDR3 having the amino acid sequence of SEQ ID NO: 73;
an antibody (for example, Ab14) that includes a heavy chain CDR1 having the amino acid sequence of SEQ ID NO: 74, a heavy chain CDR2 having the amino acid sequence of SEQ ID NO: 75, a heavy chain CDR3 having the amino acid sequence of SEQ ID NO: 76, a light chain CDR1 having the amino acid sequence of SEQ ID NO: 77, a light chain CDR2 having the amino acid sequence of SEQ ID NO: 78, and a light chain CDR3 having the amino acid sequence of SEQ ID NO: 79;
an antibody (for example, Ab51) that includes a heavy chain CDR1 having the amino acid sequence of SEQ ID NO: 80, a heavy chain CDR2 having the amino acid sequence of SEQ ID NO: 81, a heavy chain CDR3 having the amino acid sequence of SEQ ID NO: 82, a light chain CDR1 having the amino acid sequence of SEQ ID NO: 83, a light chain CDR2 having the amino acid sequence of SEQ ID NO: 84, and a light chain CDR3 having the amino acid sequence of SEQ ID NO: 85; and
an antibody (for example, Ab55) that includes a heavy chain CDR1 having the amino acid sequence of SEQ ID NO: 86, a heavy chain CDR2 having the amino acid sequence of SEQ ID NO: 87, a heavy chain CDR3 having the amino acid sequence of SEQ ID NO: 88, a light chain CDR1 having the amino acid sequence of SEQ ID NO: 89, a light chain CDR2 having the amino acid sequence of SEQ ID NO: 90, and a light chain CDR3 having the amino acid sequence of SEQ ID NO: 91.

By "compete for binding" is meant that an antibody or other antigen-binding moieties have capacity to interfere with binding of other antibodies or antigen-binding moieties to a particular antigen in a standard competitive binding assay. The capacity or extent to which an antibody or other antigen-binding moieties are capable of interfering with the binding of another antibody or antigen-binding moieties to a particular antigen, and, therefore whether it can be said to cross-compete according to the present invention, can be determined using standard competition binding assays. One suitable assay includes using the Biacore technique in which a level of interaction can be measured using a surface plasmon resonance technique. Another assay for measuring cross-competition uses an ELISA-based approach. A high-throughput process for "epitope binning" antibodies based upon their cross-competition is described in International Patent Application No. WO 2003/48731.

It was identified that the A56 antibodies cross-inhibit binding to A56 despite their epitope sequences being not completely identical. This means that a specific conformation formed between an A56-binding molecule and A56, that is, a conformational epitope of A56, is important for binding to A56. In particular, the antibody Ab19 inhibited binding to A56 to a relatively lesser extent than the other A56 antibodies; however, the antibody Ab19 still remarkably inhibited binding of the other A56 antibodies to A56. Analysis of the binding between the antibody Ab19 and A56 shows strong antigen-antibody binding only at K61 and S62 in A56. This suggests that a sequence of amino acids such as K61 and S62 (or K91 and K92 which provide similar electrostatic properties) in A56 is important for specific binding to A56, and other additional conformational epitopes contribute to stronger binding to A56.

As used herein, the term "antibody" refers to an immune protein that binds to an antigen and interferes with the action thereof, or removes the antigen. There are five types of antibodies: IgM, IgD, IgG, IgA, and IgE, each of which contains a heavy chain produced from the heavy chain constant region gene µ, δ, γ, α, or ε. In antibody techniques, IgG is mainly used. IgG includes four isotypes of IgG1, IgG2, IgG3, and IgG4, each of which may have different structural and functional properties.

The IgG forms a very stable Y-shaped structure (molecular weight: about 150 kDa) made of two heavy chain (about 50 kDa) proteins and two light chain (about 25 kDa) proteins. An antibody has a light chain and a heavy chain, and each chain is divided into a variable region whose amino acid sequence differs from antibody to antibody, and a constant region whose amino acid sequence is the same among antibodies. The heavy chain constant region includes CH1, H (hinge), CH2, CH3 domains. Each of the domains consists of two β-sheets, and these domains are linked by intramolecular disulfide bonds. The two variable regions in the heavy and light chains associate together to form an antigen-binding site. The site exists in each of the two arms on the Y-shape. In the Y-shape, the part capable of binding to an antigen is called an antibody binding fragment (Fab), and the part that does not bind to an antigen is called a crystalizable fragment (Fc). Fab and Fc are connected by a flexible hinge region.

As used herein, the term "CDR" refers to a site that binds to an antigen, the site being a hypervariable region present in heavy and light chain variable regions of an antibody and whose amino acid sequence differs from antibody to antibody. Looking at the three-dimensional structure of an antibody, CDR is in a loop shape on the antibody surface; and below the loop, there is a framework region (FR) that structurally supports CDR. Each of the heavy and light chains has three loop structures, and these six loop-region structures associate with each other to come in direct contact with an antigen. Antigen binding sites on the six loop-region structures are referred to as CDR1, CDR2, CDR3, CDR4, CDR5, and CDR6, respectively, for convenience.

In addition, the antibody fragment may be any one selected from the group consisting of Fab, scFv, F(ab)₂, and Fv. The antibody fragments refer to antigen-binding domains, excluding the crystalizable region (Fc region) that has an effector function to transmit antigen-binding stimulation to cells, complements, or the like, and may include third-generation antibody fragments such as single domain antibody or minibody.

In addition, the antibody fragment has the following advantages: the antibody fragment is smaller in size than a fully-structured IgG, which results in improved penetration into tissues or tumors; and the antibody fragment can be produced in bacteria, which decreases production costs. In addition, the antibody fragment does not have Fc, and thus is used in a case where the function of transmitting antigen-binding stimulation to cells, complements, or the like is not desired. The antibody fragment has a short half-life in the human body, and thus is suitable for *in vivo* diagnostics; however, replacement of some basic, acidic, or neutral amino acids, which are in the amino acids constituting an antibody, may change a unique isoelectric point (pI) of the antibody. Such a change in the isoelectric point of the antibody may induce changes such as decreasing *in vivo* toxic side effects of the antibody or increasing water solubility of the antibody. Thus, for a therapeutic antibody, a fully-structured IgG can be used in consideration of affinity or structural form.

The antibody can be easily produced by known monoclonal antibody production techniques. A method for producing a monoclonal antibody may be performed by preparing a hybridoma using B lymphocytes obtained from an immunized animal, or may be performed by using a phage display technique. However, the present invention is not limited thereto.

The antibody or a fragment thereof may be used as a part of a chimeric antigen receptor (CAR). Specifically, the chimeric antigen receptor includes an antigen-binding domain, a transmembrane domain, and an intracellular signaling domain, in which the antibody or a fragment thereof may be used as the antigen-binding domain.

The antigen-binding domain refers to a region of an antibody which binds to an antigen. The antigen-binding domain may be an antibody or an antigen-binding fragment thereof. Preferably, the antigen-binding domain may be an antigen-binding fragment. In addition, the antigen-binding fragment may be a fragment having one antigen-binding site formed by linking one heavy chain and one light chain, which are in an antibody, via a disulfide bond. The antigen-binding fragment may be any one selected from the group consisting of scFv, Fab, and Fab'; and preferably, the antigen-binding fragment may be scFv. That is, the antigen-binding domain may be scFv.

The transmembrane domain refers to a region that connects a domain, to which an antigen binds, and a domain, which transmits an intracellular signal, in a structure of a protein located in the cell membrane and penetrates the cell membrane; and the transmembrane domain allows the protein located in the cell membrane to be anchored to the cell membrane. The transmembrane domain may be derived from any one selected from the group consisting of T-cell receptor, CD3δ, CD3ε, CD3γ, CD3ζ, CD4, CD5, CD8α, CD9, CD16, CD22, CD27, CD28, CD33, CD37, CD45, CD64, CD80, CD86, CD134, CD137, CD152, CD154, AMN, and PD-1. Specifically, the transmembrane domain may be derived from CD8α.

The intracellular signaling domain refers to a region that transmits a signal into a cell to induce responses such as cell activation, cytotoxic factor release, cytokine production, and proliferation in a case where an antigen receptor (antigen-binding domain) present on the cell surface recognizes an extracellular antigen. In addition, in general, a signal transmitted through one antigen receptor (antigen-binding domain) alone is insufficient for cell activation, and thus a secondary or co-stimulatory signal is required. Accordingly, the intracellular signaling domain may include a primary signaling domain and a secondary signaling domain and/or a co-stimulatory domain. Specifically, the intracellular signaling domain may include a co-stimulatory domain and a primary signaling domain.

The co-stimulatory domain may be derived from at least one molecule selected from the group consisting of TLR1, TLR2, TLR3, TLR4, TLR5, TLR6, TLR7, TLR8, TLR9, TLR10, CARD11, CD2, CD7, CD27, CD28, CD30, CD40, CD54 (ICAM), CD83, CD134 (OX40), CD137(4-1BB), CD278 (ICOS), DAP10, LAT, NKD2C, SLP76, TRIM, and ZAP70. Specifically, the co-stimulatory domain may be derived from CD137(4-1BB).

The primary signaling domain may be derived from FcRy, FcRβ, CD3γ, CD3δ, CD3ε, CD3ζ, CD22, CD79a, CD79b, or CD66d. In particular, for T cells, signals are transmitted into a cell through the chain γ, δ, ε, or ζ of CD3; and in a case of producing chimeric antigen receptor T cells (CAR-T cells), the chain γ, δ, ε, or ζ of CD3 may be used as the primary signaling domain. Specifically, the primary signaling domain may be derived from CD3ζ.

CAR-T cells may be produced using a vector that contains a polynucleotide encoding the chimeric antigen receptor. The polynucleotide may be prepared or engineered, expressed, and delivered using any of a variety of established techniques known and available in the art. To express a desired chimeric antigen receptor on the T cell surface, a polynucleotide encoding the chimeric antigen receptor may be inserted into an appropriate vector.

For the vector, a variety of vectors known in the art may be used; and depending on the type of host cell intended to produce the antigen receptor, expression regulatory sequences such as promoter, terminator, and enhancer, sequences for membrane targeting or secretion, and the like may be appropriately selected and combined in various ways according to the purpose. Specifically, the vector includes, but is not limited to, plasmid vector, cosmid vector, bacteriophage vector, viral vector, and the like. Suitable vectors include a signal sequence or leader sequence for membrane targeting or secretion, in addition to expression regulatory elements such as promoter, operator, start codon, stop codon, polyadenylation signal, and enhancer, and may be constructed in various ways according to the purpose.

For introducing the vector into immune cells, methods known in the art may be used. For example, the vector may be introduced into the cells using transient transfection, microinjection, transduction, cell fusion, calcium phosphate precipitation, liposome-mediated transfection, DEAE-dextran-mediated transfection, polybrene-mediated transfection, electroporation, or gene gun, or using other known methods for introducing a nucleic acid into cells (Wu et al., J. Bio. Chem., 267:963-967, 1992; Wu and Wu, J. Bio. Chem., 263:14621-14624, 1988). However, the present invention is not limited thereto.

Transduced or transfected immune cells are proliferated after vector introduction. In an embodiment, transfected immune cells may be proliferated by being incubated for at least about 1 day, 2 days, 3 days, 4 days, 5 days, 6 days, 7 days, 8 days, 9 days, 10 days, 11 days, 12 days, 13 days, or 14 days, and preferably for 12 to 14 days.

Methods for identifying whether the vector has been introduced well into the immune cells include, for example, molecular biological assays well known to those skilled in the art, such as Southern and Northern blotting, RT-PCR and PCR; biochemical assays, such as detection of the presence or absence of a particular peptide by immunological methods (for example, ELISA and Western blotting).

The A56-binding molecule or a fragment thereof according to the present invention may bind to protein A56 with binding affinity of less than about 1.0 nM (1 × 10⁻⁸) K_{D} (M) as measured by surface plasmon resonance. Specifically, the binding molecule or a fragment thereof may have binding affinity with protein A56 of less than 1× 10⁻⁹, 9× 10⁻¹⁰, or 8×10⁻¹⁰ K_{D} (M). In an embodiment of the present invention, the binding molecules in the embodiment were determined to have binding affinities of 7.39×10⁻¹⁰, 2.28×10⁻¹⁰, 1.96×10⁻¹⁰, 4.92×10⁻¹⁰, and 4.38×10⁻¹⁰K_{D} (M).

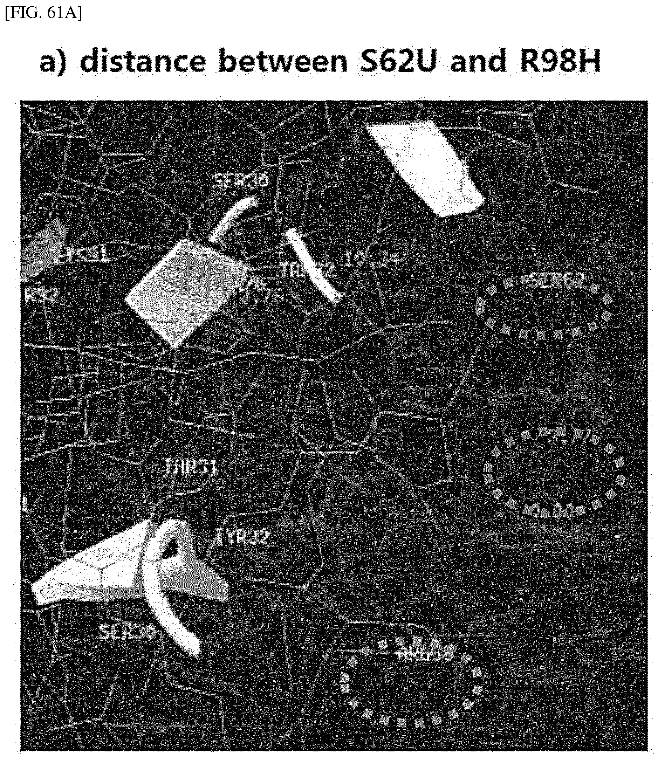

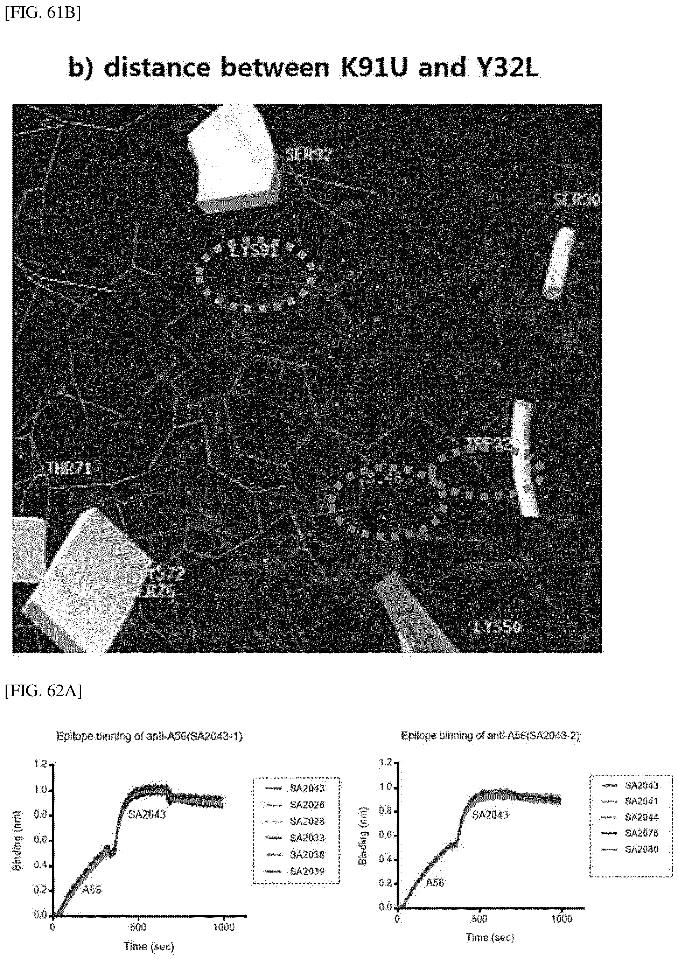

In the present invention, to identify a novel conformational epitope of protein A56, a structure of the complex formed by binding of the protein A56 to an anti-A56 antibody in an embodiment was crystallized at a resolution of 1.8 Å (FIGS. 61 to 65). As a result, a novel conformational epitope of the protein A56 and a paratope of the anti-A56 antibody in an embodiment were identified, and are shown in Table 1.

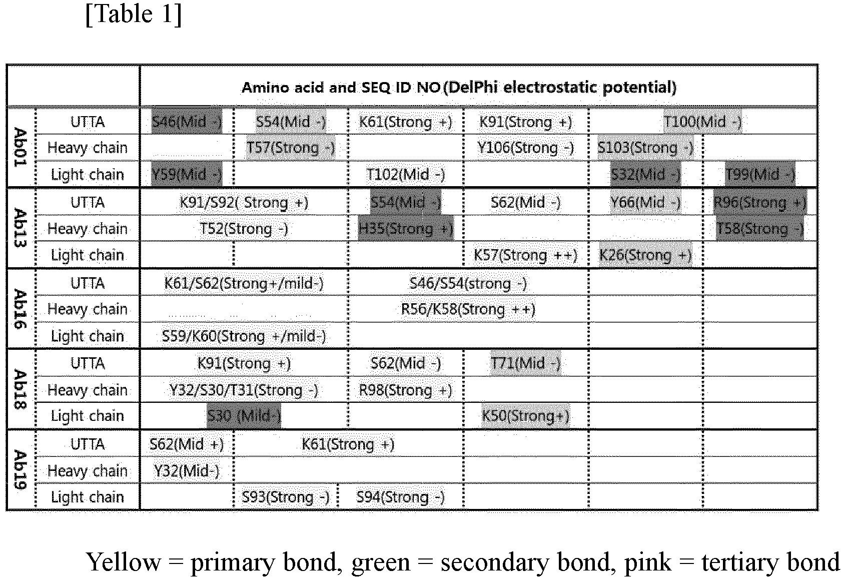

The binding molecule or a fragment thereof may bind to the protein A56 within an intermolecular distance of 6.5 Å. The binding molecule or a fragment thereof may form a van der Waals bond, a hydrophobic bond, or an electrostatic bond with the protein A56.

The binding molecule or a fragment thereof may be used to decrease burden of cancer cells by being administered together with an oncolytic virus. The burden of cancer cells may refer to the weight, volume, or number of cancer cells. Cancer cells, which have survived even infection with an oncolytic virus, express protein A56 on the cell surface. Thus, the binding molecule or a fragment thereof, which specifically binds to such A56, according to the present invention enables secondary anticancer therapy or targeting for secondary anticancer therapy.

The oncolytic virus may be a vaccinia virus. The vaccinia virus may be, but is not limited to, one of the following vaccinia virus strains: Western Reserve (WR), New York vaccinia virus (NYVAC), Wyeth (The New York City Board of Health; NYCBOH), LC16m8, Lister, Copenhagen, Tian Tan, USSR, TashKent, Evans, International Health Division-J (IHD-J), and International Health Division-White (IHD-W).

The oncolytic virus may be one in which thymidine kinase (TK) gene is deleted. Specifically, the oncolytic virus may be a recombinant vaccinia virus in which a thymidine kinase gene is deleted.

As used herein, the term "thymidine kinase (TK)" refers to an enzyme that is called thymidine kinase and involved in nucleotide biosynthesis. The TK is an enzyme used for nucleotide biosynthesis in both cells and viruses. Here, for the cells, normal cells do not divide anymore, and thus no TK exists therein; and even for rapidly dividing cells such as hair follicle cells, TK is not present in an amount sufficient for viruses to utilize. From these viewpoints, a virus is allowed to proliferate only in the presence of cancer cells, in which TK is present, by deleting TK gene, so that the cancer cells can be selectively killed.

The oncolytic virus may contain a nucleic acid encoding protein A56 or a fragment thereof. The protein A56 or a fragment thereof is as described above. The protein A56 may be wild-type protein A56 or a variant of the protein A56. The nucleic acid encoding the protein A56 or a fragment thereof may be a nucleic acid encoding wild-type protein A56 or a variant of the protein A56. The oncolytic virus may be co-administered with hydroxyurea simultaneously, sequentially, or in reverse order.

In another aspect of the invention, there is provided a method for decreasing burden of cancer cells in a subject, comprising a step of administering, to the subject, the binding molecule or a fragment thereof. The binding molecule or a fragment thereof is as described above.

The subject may be a mammal including a human, and may be a non-human animal. The term "non-human animal" refers to any vertebrate and may include mammals and non-mammals, such as non-human primates, sheep, dogs, cats, horses, cattle, chickens, amphibians, and reptiles. In addition, the subject means a subject who is suffering from a cancer disease or a disease in a state that can be alleviated, inhibited, or treated by administering the oncolytic virus.

A dosage of the binding molecule or a fragment thereof varies depending on the subject's condition and body weight, the severity of disease, the type of drug, the route and period of administration, and can be appropriately selected by a person skilled in the art.

The binding molecule or a fragment thereof may be administered parenterally, and such administration may be performed by any suitable method, such as intratumoral, intraperitoneal, subcutaneous, intradermal, intranodal, or intravenous administration. Among these, intratumoral, intraperitoneal, or intravenous administration may be preferred.

Regarding the administration route, dosage, and frequency of administration, the binding molecule or a fragment thereof may be administered to a subject in a variety of ways and amounts depending on the subject's condition and the presence or absence of side effects; and the optimal administration route, dosage, and frequency of administration therefor may be selected by a person skilled in the art within a suitable range. In addition, the binding molecule or a fragment thereof may be administered in combination with another drug or physiologically active substance whose therapeutic effect is known for the disease to be treated, or may be formulated in the form of a combination preparation with the other drug. Specifically, the binding molecule or a fragment thereof may be provided in the form of an injection.

In yet another aspect of the present invention, there is provided a pharmaceutical composition for preventing or treating cancer, comprising the binding molecule or a fragment thereof. The binding molecule or a fragment thereof is as described above. The pharmaceutical composition may further comprise hydroxyurea.

The pharmaceutical composition may further comprise a physiologically acceptable carrier. In addition, the pharmaceutical composition may further comprise suitable excipients and diluents commonly used in the preparation of pharmaceutical compositions. In addition, the pharmaceutical composition may be formulated in the form of oral preparations such as powders, granules, tablets, capsules, suspensions, emulsions, syrups, and aerosols, external preparations, suppositories, or injections according to conventional methods, and used. Specifically, the anticancer agent may be in the form of an injection. Suitable formulations known in the art may be those disclosed in the literature (Remington's Pharmaceutical Science, 1985).

The pharmaceutical composition may be intended for preventing or treating cancer selected from the group consisting of lung cancer, colorectal cancer, prostate cancer, thyroid cancer, breast cancer, brain cancer, head and neck cancer, esophageal cancer, skin cancer, thymus cancer, gastric cancer, colon cancer, liver cancer, ovarian cancer, uterine cancer, bladder cancer, rectal cancer, gallbladder cancer, biliary tract cancer, pancreatic cancer, lymphoma, acute leukemia, multiple myeloma, and combinations thereof.

In addition, for the pharmaceutical composition, examples of the carrier, the excipient, and the diluent may include sodium chloride, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methyhydroxybenzoate, propylhydroxybenzoate, talc, magnesium stearate, mineral oil, and the like. In a case where the anticancer agent is formulated, preparation thereof may be made using diluents or excipients such as fillers, extenders, binders, wetting agents, disintegrants, and surfactants which are commonly used.

For the pharmaceutical composition, preparations for parenteral administration may include sterile aqueous solutions, non-aqueous solvents, suspensions, emulsions, lyophilized preparations, suppositories, and the like. For the non-aqueous solvents and the suspensions, propylene glycol, polyethylene glycol, vegetable oil such as olive oil, injectable ester such as ethyl oleate, and the like may be used. As bases of the suppositories, Witepsol, macrogol, Tween 61, cacao fat, laurin fat, glycerogelatin, and the like may be used.

The cancer may be solid cancer or blood cancer. Specifically, the solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof. In addition, the blood cancer may be any one selected from the group consisting of lymphoma, acute leukemia, multiple myeloma, and combinations thereof.

In still yet another aspect of the present invention, there is provided a kit for preventing or treating cancer, comprising the binding molecule or a fragment thereof and an oncolytic virus. The binding molecule or a fragment thereof and the oncolytic virus are as described above. The kit may further comprise hydroxyurea.

A dosage of the oncolytic virus varies depending on the subject's condition and body weight, the severity of disease, the type of drug, the route and period of administration, and can be appropriately selected by a person skilled in the art. The dosage may be such that a patient receives an oncolytic virus at 1×10⁵ to 1×10¹⁸ of virus particles, infectious virus units (TCID₅₀), or plaque forming units (pfu). Specifically, the dosage may be such that a patient receives an oncolytic virus at 1×10⁵, 2×10⁵, 5×10⁵, 1×10⁶ 2×10⁶ , 5×10⁶, 1×10⁷ , 2×10⁷, 5×10⁷, 1×10⁸, 2×10⁸, 5×10⁸, 1×10⁹, 2×10⁹, 5×10⁹, 1×10¹⁰ , 5×10¹⁰ , 1×10¹¹, 5×10¹¹ , 1×10¹², 1×10¹³, 1×10¹⁴, 1×10¹⁵ 1×10¹⁶ , 1×10¹⁷ , or higher of virus particles, infectious virus units, or plaque forming units, and various numerical values and ranges between the above-mentioned numerical values may also be included therein. Preferably, the oncolytic virus may be administered at a dose of 1×10⁵ to 1×10¹⁰ pfu. More preferably, the oncolytic virus may be administered at a dose of equal to or greater than 1×10⁵ and lower than 1×10⁹ pfu.

The oncolytic virus may be administered parenterally, and such administration may be performed by any suitable method, such as intratumoral, intraperitoneal, subcutaneous, intradermal, intranodal, or intravenous administration. Among these, intratumoral, intraperitoneal, or intravenous administration may be preferred.

Regarding the administration route, dosage, and frequency of administration, the oncolytic virus may be administered to a subject in a variety of ways and amounts depending on the patient's condition and the presence or absence of side effects; and the optimal administration route, dosage, and frequency of administration therefor may be selected by a person skilled in the art within a suitable range. In addition, the oncolytic virus may be administered in combination with another drug or physiologically active substance whose therapeutic effect is known for the disease to be treated, or may be formulated in the form of a combination preparation with the other drug. Specifically, the oncolytic virus may be provided in the form of an injection.

In still yet another aspect of the invention, there is provided a use of the binding molecule or a fragment thereof for the treatment of cancer.

### Mode for the Invention

Hereinafter, the present invention will be described in more detail by way of the following examples. However, the following examples are for illustrative purposes only, and the scope of the present invention is not limited thereto.

### I. Construction of vector that contains nucleic acid encoding protein A56 or fragment thereof, and identification of expression thereof on tumor cell surface

### Preparation 1.1. Construction of vector that contains nucleic acid encoding protein A56 or fragment thereof

In order to construct plasmids encoding the wild-type protein A56 and fragments thereof, primers for removing specific regions were prepared, and overlapping PCR was performed by forming a region overlapping with GFP.

### Preparation 1.2. Production of oncolytic vaccinia virus that contains nucleic acid encoding protein A56 or fragment thereof

In order to produce oncolytic vaccinia viruses containing protein A56, HeLaS3 (ATCC) cell line was seeded in 6-well plates at 4× 10⁵ cells per well, and then prepared in EMEM medium containing 10% fetal bovine serum. Treatment with each of the wild-type vaccinia viruses of Wyeth strain and Western Reserve strain was performed at an MOI of 0.05. After 2 hours, the medium was replaced with EMEM medium containing 2% fetal bovine serum, and then a vector, which contains a reporter gene and a gene for insertion, was transfected into the cells using Xfect reagent buffer. Culture was performed for 4 hours. Subsequently, the medium was replaced with EMEM medium containing 2% fetal bovine serum, and then the cells were further cultured for 72 hours. Finally, the infected cells were collected, and then freezing and thawing were repeated 3 times. The cells were lysed by sonication, and a sucrose cushion method was used to obtain free oncolytic vaccinia viruses (OTS-412, WOTS-418) that contain a nucleic acid encoding the protein A56 or a fragment thereof.

### Reference Example 1. Comparison of sequences of protein A56 for respective vaccinia virus strains

A request for gene sequencing of the membrane protein A56s in OTS-412 of Wyeth strain and WOTS-418 of Western Reserve strain, each of which is a vaccinia virus with TK region deleted, was made to Macrogen. Regarding the respective sequences for the protein A56s in OTS-412 and WOTS-418, alignment was performed through NCBI Blast and Uniprot. As a result, 100% identical sequences were not found; and in particular, it was identified that the amino acids at positions 245 to 250 were deleted. Comparison was performed with 4 other strains having high sequence homology

### Experimental Example 1. Expression of protein A56 on cancer cell surface

The oncolytic vaccinia virus that contains a nucleic acid encoding protein A56 or a fragment thereof, which was produced in Preparation Example 1.2, was used to infect a human lung cancer cell line (A549), a human colorectal cancer cell line (HCT-116), or a human melanoma cell line (SK-MEL-5), to identify whether the protein A56 was expressed on the cancer cell surface.

Specifically, A549 (lung carcinoma, ATCC, USA), HCT-116 (colorectal carcinoma, Korea Cell Line Bank), or SK-MEL-5 (human melanoma, Korea Cell Line Bank) cell line was seeded on a cover glass in a 12-well-plate at 3.5×10⁴ cells per well. Subsequently, the cells were infected with the oncolytic vaccinia virus at an MOI of 0.1, and then incubated for 30 hours at a condition of 37°C and 5% CO₂. Each of the incubated cell lines was harvested. Then, treatment with 4% (v/v) paraformaldehyde (PFA), 1% (v/v) BSA, anti-A56 primary antibody (cat no. ABIN1606294, Antibodies-Online), which was diluted at a ratio of 1:500, and secondary antibody (Alexa 594, cat no. A21205, Invitrogen), which was diluted at a ratio of 1:200, was performed. DAPI staining was performed. Then, a sample of each cell line was placed on a slide glass and observed using a confocal microscope (Olympus, FV1000).

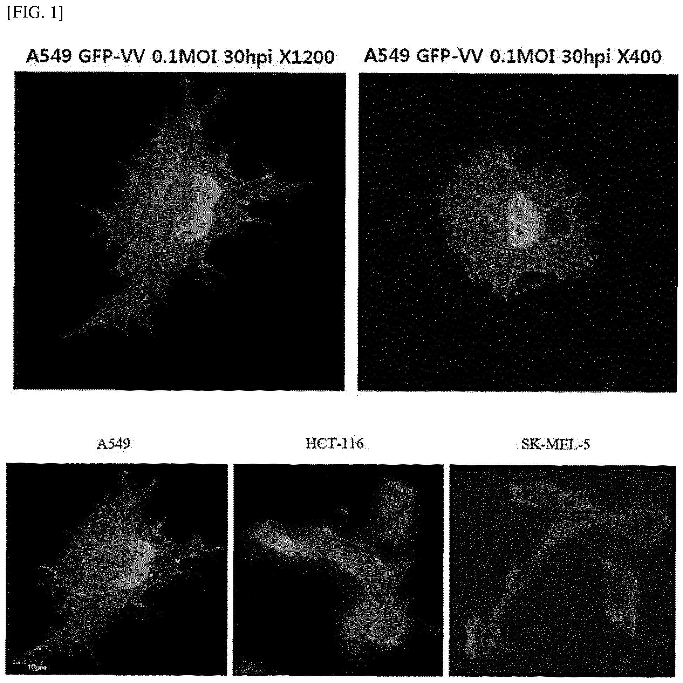

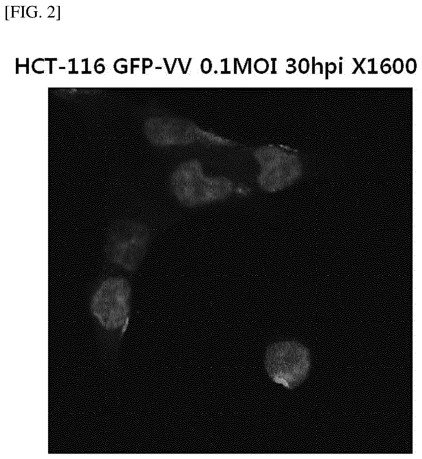

As a result, it was identified that the protein A56 was expressed on the cell surface of the A549 and HCT-116 cell lines infected with the oncolytic vaccinia virus (FIGS. 1 and 2).

### Experimental Example 2. Identification of expression of protein A56 on mouse tumor tissue surface (I)

A cancer-induced mouse model was subjected to intraperitoneal administration of the oncolytic vaccinia virus that contains a nucleic acid encoding protein A56 or a fragment thereof, which was produced in Preparation Example 1.2, to identify whether the protein A56 was expressed on the tissue surface.

Specifically, BALB/c nude mice were subcutaneously transplanted with HCT-116 colorectal cancer cell line (Korea Cell Line Bank, KCLB) at 6.3×10⁶ cells to induce cancer. When the average tumor volume reached 150 mm³ to 200 mm³, the mice were subjected to intraperitoneal administration of the oncolytic vaccinia virus, which was produced in Preparation Example 1.2, at a dose of 2×10⁷ pfu. Then, on day 4, the mice were sacrificed, and tumor tissues, and brain, heart, lung, muscle, kidney, liver, and spleen tissues were collected therefrom.

Immunofluorescence staining for protein A56 was performed in the same manner as in Experimental Example 1. DAPI staining was performed. Then, a sample of each tissue was placed on a slide glass and observed using a fluorescence microscope.

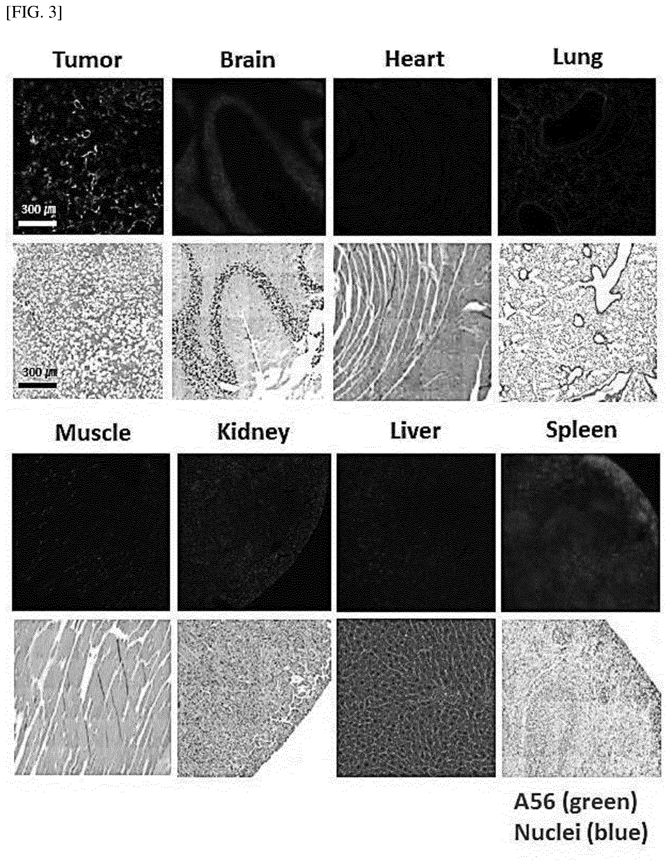

As a result, it was identified that the protein A56 was expressed on the tumor tissue surface in the mice administered with the oncolytic vaccinia virus. On the other hand, it was identified that the protein A56 was not expressed in the brain, heart, lung, muscle, kidney, liver, and spleen tissues (FIG. 3). From these results, it was identified that in a case where a binding molecule is produced which targets protein A56 expressed on the tumor tissue surface following administration of the oncolytic vaccinia virus, this antibody can be utilized as anticancer immunotherapy.

### Experimental Example 3. Identification of expression of protein A56 on rabbit tissue surface

Normal rabbits were subjected to intravenous administration of the oncolytic vaccinia virus that contains a nucleic acid encoding protein A56 or a fragment thereof, which was produced in Preparation Example 1.2, to identify whether the protein A56 was expressed on the tissue surface. In this way, toxicity risk was analyzed.

Specifically, New Zealand rabbits were subjected to intravascular administration of the oncolytic vaccinia virus produced in Preparation Example 1.2 at a dose of 1×10⁸ pfu or 1×10⁹ pfu. Then, on week 3 or 8, the rabbits were sacrificed, and brain, heart, lung, muscle, kidney, liver, and spleen tissues were collected therefrom.

Immunofluorescence staining for protein A56 was performed in the same manner as in Experimental Example 1. DAPI staining was performed. Then, a sample of each tissue was placed on a slide glass and observed using a fluorescence microscope.

As a result, it was identified that the protein A56 was not expressed in the heart, lung, muscle, kidney, liver, and spleen tissues of the rabbits administered with the oncolytic vaccinia virus.

On the other hand, a fluorescence reaction was detected in the brain tissue of the rabbits administered with the oncolytic vaccinia virus. To identify whether the detected fluorescence reaction is a non-specific reaction of an anti-A56 antibody, the brain tissue of normal rabbits, which were not administered with the oncolytic vaccinia virus, was subjected to immunofluorescence staining in the same manner as above, and then a fluorescence reaction therein was checked using a fluorescence microscope.

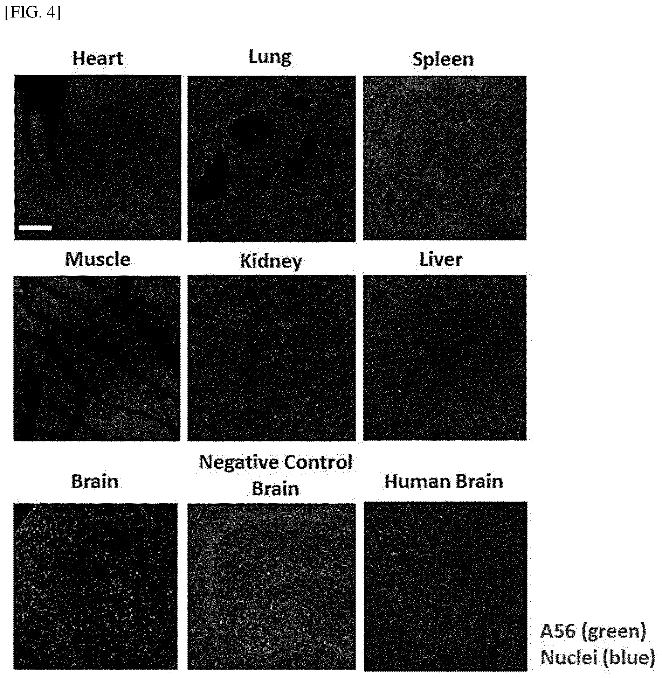

As a result, a fluorescence reaction was detected even in the brain tissue of rabbits that were not administered with the oncolytic vaccinia virus. From this result, it was identified that such a fluorescence reaction was a non-specific reaction (FIG. 4).

In addition, in a case where brain tissue (Pusan National University Yangsan Hospital, Korea) of normal humans, who were not administered with the oncolytic vaccinia virus, was subjected to immunofluorescence staining in the same manner as above, a weak fluorescence reaction was detected using a fluorescence microscope. However, from the viewpoint that an antibody does not cross the blood brain barrier, it is determined that an anti-A56 antibody will not cause a non-specific reaction unless the antibody is administered directly into the ventricle.

### Experimental Example 4. Identification of expression of protein A56 on mouse tumor tissue surface (II)

A cancer-induced mouse model was subjected to co-administration of the oncolytic vaccinia virus that contains a nucleic acid encoding protein A56 or a fragment thereof, which was produced in Preparation Example 1.2, and hydroxyurea, to identify whether the protein A56 was expressed on the tissue surface.

Specifically, BALB/c nude mice were subcutaneously transplanted with Renca cancer cell line (Korea Cell Line Bank) at 6.3×10⁶ cells to induce cancer. When the average tumor volume reached 150 mm³ to 200 mm³, the mice were subjected to intratumoral administration of the oncolytic vaccinia virus, which was produced in Preparation Example 1.2, at a dose of 2×10⁷ pfu, and to administration of hydroxyurea at a dose of 30 mg/kg.

The produced renal cancer cell-transplanted mice were divided into three groups (n=4). The group receiving intratumoral administration of saline was set as a control group, and the group receiving administration of the oncolytic vaccinia virus (1×10⁷ pfu) alone and the group receiving co-administration of the oncolytic vaccinia virus (1×10⁷ pfu) and hydroxyurea (30 mg/kg) were set as experimental groups. The oncolytic vaccinia virus was intratumorally administered twice on days 0 and 14, and the hydroxyurea was intraperitoneally administered 6 times per week from 1 day before administration of the oncolytic vaccinia virus to day 21 after administration of the oncolytic vaccinia virus, except for the day when the oncolytic vaccinia virus was administered.

The mice were sacrificed on days 7, 10, and 14 after first administration of the oncolytic vaccinia virus, and tumor tissues were collected therefrom. Also, the mice were sacrificed on days 21, 24, and 28 after second administration of the oncolytic vaccinia virus, and tumor tissues were collected therefrom. Immunofluorescence staining for protein A56 was performed in the same manner as in Experimental Example 1. DAPI staining was performed. Then, a sample of each tissue was placed on a slide glass and observed using a fluorescence microscope.

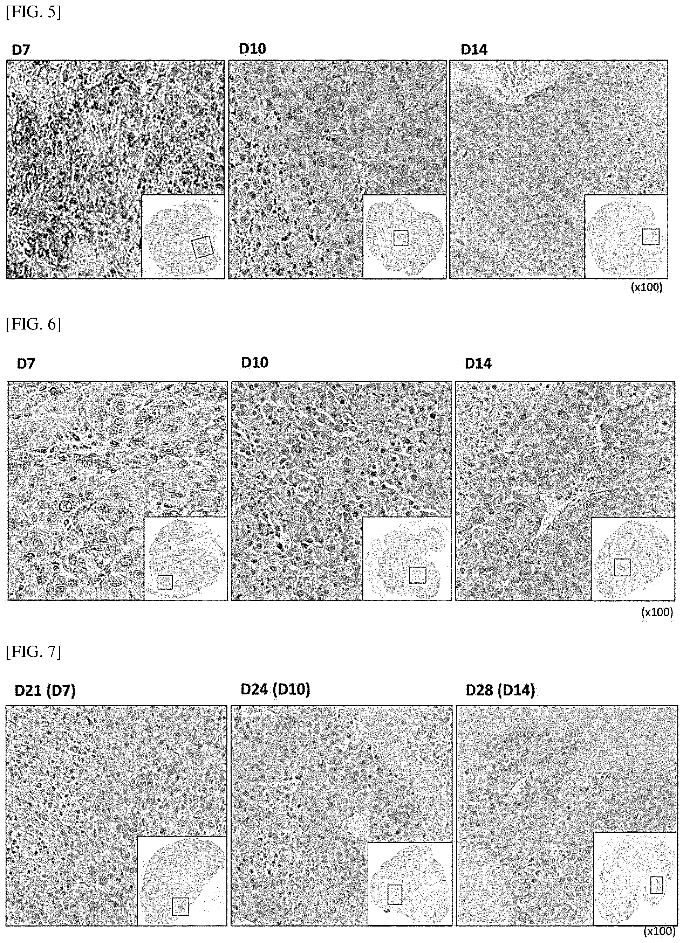

As a result, it was identified that the protein A56 was clearly expressed on the tumor surface of the mice of the group having received administration of only the oncolytic vaccinia virus and the group having received co-administration of the oncolytic vaccinia virus and the hydroxyurea, until days 7, 10, and 14 after first administration of the oncolytic vaccinia virus (FIGS. 5 and 6).

### Experimental Example 5. Expression of protein A56 or fragment thereof on cell surface

The plasmid encoding the protein A56 or a fragment thereof was used to treat HeLa cell line, to identify whether the protein A56 was expressed on the cell surface. For the fragment of protein A56, primers for removing specific regions from the wild-type protein A56 were prepared, and overlapping PCR was performed by forming a region overlapping with GFP.

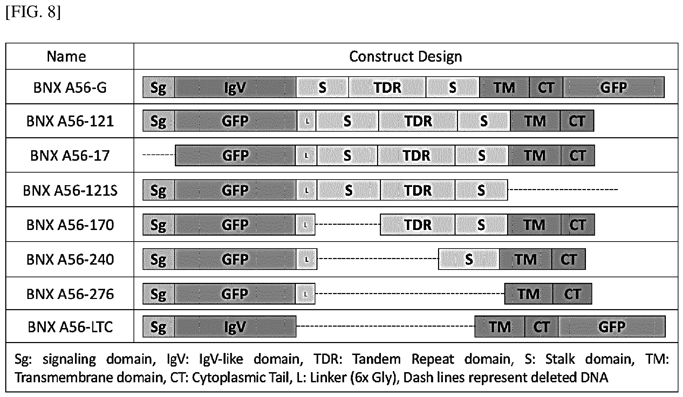

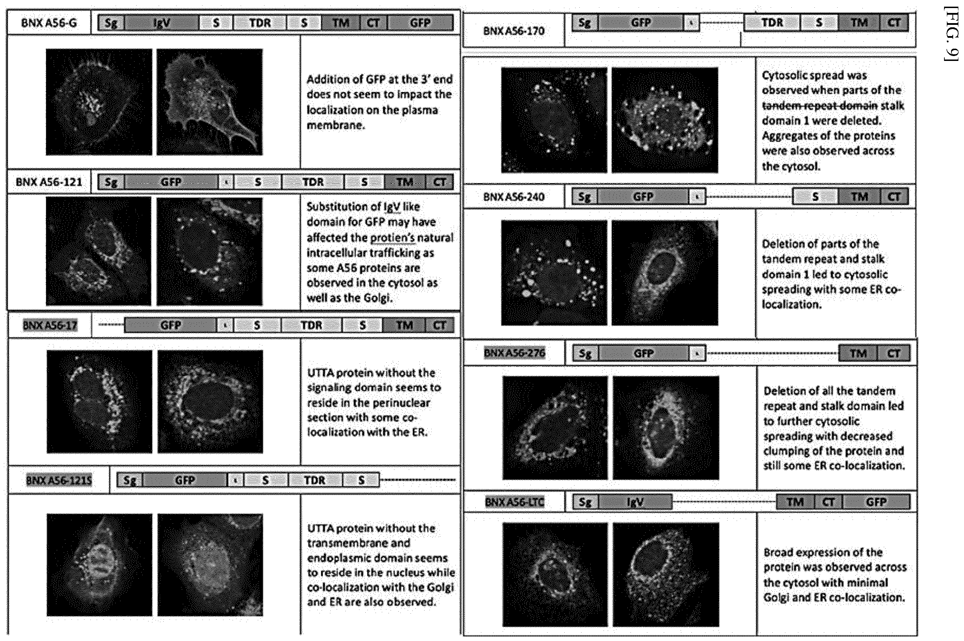

Specifically, HeLa (cervical cancer cells, ATCC, USA) cell line was seeded on a cover glass in a 12-well-plate at 3.5×10⁴ cells per well. Subsequently, the plasmid for the A56 fragment diluted by being mixed with Xfect reaction buffer and Xfect polymer was incubated with the HeLa cell line at a condition of 37°C and 5% CO₂ for 30 hours. Each incubated cell line was harvested. Then, treatment with 4% (v/v) paraformaldehyde (PFA), 1% (v/v) BSA, anti-A56 antibody (cat no. ABIN1606294, Antibodies-Online), which was diluted at a ratio of 1:500, and secondary antibody (Alexa 594, cat no. A21205, Invitrogen), which was diluted at a ratio of 1:200, was performed. The nucleus was stained with DAPI, and the Golgi apparatus was stained with a fluorescent dye. Thereafter, a sample of the cell line was placed on a slide glass and observed using a confocal microscope (Olympus, FV1000). The results are illustrated in FIGS. 8 and 9, and Table 2.

**[Table 2]**

| | Nucleus | Golgi Apparatus | ER | Cytosol(broad) | Plasma Membrane |
|---|---|---|---|---|---|
| A56-G | --- | + | + | + | +++ |
| A56-121 | --- | ++ | + | + | + |
| A56-17 | --- | --- | +++ | --- | --- |
| A56-121S | | + | + | + | --- |
| A56-170 | --- | --- | + | ++ | --- |
| A56-240 | --- | --- | ++ | + | --- |
| A56-276 | --- | --- | ++ | + | --- |
| BNX A56-LTC | --- | + | ++ | + | --- |

Wild-type A56 (A56-G) and fragments of A56 obtained by partial truncation of six regions of A56 were allowed to be expressed on the cell surface. As a result, it was identified that only wild-type A56 (A56-G) and A56-121, in which the IgV-like domain is truncated, reached the cell surface and were expressed thereon. It was identified that among the A56 variants in which the IgV-like domain is excluded, variants, in which signal peptides, transmembrane domain, stalk region, and tandem repeats regions are truncated individually or in combination, were not expressed on the plasma membrane. In addition, it was identified that in a case of including the IgV-like domain, even a variant including only the transmembrane domain was not expressed on the plasma membrane. That is, it was identified that variants, in which the IgV-like domain is excluded and which include five regions, were expressed on the plasma membrane.

### II. Production of antibody binding to protein A56 or fragment thereof

### Preparation Example 2. Purification and production of protein A56

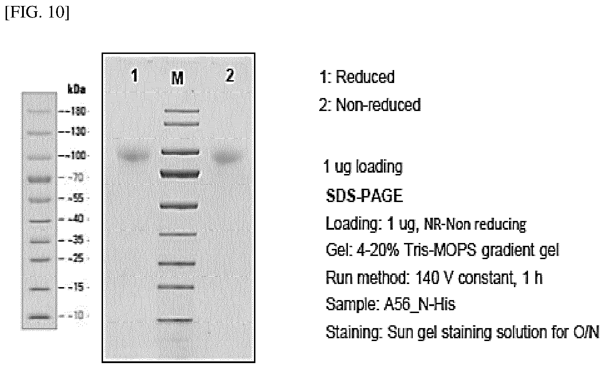

Vector DNA (N293F-A56-C-HIS) was amplified, introduced into HEK293F cells, and overexpressed therein. Subsequently, primary purification was performed by affinity chromatography (Ni-NTA), and then secondary purification was performed by cation exchange chromatography (CEX). In this way, A56-C-HIS protein was finally produced (FIG. 10).

### Preparation Example 3. Production of antibody binding to protein A56 or fragment thereof (I)

A request for production of anti-A56 antibodies, which specifically bind to the protein A56, or a variant or fragment thereof, was made to Ybiologics. 61 antibodies were produced using phage library techniques and CDRs thereof were analyzed. Phage libraries were added to a tube coated with A56 antigen, and biopanning was performed to find binding hits. Phages having specific binding were selected by performing washing 3 times on average. Three panning processes were performed. Then, affinity tests were performed, and colonies showing high affinity were picked at small amounts to identify whether the colonies exhibit affinity to the actual antigen. Sets with a relatively high number of hits were chosen, and an automated system was used for picking and hit selection.

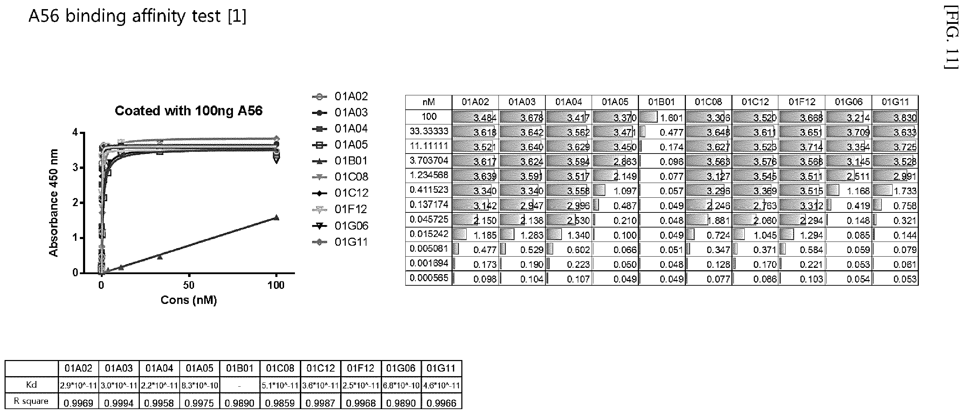

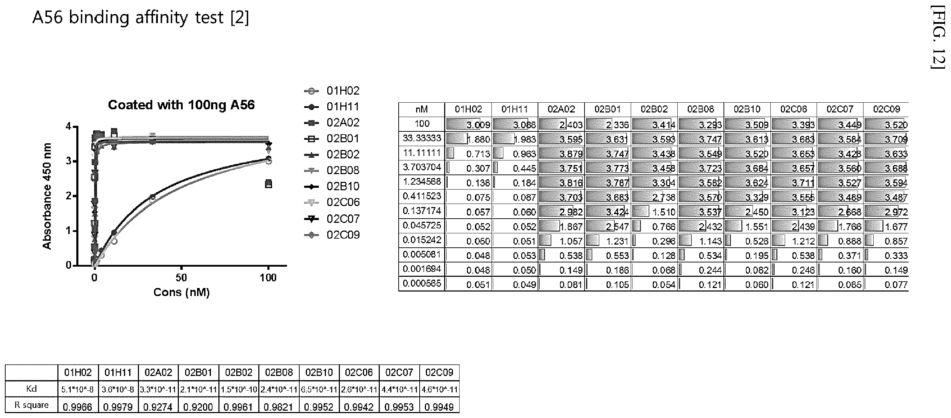

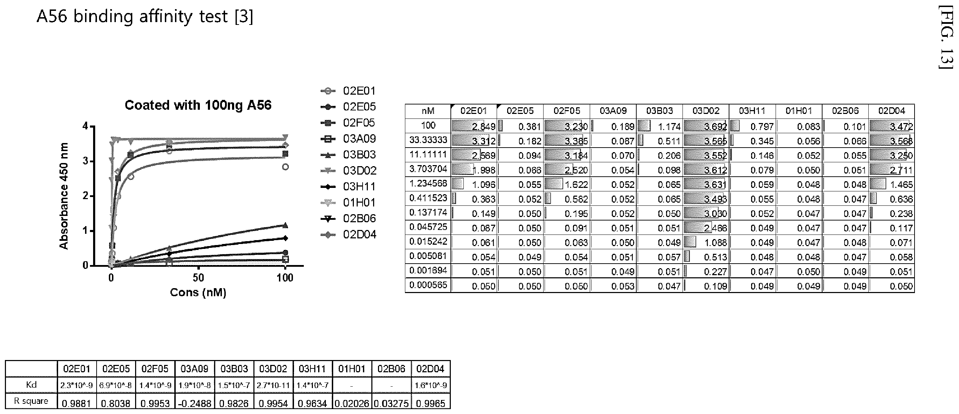

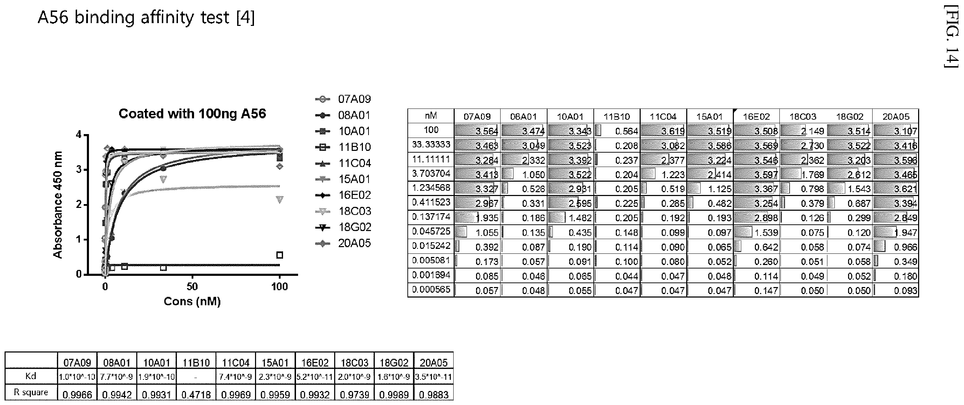

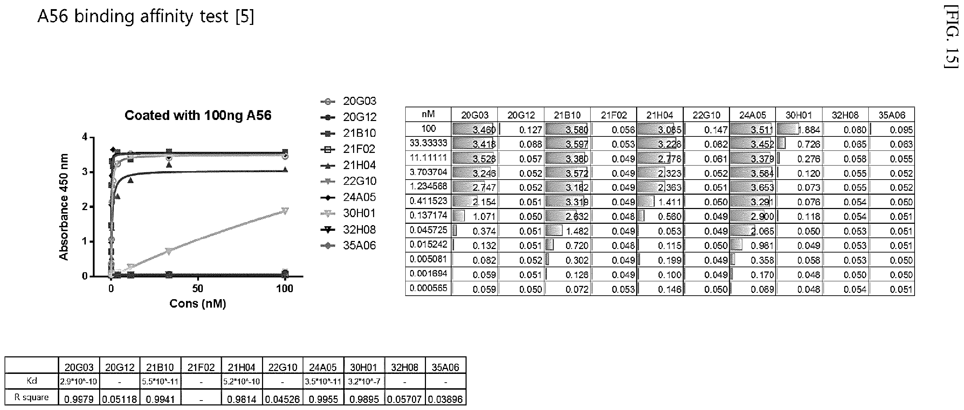

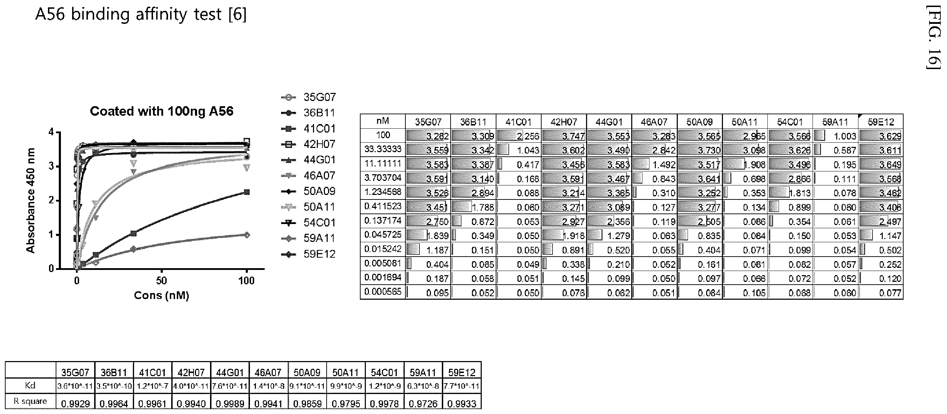

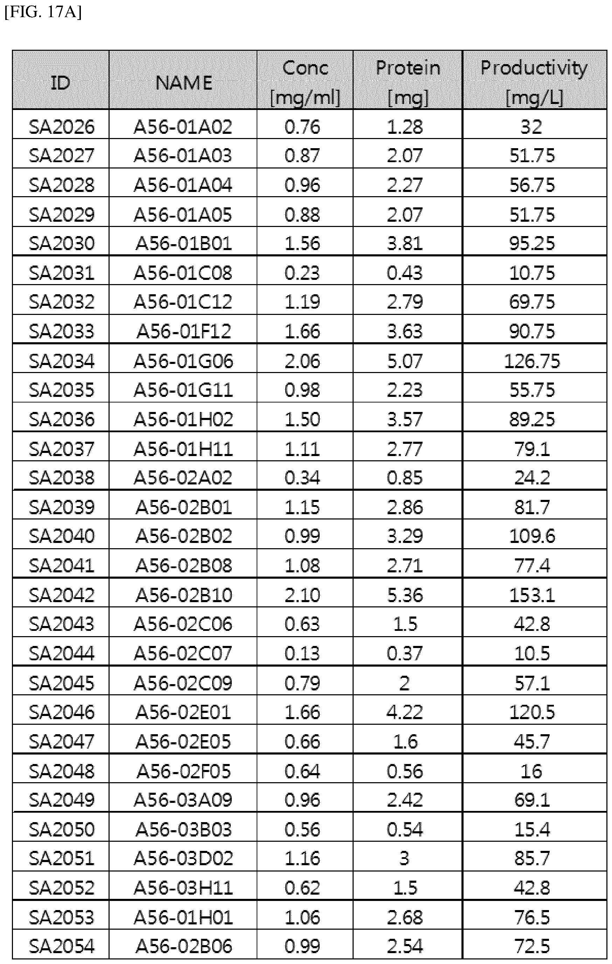

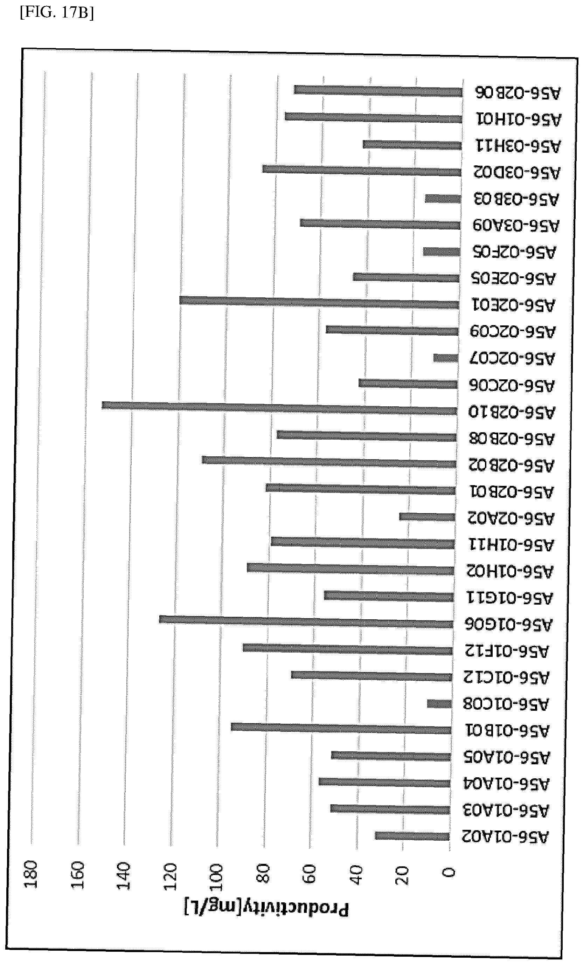

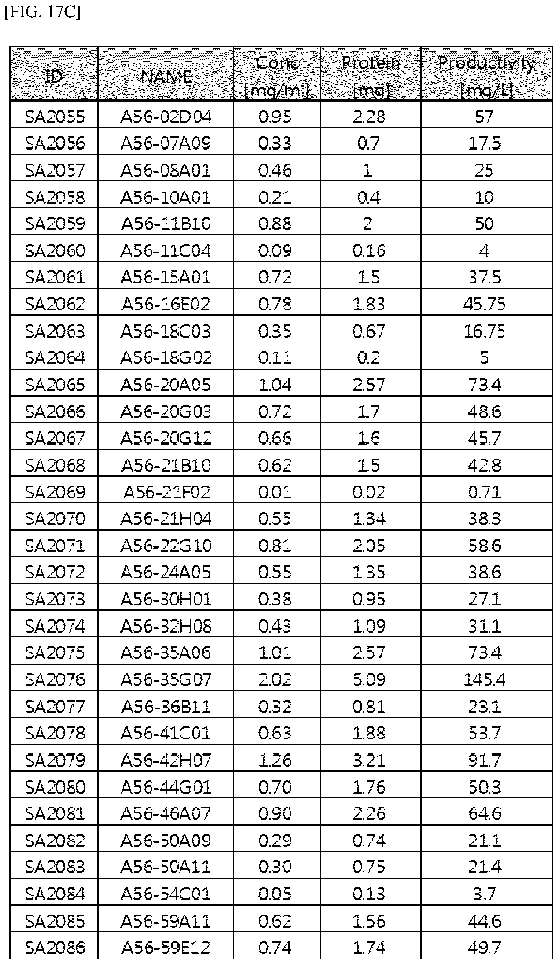

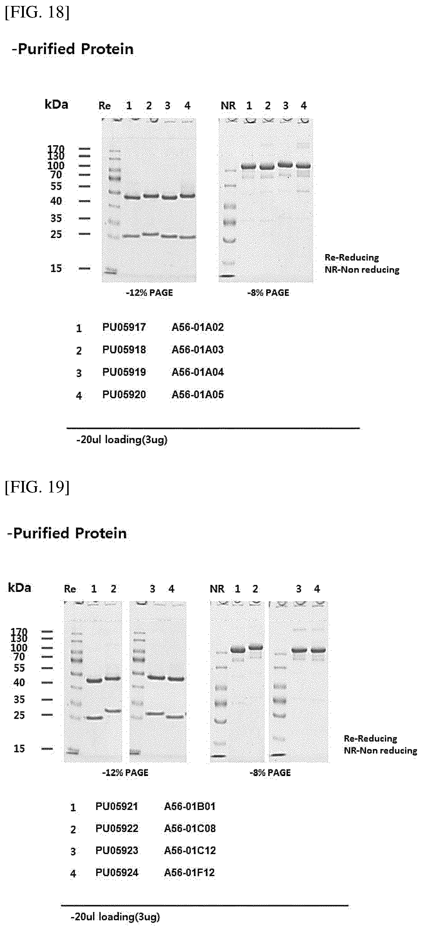

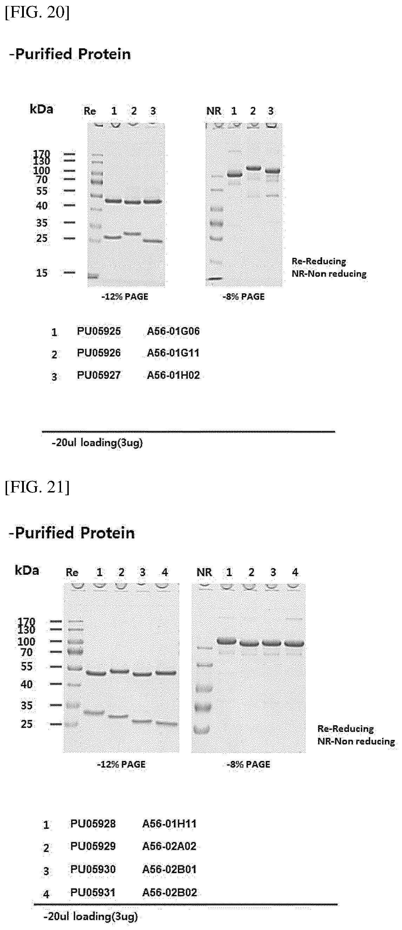

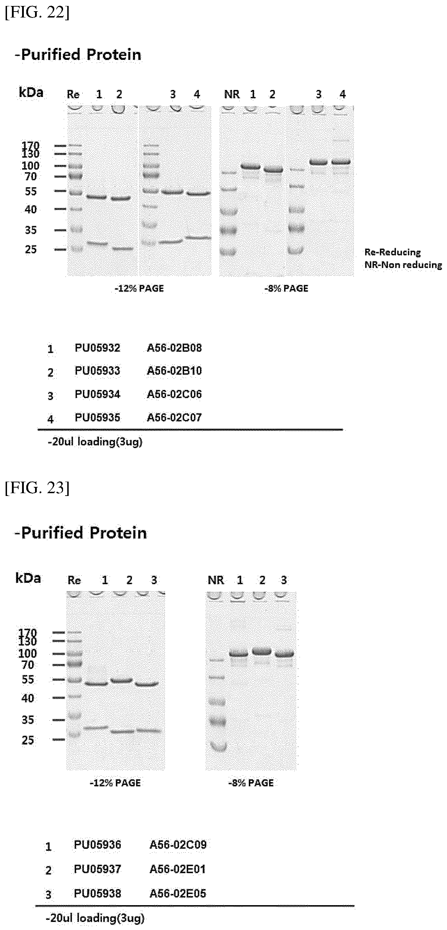

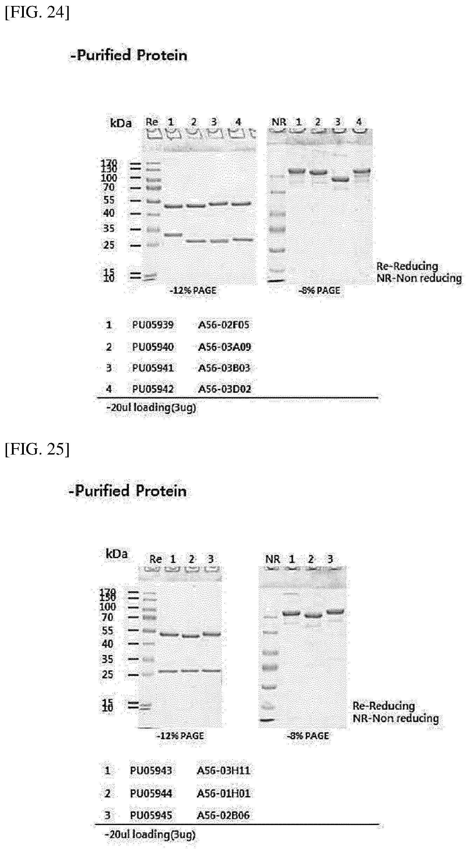

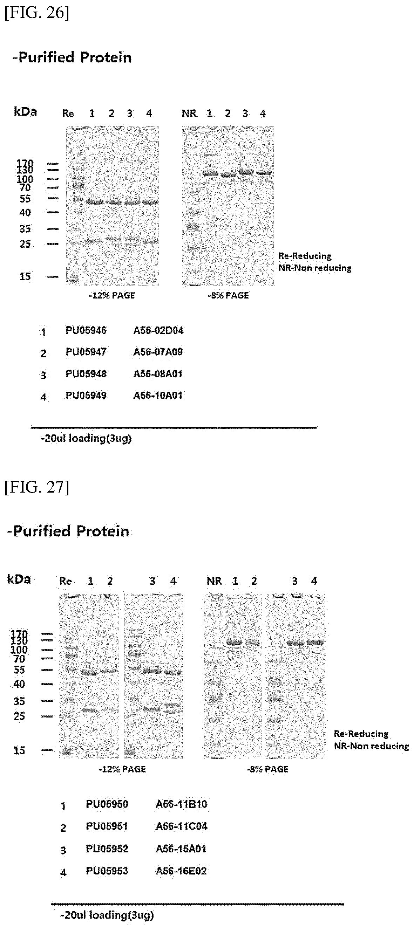

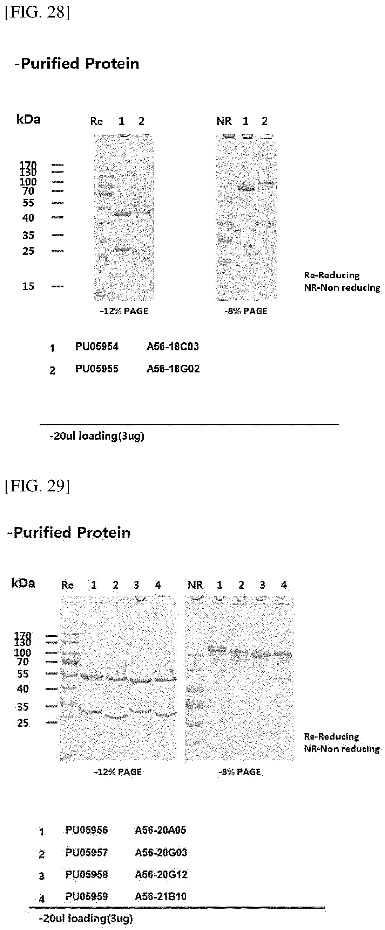

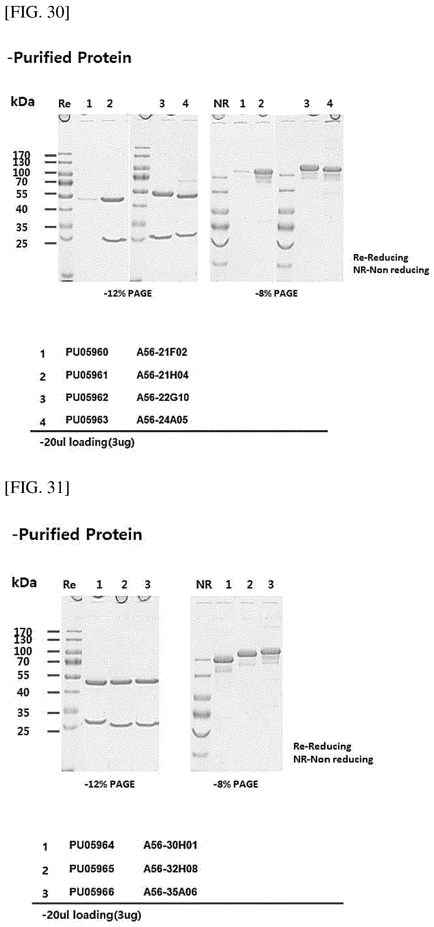

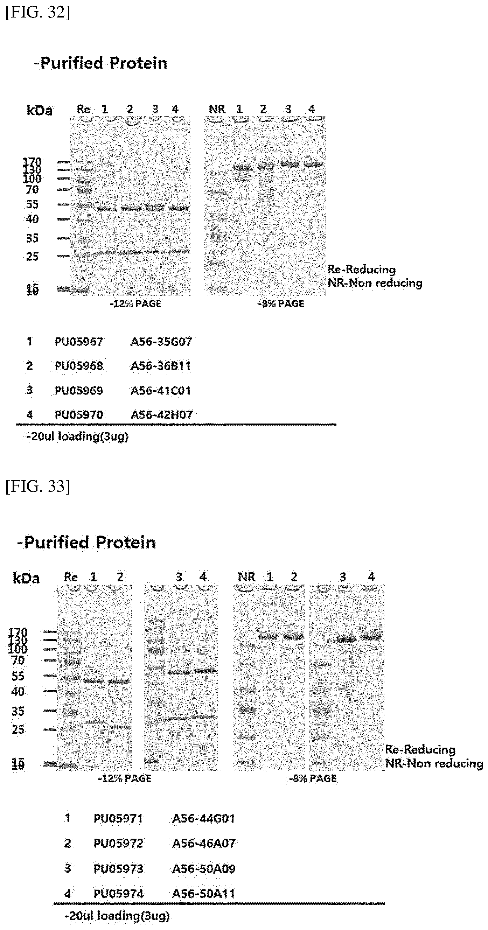

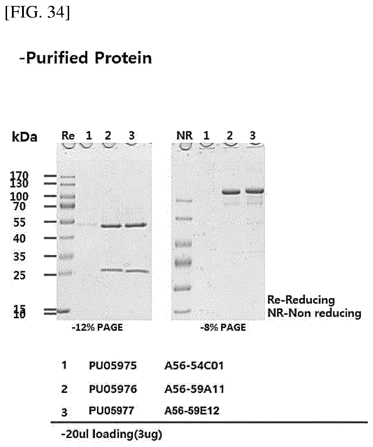

Affinity between each of the thus-produced anti-A56 antibodies and the protein A56 was measured. The results are illustrated in FIGS. 11 to 16. In addition, productivity of each anti-A56 antibody was illustrated in FIGS. 17A to 17C. In addition, FIGS. 18 to 34 illustrate the results obtained by identifying each of the produced anti-A56 antibodies through SDS-PAGE.

### Experimental Example 6. Affinity measurement for anti-A56 antibody

Each well of an immuno-tube was coated with protein A56, and then a blocking process was performed. After the blocking process, each well was allowed to react with an antibody, which was subjected to three-fold serial dilution starting from 100 nM, at room temperature for a predetermined time. Thereafter, washing with PBS was performed three times, and then treatment with a secondary antibody was also performed at room temperature for a predetermined time. Subsequently, affinity of each anti-A56 antibody to the protein A56 was measured at respective concentrations.

### Experimental Example 7. Comparison of amino acid sequence homology among protein A56s for respective vaccinia virus strains

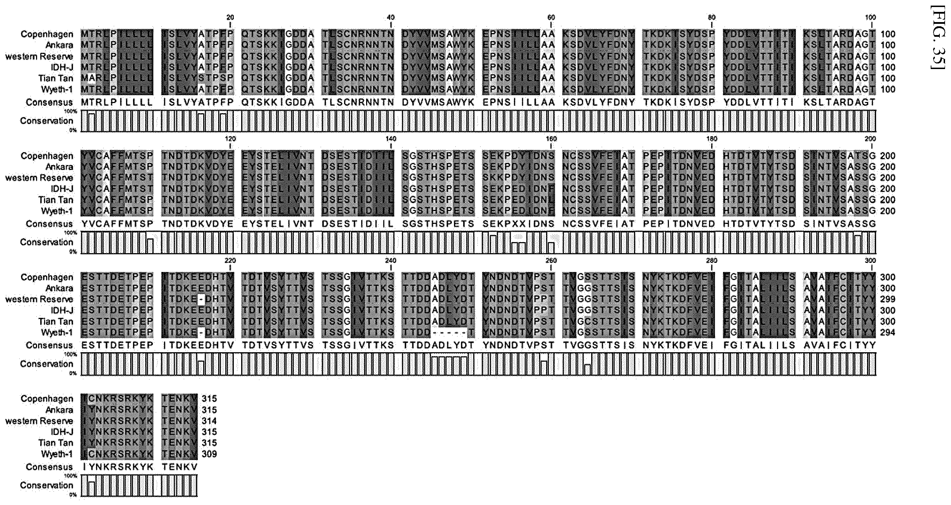

Comparison of amino acid sequence homology among protein A56s for the vaccinia virus strains, that is, Copenhagen, Ankara, Western Reserve (WR), International Health Division-J (IHD-J), Tian Tan, and Wyeth (The New York City Board of Health; NYCBOH). As a result, as illustrated in FIG. 35, it was identified that in the amino acid sequences of the protein A56s of all strains, the amino acids at positions 30 to 90 were identical.

### III. Identification of conformational epitope of protein A56

### Example 1. Distinguishing between linear and conformational epitopes on protein A56

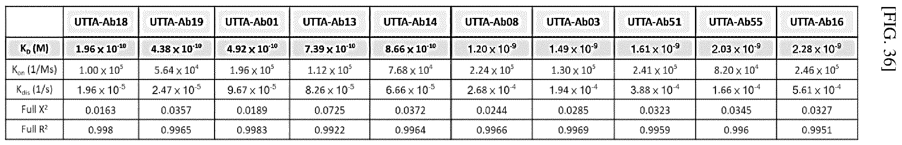

Western blot analysis was performed for ten anti-A56 antibodies (Ab18, Ab19, Ab01, Ab13, Ab14, Ab08, Ab03, Ab51, Ab55, Ab16), which bind to protein A56, to distinguish between linear and conformational epitopes. The ten anti-A56 antibodies were obtained by selecting the top ten antibodies, based on binding affinity, among the sixty-one antibodies that were selected as representative antibodies from a human phage library and specifically bind to protein A56 (FIG. 36).

Specifically, HeLa cell line was infected with OTS-412; and after 24 hours, the Hela cells were collected and lysed to extract total protein. The protein was subjected to a denaturation process, and then loaded onto an SDS-PAGE gel for electrophoresis. After the electrophoresis, the protein was transferred to a polyvinylidene difluoride (PVDF) membrane to react with the ten anti-A56 antibodies, which are primary antibodies, respectively. Then, washing with PBST (PBS: Donginbiotech Co., Ltd, Tween 20: Sigma) was performed. Subsequently, the protein was allowed to react with a secondary antibody (Goat Anti-human IgG Fc cross-absorbed, HRP (Abcam, cat no. ab98624). Washing with PBST was performed again. Then, treatment with a luminescent reagent (ECL, Amersham Biosciences) was performed, and an imaging system (chemiluminescent imaging system) was used for identification. Here, recombinant protein A56 (A56-C-His, 1.56 mg/mL) and commercial anti-A56 antibody (Immune Technology Corp., cat no. IT-012-006M1) were used as positive control groups.

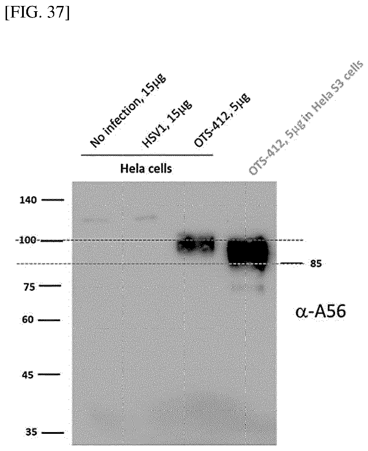

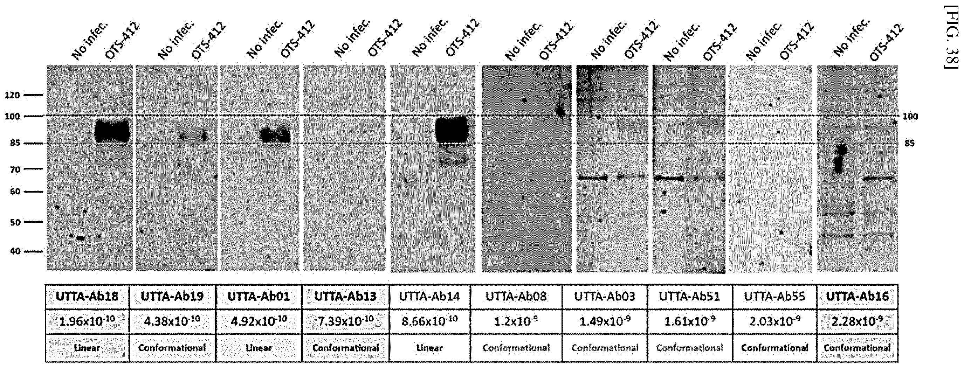

As a result, first, as illustrated in FIG. 37, for the positive control groups, it was found that the protein A56 had a molecular weight corresponding to a band of 85 kD to 100 kD, whereas most of the ten anti-A56 antibodies had a band size or intensity weaker than the positive control groups (FIG. 38). In particular, for Ab03, Ab08, Ab13, Ab19, Ab51, Ab55, and Ab16, many weak bands with small sizes were observed or no band was observed. Absence of a band or a band that is not large in size and intensity means that the epitope is three-dimensionally formed.

Furthermore, for epitope mapping on the seven anti-A56 antibodies, Ab18, Ab13, and Ab16 were preferentially mapped, and then epitopes for the two high-binding antibodies (Ab01 and Ab19) were identified.

### Example 2. Analysis of protein A56 and intact protein of anti-A56 antibody and characterization of binding complex

Prior to epitope mapping, respective samples (A56-C-His, three antibodies (Ab13, Ab16, Ab18) and antigen-antibody binding complexes (A56-C-His/Ab13, A56-C-His/Ab16, A56-C-His/Ab18) were characterized to analyze the sample integrity and the degree of aggregation of the binding complex.

Specifically, for intact mass spectrometry of a control group, protein A56 and each of the anti-A56 antibodies (Ab13, Ab16, Ab18) were mixed in an amount of 5 µl each. Then, 1 µl of 10 µl of the mixture was mixed with acetonitrile/water at a 1:1 ratio and 0.1% TFA (K200 MALDI Kit) in re-crystallized sinapinic acid matrix (10 mg/ml), placed on a MALDI plate (SCOUT 384), and crystallized at room temperature. Then, molecular weight measurement was performed three times by MALDI-MS (mass spectrometer).

Here, mass spectrometry of the complex (A56-C-His/anti-A56 antibody) was performed by cross-linking high-mass matrix-assisted laser desorption ionization-time of flight mass spectrometry (MALDI-TOF MS, Autoflex II MALDI ToF mass spectrometer, Bruker). 9 µl of the antigen-antibody binding complex, which remained after performing the intact mass spectrometry, was mixed with 1 µl of cross-linking reagent (K200 Stabilizer, 2 mg/ml) and allowed to react at room temperature for 180 minutes. Then, MALDI ToF MS was performed using a standard nitrogen laser (MS: Linear and Positive mode, Ion Source 1: 20 KV, 2: 17 kV, Lens: 12kV, Pulse Ion Extraction: 400 ns; HM4: Gain Voltage: 3.14 kV, Acceleration Voltage: 20 kV).

As a result, a molecular weight, which is almost similar to a peak value of the control group, was detected in the sample identified by cross-linking (Table 3), and no non-covalent complex was detected. Thus, it was identified that the samples to be used for analysis were not agglomerated or damaged.

**[Table 3]**

| | **A56-C-His** | **Ab13** | **Ab16** | **Ab18** | **Ab01** | **Ab19** |
|---|---|---|---|---|---|---|
| **Control** | 60.627 | 147.872 | 149.841 | 151.684 | 149.102 | 151.192 |
| **Cross-Link** | 62.921 | 153.432 | 154.374 | 157.272 | 155.922 | 154.921 |

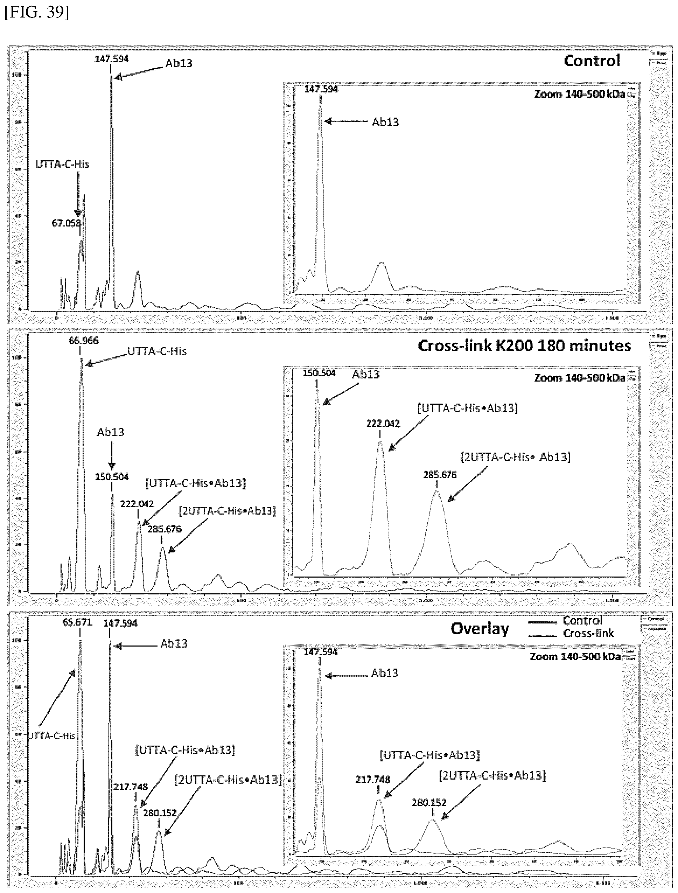

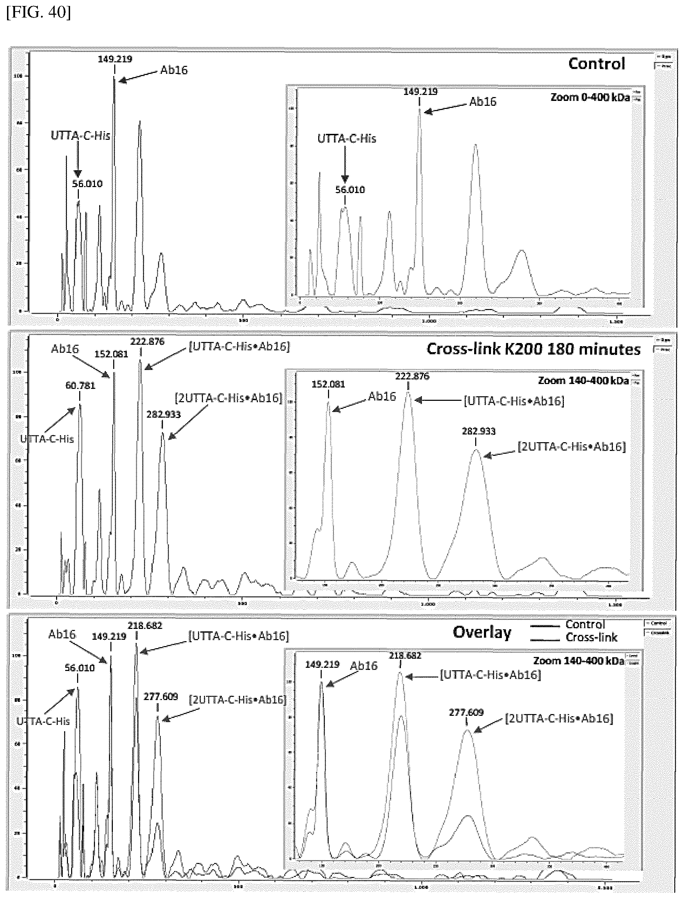

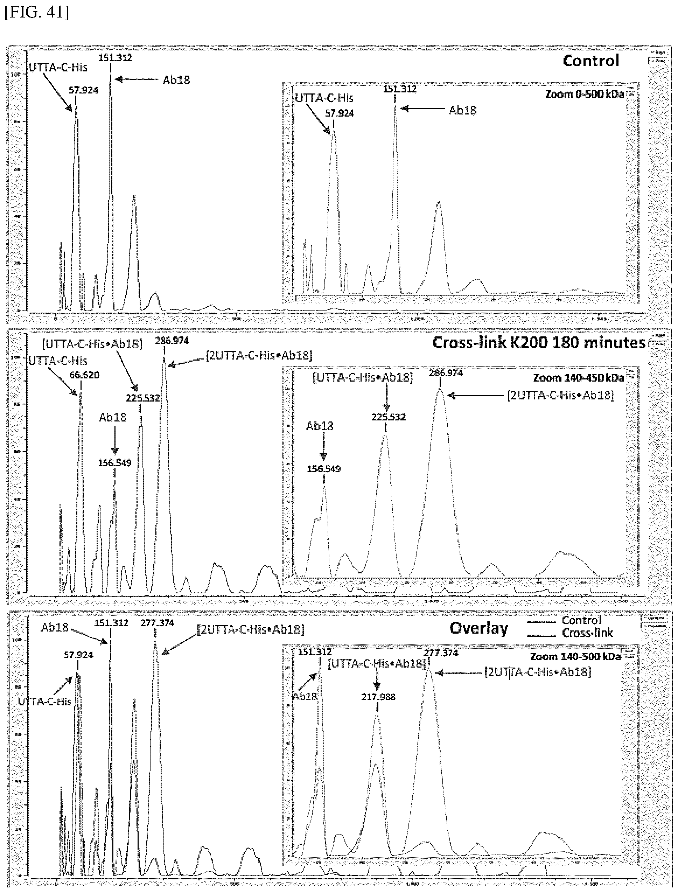

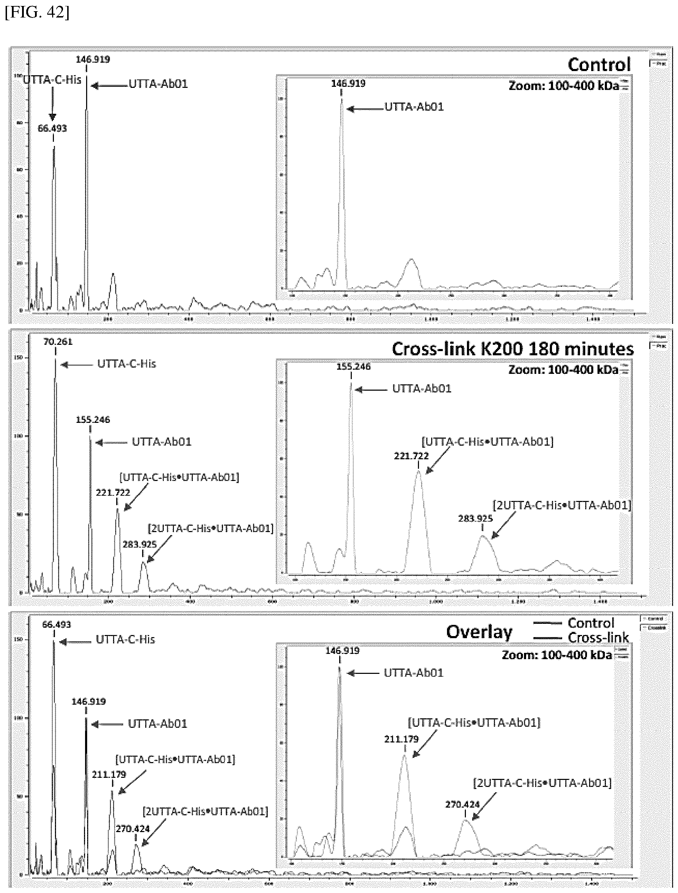

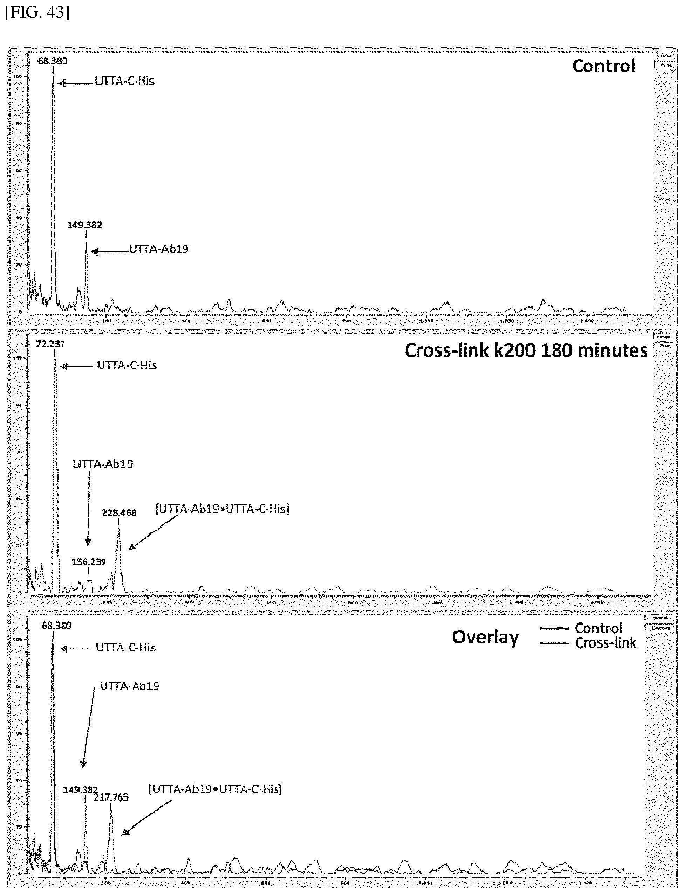

Furthermore, the antigen-antibody binding complexes (A56-C-His/Ab13, A56-C-His/Ab16, A56-C-His/Ab18, A56-C-His/Ab01, A56-C-His/Ab19) were stabilized by treatment with a dedicated reagent for cross-linking of non-covalent protein complex, and then High-Mass MALDI analysis was performed. As a result, two types of peaks were observed. These data were evaluated with Complex Tracker Software to identify the presence of A56·Ab and 2A56·Ab complexes (FIGS. 39 to 43). In addition, the respective samples and the molecular weights of the complexes are shown in Table 4.

**[Table 4]**

| | **Ab13** | | **Ab16** | | **Ab18** | |
|---|---|---|---|---|---|---|
| **Control** | A56-C-His | 67.058 | A56-C-His | 56.010 | A56-C-His | 57.924 |
| | Ab13 | 147.594 | Ab16 | 149.219 | Abl8 | 151.312 |
| **Cross-Link** | [A56-C-His·b13][2A56-C-His·b13] | 222.042 | [A56-C-His·b16] | 222.876 | [A56-C-His·b18] | 225.532 |
| | | 285.676 | [2A56-C-His·b16] | 282.933 | [2A56-C-His·b18] | 286.974 |
| **Comple x Tracker** | [A56-C-His·b13] | 217.748 | [A56-C-His·b16] | 218.682 | [A56-C-His·b18] | 217.988 |
| | [2A56-C-His·b13] | 280.152 | [2A56-C-His·b16] | 277.609 | [2A56-C-His·b18] | 277.374 |

| **Control** | **Ab01** | | **Ab19** | | | |
|---|---|---|---|---|---|---|
| **Cross-Link** | A56-C-His | 66.493 | A56-C-His | 68.380 | | |
| | AbOl | 146.919 | Abl9 | 149.382 | | |
| **Comple x Tracker** | [A56-C-His·b01][2A56-C-His·b01] | 221.722 | [A56-C-His·b19] | 228.468 | | |
| | | 283.925 | | | | |
| | [A56-C-His·b01] [2A56-C-His·b01] | 211.179 | [A56-C-His·b19] | 217.765 | | |
| | | 270.424 | | | | |

### Example 3. Epitope mapping of protein A56 to five anti-A56 antibodies

The amino acid sequence numbering in A56 as shown in Examples 3.2 to 3.7 and the drawings mentioned therein is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).

### Example 3.1. Sequencing of protein A56

For epitope mapping of the protein A56, a sequence of the protein A56 was first identified. The protein A56 was fragmented into peptides by treatment with five proteolytic enzymes (trypsin, chymotrypsin, ASP-N, elastase, and thermolysin). Then, for the digested peptides, mass fingerprint information was obtained using LTQ-Orbitrap MS (mass spectrometry), and comparative analysis was performed on whether or not the information matches the sequence information of the existing protein A56.

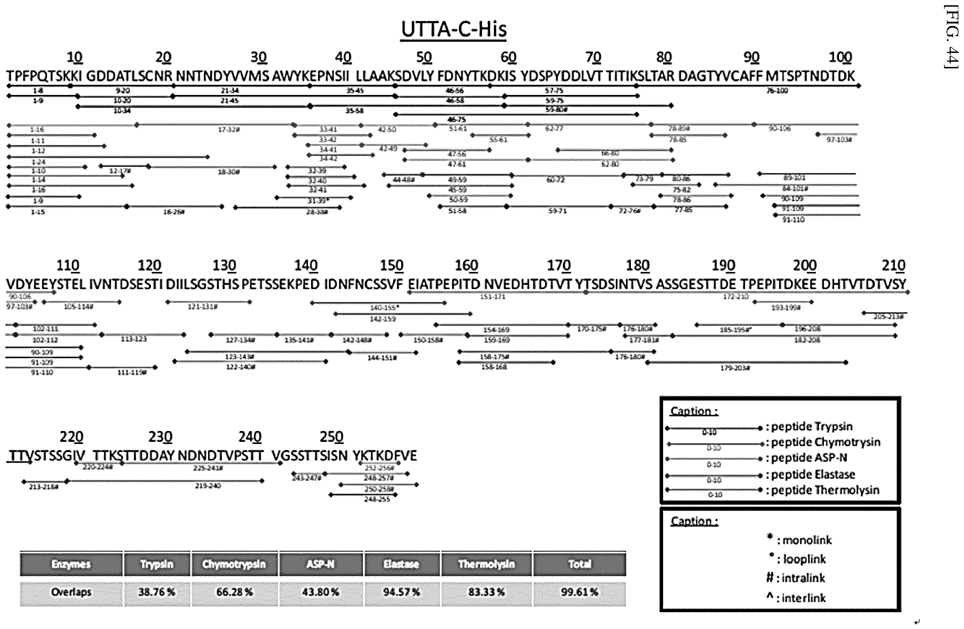

As a result, as illustrated in FIG. 44, it was identified that the amino acid fragment obtained by digestion of the protein A56 with trypsin had a sequence coverage of 38.76%; the amino acid fragment obtained by digestion of the protein A56 with chymotrypsin had a sequence coverage of 66.28%; the amino acid fragment obtained by digestion of the protein A56 with ASP-N had a sequence coverage of 43.80%; the amino acid fragment obtained by digestion of the protein A56 with elastase had a sequence coverage of 94.57%; and the amino acid fragment obtained by digestion of the protein A56 with thermolysin had a sequence coverage of 83.33%. Taking all sequences identified by the five proteolytic enzymes into consideration, it was identified that they were 99.61% identical, in terms of sequence information, with the existing protein A56.

### Example 3.2. Epitope mapping of protein A56 to anti-A56 antibody (Ab13)

A sample of the antigen-antibody binding complex (A56-C-His/Ab13) was subjected to combined treatment with deuterated cross-linking and various proteolytic enzymes, so that the sample underwent alkylation and reduction. Subsequently, molecular weight data were obtained therefrom using High-Mass MALDI MS and nLC-LTQ-Orbitrap MS, and then analyzed using software (XQuest, Stavrox).

As a result, a sequence of the site where the protein A56 and each anti-A56 antibody (Ab13) are in direct contact with each other was identified using cross-linking. Based on this, the epitope and paratope as shown in Table 5 were identified as follows.

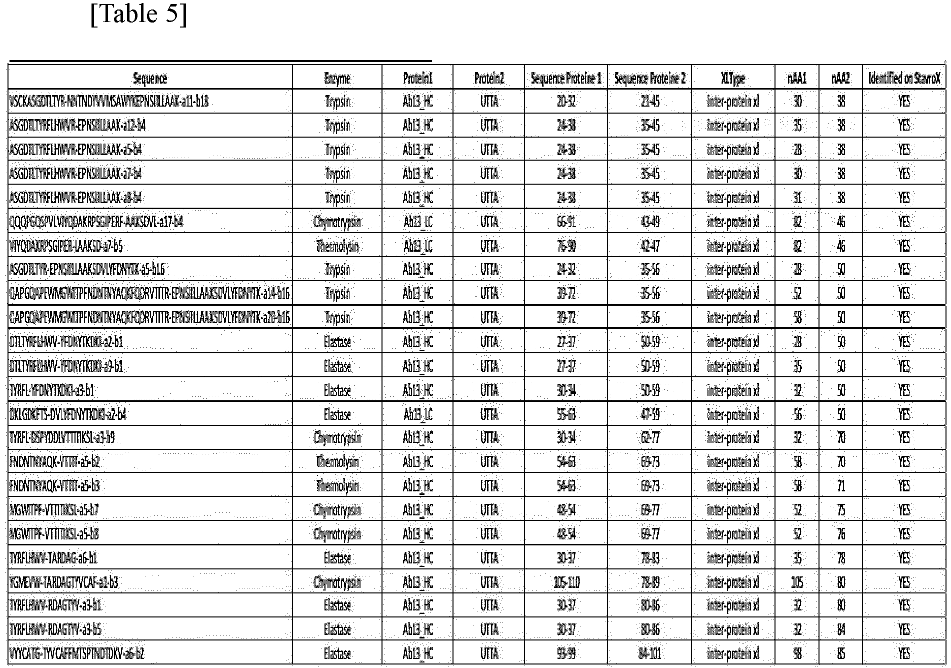

As shown in Table 5, it was identified that the epitope on the site where A56-C-His and the anti-A56 antibody (Ab13) bind was at positions 38, 46, 50, 70, 71, 75, 76, 78, 80, 84, and 85 in the amino acid sequence of A56-C-His.

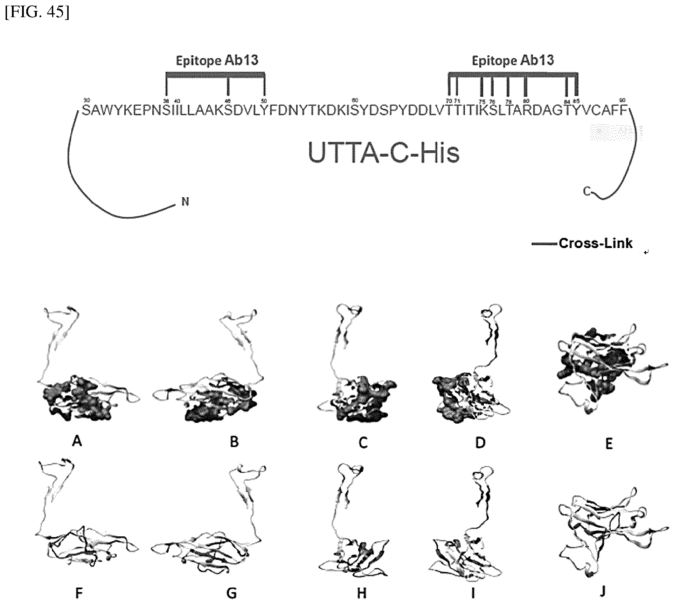

Specifically, as illustrated in FIG. 45, the epitope positions of amino acid residues at positions 30 to 90 in the amino acid sequence of A56-C-His are indicated in red (38, 46, 50, 70, 71, 75, 76, 78, 80, 84, 85); and in a case where a protein structure of A56-C-His (at positions 2 to 148 in the amino acid sequence) was represented in silico using Swiss-Model software, the site where Ab13 and A56-C-His bind is indicated in blue. Among the amino acid sequence of A56-C-His, it was identified that the site identified as a binding site corresponds to positions 38 to 50 (SIILLAAKSDVLY) and positions 70 to 85 (TTITIKSLTARDAGTY).

### Example 3.3. Epitope mapping of protein A56 to anti-A56 antibody (Ab16)

A sample of the antigen-antibody binding complex (A56-C-His/Ab16) was subjected to combined treatment with deuterated cross-linking and various proteolytic enzymes, so that the sample underwent alkylation and reduction. Subsequently, molecular weight data were obtained therefrom using High-Mass MALDI MS and nLC-LTQ-Orbitrap MS, and then analyzed using software (XQuest, Stavrox).

A sequence of the site where the protein A56 and each anti-A56 antibody (Ab16) are in direct contact with each other was identified using cross-linking. Based on this, the epitope and paratope as shown in Table 6 were identified as follows.

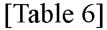

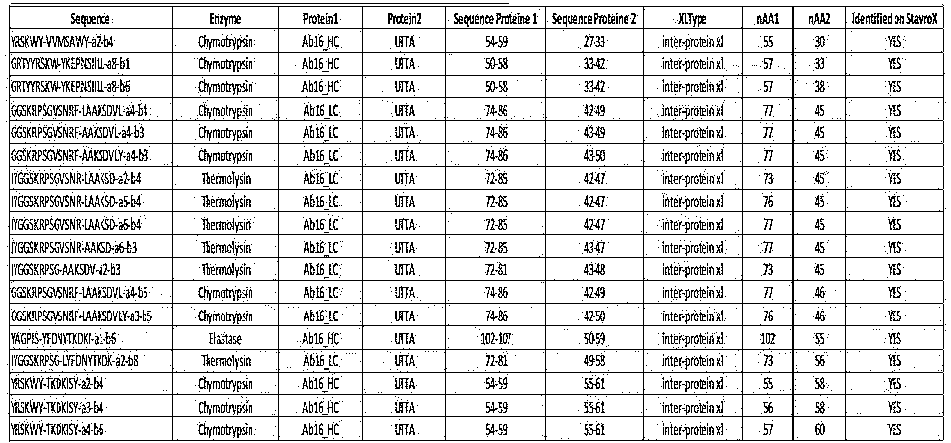

As shown in Table 6, it was identified that the epitope on the site where A56-C-His and the anti-A56 antibody (Ab16) bind was at positions 30, 33, 38, 45, 46, 55, 56, 58, and 60 in the amino acid sequence of A56-C-His.

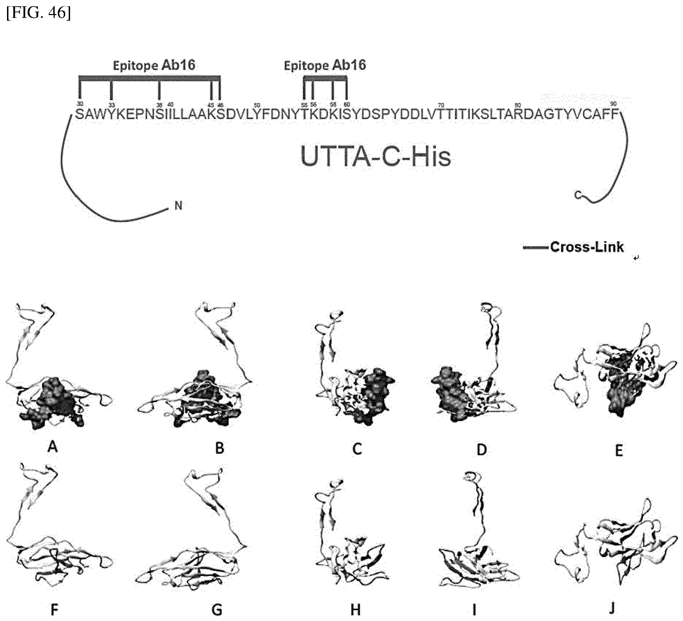

Specifically, as illustrated in FIG. 46, the epitope positions of amino acid residues at positions 30 to 90 in the amino acid sequence of A56-C-His are indicated in red (30, 33, 38, 45, 46, 55, 56, 58, 60); and in a case where a protein structure of A56-C-His (at positions 2 to 148 in the amino acid sequence) was represented in silico using Swiss-Model software, the site where Ab16 and A56-C-His bind is indicated in blue. Among the amino acid sequence of A56-C-His, it was identified that the binding site corresponds to positions 30 to 46 (SAWYKEPNSIILLAAKS) and positions 55 to 60 (TKDKIS).

### Example 3.4. Epitope mapping of protein A56 to anti-A56 antibody (Ab18)

A sample of the antigen-antibody binding complex (A56-C-His/Ab18) was subjected to combined treatment with deuterated cross-linking and various proteolytic enzymes, so that the sample underwent alkylation and reduction. Subsequently, molecular weight data were obtained therefrom using High-Mass MALDI MS and nLC-LTQ-Orbitrap MS, and then analyzed using software (XQuest, Stavrox).

A sequence of the site where the protein A56 and each anti-A56 antibody (Ab18) are in direct contact with each other was identified using cross-linking. Based on this, the epitope and paratope as shown in Table 7 were identified as follows.

**[Table 7]**

| **Sequence** | **Enzyme** | **Protein1** | **Protein2** | **Sequence Proteine 1** | **Sequence Proteine 2** | MLType | **nAA1** | **nAA2** | **Identified on StavroX** |
|---|---|---|---|---|---|---|---|---|---|
| AVYYCARWG-LAAKSD-a7-b5 | Themolysin | Ab18_HC | UΠA | 92.100 | 42-47 | inter-protein xl | 98 | 46 | YES |
| LSCAASGFTFSTYGMTWVR-SDYLYLYFDNYTK-a13-b5 | Trypsin | Ab18_HC | UΠA | 20-38 | 46-56 | inter-protein xl | 32 | 50 | YES |
| LLIYKASDLESGVPSR-SDVLYFDNYTK-a5-b10 | Trypsin | Ab18_LC | UΠA | 68-83 | 46-56 | inter-protein xl | 72 | 55 | YES |
| TFSTYGMTW-TKDKISY-a5-b2 | Cbymotrypsin | Ab18_HC | UΠA | 28-36 | 55-61 | inter-protein xl | 32 | 56 | YES |
| TFSTYGMTW-TKDKISY-a3-b6 | Chymotrypsin | Ab18_HC | UΠA | 28-36 | 55-61 | inter-protein xl | 30 | 60 | YES |
| ISGGGSSTNYADS-LVTT-a8-b4 | Thermolysin | Ab18_HC | UΠA | 51-63 | %73 | inter-proteinxl | 58 | 71 | YES |
| ASGFTFSTYG-VTTTKS-a9-b7 | Thermolysin | Ab18_HC | UΠA | 24-33 | 69-76 | inter-protein xl | 32 | 75 | YES |
| SPWLAWYQQKPG-TIKSL-a1-b3 | Elastase | Ab18_LC | UΠA | 51-63 | 73-77 | inter-protein xl | 52 | 75 | YES |
| FSTYGMTW-IKSLT-a3-b3 | Thermolysin | Ab18_HC | UΠA | 29-36 | 14-18 | inter-proteinxl | 31 | 76 | YES |

As shown in Table 7, it was identified that the epitope on the site where A56-C-His and the anti-A56 antibody (Ab18) bind was at positions 46, 50, 55, 56, 60, 71, 75, and 76 in the amino acid sequence of A56-C-His.

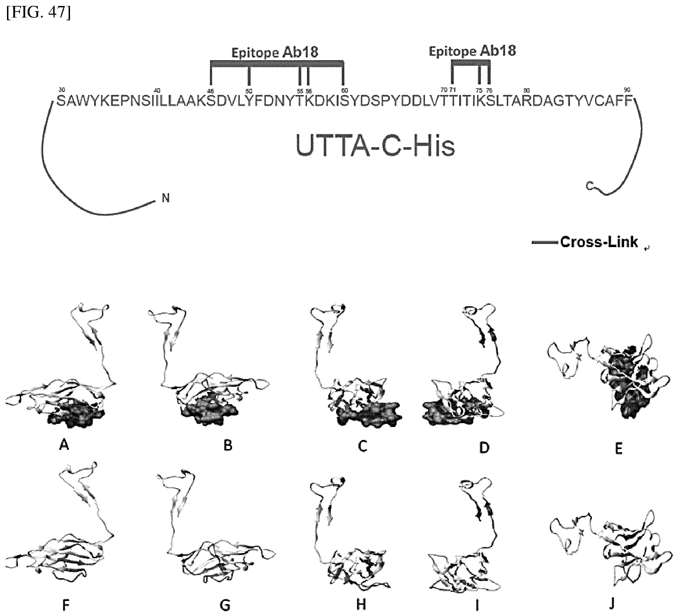

Specifically, as illustrated in FIG. 47, the epitope positions of amino acid residues at positions 30 to 90 in the amino acid sequence of A56-C-His are indicated in red (46, 50, 55, 56, 60; 71, 75, 76); and in a case where a protein structure of A56-C-His (at positions 2 to 148 in the amino acid sequence) was represented in silico using Swiss-Model software, the site where Ab18 and A56-C-His bind is indicated in blue. Among the amino acid sequence of A56-C-His, it was identified that the site identified as a binding site corresponds to positions 46 to 60 (SDVLYTKDKIS) and positions 71 to 76 (TITIKS).

### Example 3.5. Epitope mapping of protein A56 to anti-A56 antibody (Ab01)

A sample of the antigen-antibody binding complex (A56-C-His/Ab01) was subjected to combined treatment with deuterated cross-linking and various proteolytic enzymes, so that the sample underwent alkylation and reduction. Subsequently, molecular weight data were obtained therefrom using High-Mass MALDI MS and nLC-LTQ-Orbitrap MS, and then analyzed using software (XQuest, Stavrox).

A sequence of the site where the protein A56 and each anti-A56 antibody (Ab01) are in direct contact with each other was identified using cross-linking. Based on this, the epitope and paratope as shown in Table 8 were identified as follows.

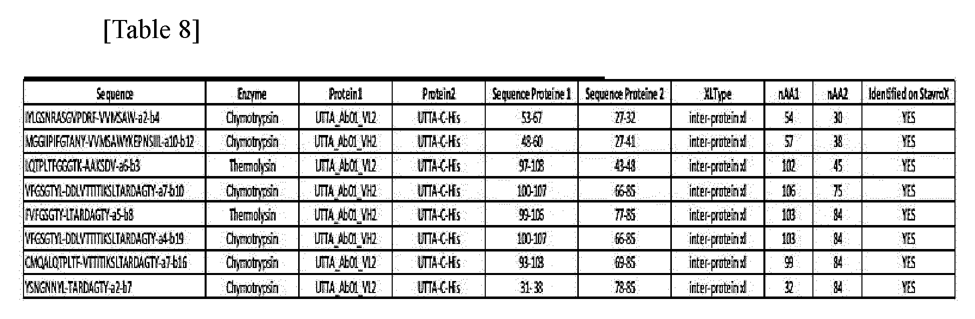

As shown in Table 8, it was identified that the epitope on the site where A56-C-His and the anti-A56 antibody (Ab01) bind was at positions 30, 38, 45, 75, and 84 in the amino acid sequence of A56-C-His.

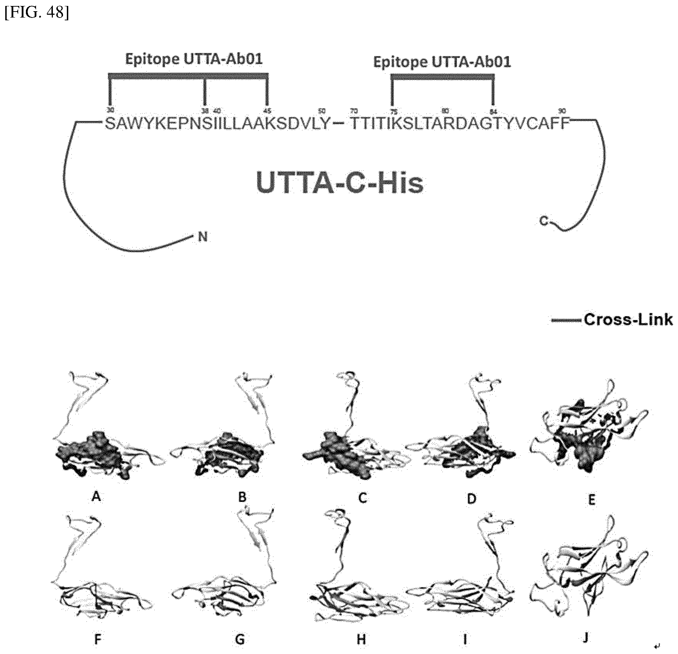

Specifically, as illustrated in FIG. 48, the epitope positions of amino acid residues at positions 30 to 90 in the amino acid sequence of A56-C-His are indicated in red (30, 38, 45, 75, 84); and in a case where a protein structure of A56-C-His (at positions 2 to 148 in the amino acid sequence) was represented in silico using Swiss-Model software, the site where Ab01 and A56-C-His bind is indicated in blue. Among the amino acid sequence of A56-C-His, it was identified that the site identified as a binding site corresponds to positions 30 to 45 (SAWYKEPNSIILLAAK) and positions 75 to 84 (KSLTARDAGT).

### Example 3.6. Epitope mapping of protein A56 to anti-A56 antibody (Ab19)

A sample of the antigen-antibody binding complex (A56-C-His/Ab19) was subjected to combined treatment with deuterated cross-linking and various proteolytic enzymes, so that the sample underwent alkylation and reduction. Subsequently, molecular weight data were obtained therefrom using High-Mass MALDI MS and nLC-LTQ-Orbitrap MS, and then analyzed using software (XQuest, Stavrox).

A sequence of the site where the protein A56 and each anti-A56 antibody (Ab19) are in direct contact with each other was identified using cross-linking. Based on this, the epitope and paratope as shown in Table 9 were identified as follows.

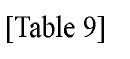

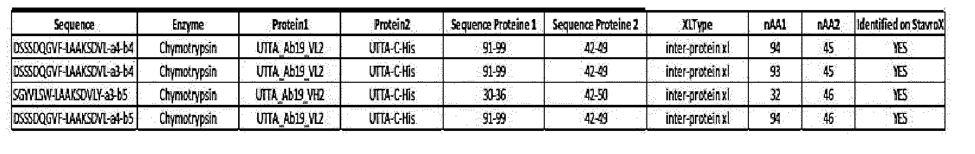

As shown in Table 9, it was identified that the epitope on the site where A56-C-His and the anti-A56 antibody (Ab19) bind was at positions 45 and 46 in the amino acid sequence of A56-C-His.

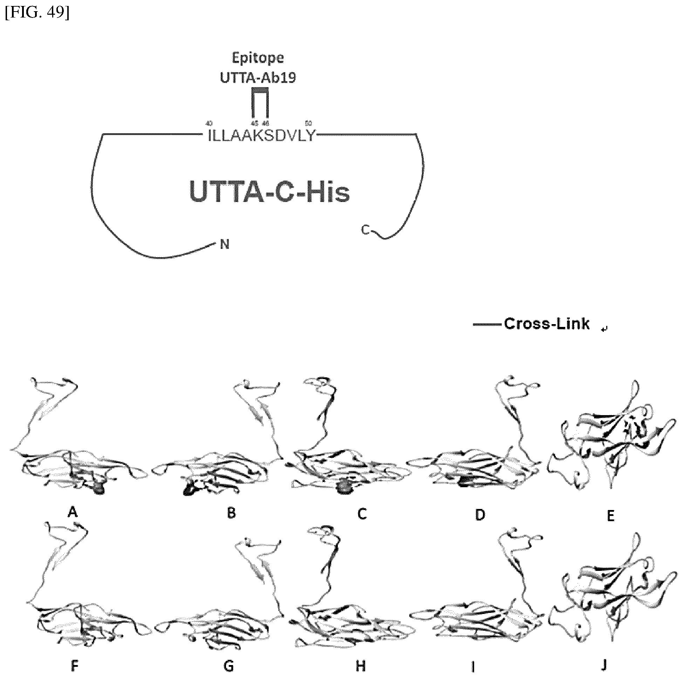

Specifically, as illustrated in FIG. 49, the epitope positions of amino acid residues at positions 30 to 90 in the amino acid sequence of A56-C-His are indicated in red (45,46); and in a case where a protein structure of UTTA-C-His (at positions 2 to 148 in the amino acid sequence) was represented in silico using Swiss-Model software, the site where Ab19 and A56-C-His bind is indicated in blue. Among the amino acid sequence of A56-C-His, it was identified that the site identified as a binding site corresponds to positions 45 and 46 (KS).

### Example 3.7. Comparison of epitope mapping of protein A56 to anti-A56 antibodies (Abl3, Ab16, Ab18, Ab01, Ab19)

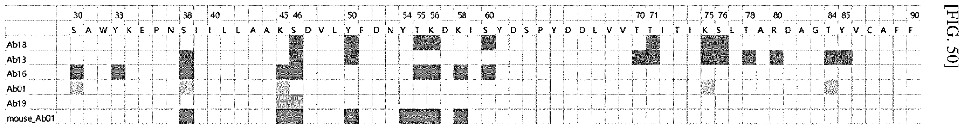

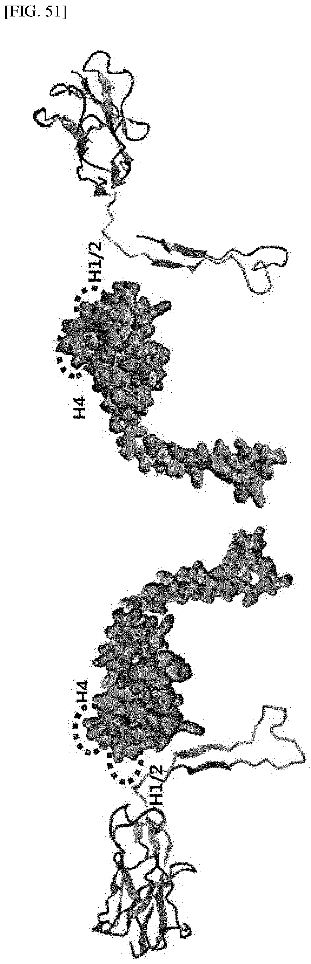

The results obtained by epitope mapping in Examples 3.2 to 3.6 were compared and illustrated in FIGS. 50 and 51. As a result, it was identified that in the amino acid sequence of the protein A56, the epitope was mapped to positions 30 to 85 corresponding to an IgG-like domain region.

In addition, the IgG-like domain of the protein A56 was modeled for primary 3D protein structure with SWISS-MODEL and structurally analyzed with iCn3D. As a result, it was identified that the domain consists of a total of 8 sheets (green), 4 helices (red), and 7 loops (blue). In particular, it was identified that Helices 1 and 2 were structurally folded right next to Helix 3.

Structural analysis of the epitope of A56 involved in binding to anti-A56 antibodies (Ab13, Ab16, Ab18, Ab01, Ab19) shows that the epitope consists of lysine (K/Lys) and arginine (R/Arg) which are basic amino acid residues having a positive charge, serine (S/Ser) and threonine (T/Thr) which are nucleophilic amino acid residues, and tyrosine (Y/Tyr) which is aromatic. The basic amino acid easily forms a hydrogen bond by a positively charged side chain, and the nucleophilic amino acid and aromatic amino acid may be partially negatively charged by an amino acid located nearby to form a hydrogen bond. The main physicochemical properties of the amino acid residues, which crosslink in the protein A56 and the anti-A56 antibody, are summarized.

### Example 4. Analysis of paratope of protein A56 for five anti-A56 antibodies

The amino acid sequence numbering in A56 as shown in Examples 4.1 to 4.5 and the drawings mentioned therein is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).

### Example 4.1. Paratope analysis of protein A56 for anti-A56 antibody (Ab13)

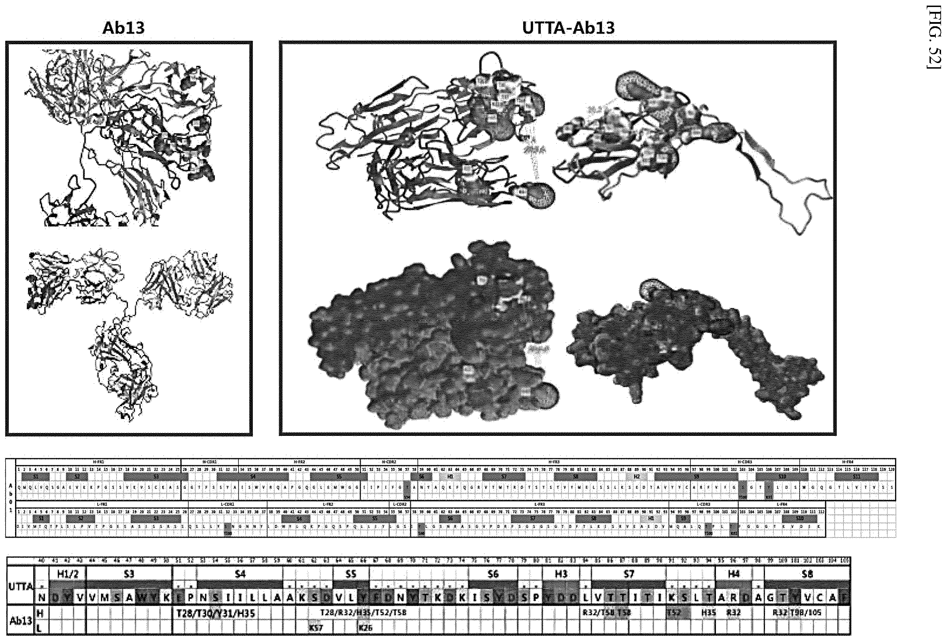

Ab13 was modeled for primary 3D protein structure with SWISS-MODEL software, and structural analysis of the amino acids that participate in binding was performed with iCn3D. As a result, it was analyzed that the amino acids serine (S/Ser) and threonine (T/Thr), which are nucleophilic, and tyrosine (Y/Tyr), which is aromatic, are distributed in the terminal exposed portion of the heavy chain, and thus have strong binding affinity with lysine (K91) and arginine (R96), which have a strong positive charge, in the protein A56; and lysine (K26) having a strong positive charge is located in CDR1 of the light chain and lysine (K57) is located in the loop between Sheet 5 (S5) and Sheet 6 (S6), and the nucleophilic amino acids serine (S) and tyrosine (Y/Tyr) in the protein A56 have binding affinity with the lysine (K26, K57) (FIG. 52).

### Example 4.2. Paratope analysis of protein A56 for anti-A56 antibody (Ab16)

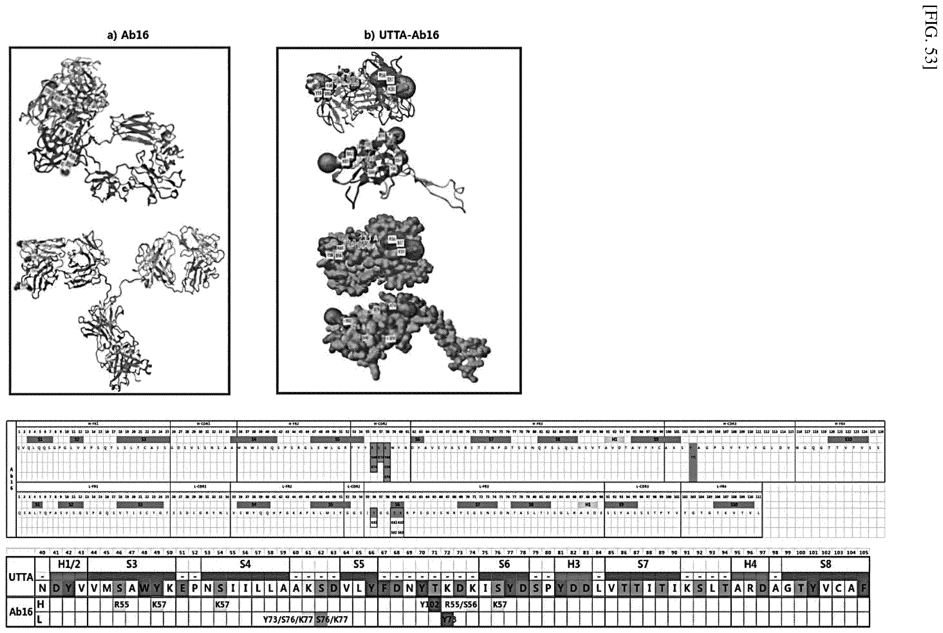

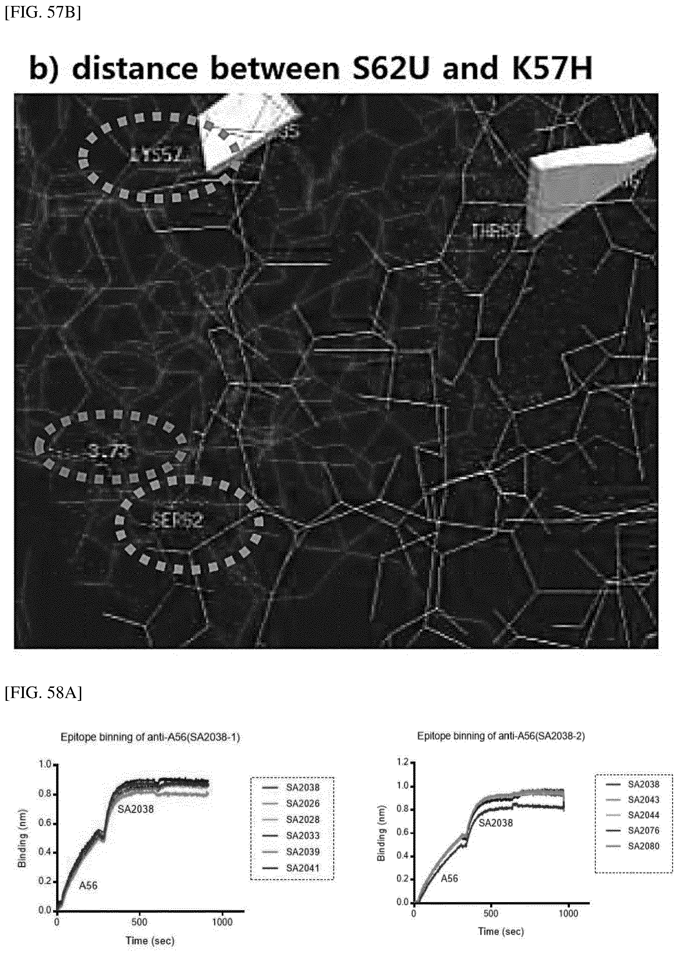

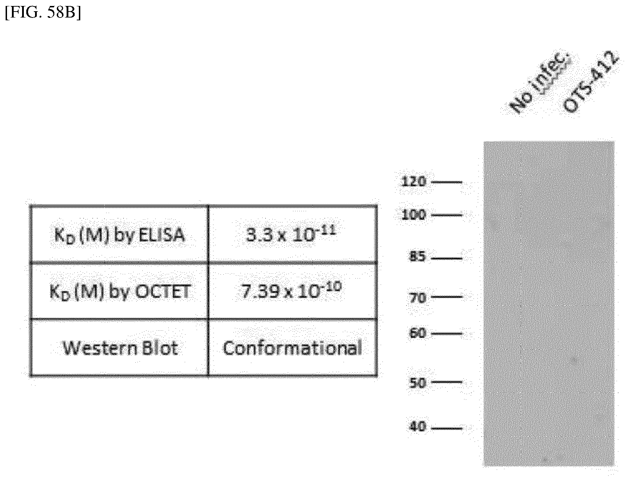

Ab16 was modeled for primary 3D protein structure with SWISS-MODEL software, and structural analysis of the amino acids that participate in binding was performed with iCn3D. As a result, it was analyzed and predicted that arginine (R56) and lysine (K58) in CDR2 located at the loop between Sheet 5 (S5) and Sheet 6 (S6), which is a terminal exposed portion of the heavy chain, have a stronger positive charge (blue mash in FIG. 58) due to the nucleophilic amino acid serine (S57) located between the two amino acids, which is advantageous in that binding to serine (S46, S54), which is a nucleophilic amino acid, in the protein A56 proceeds preferentially; and this antibody has a high level of binding affinity with the nucleophilic amino acids serine (S62) and lysine (K61) in the protein A56 due to lysine (K60) having a strong positive charge, which is located on Sheet 6 (S6) of FR3 of the light chain, and the nucleophilic amino acid serine (S) (FIG. 53).

### Example 4.3. Paratope analysis of protein A56 for anti-A56 antibody (Ab18)

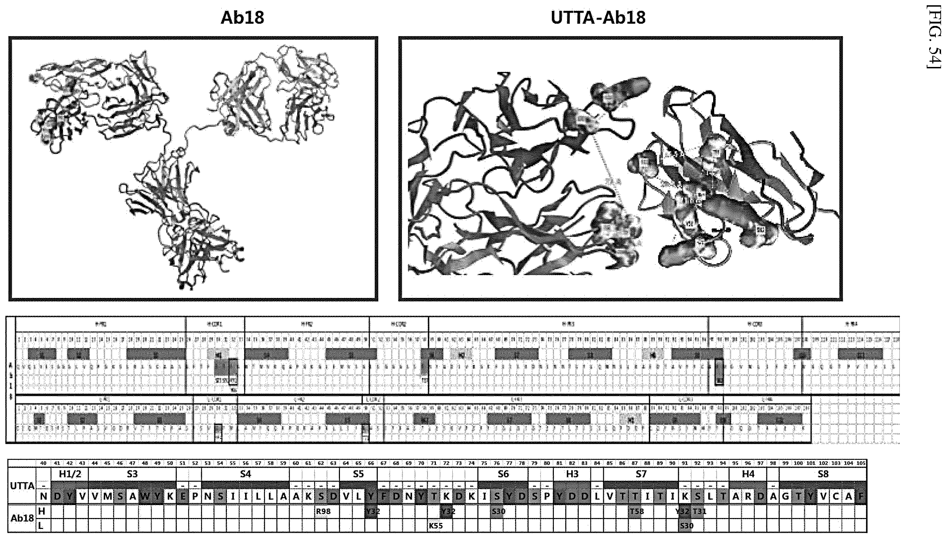

Ab18 was modeled for primary 3D protein structure with SWISS-MODEL software, and structural analysis of the amino acids that participate in binding was performed with iCn3D. As a result, it was predicted that as the amino acids serine (S/Ser) and threonine (S/Ser), which are nucleophilic, and tyrosine (Y/Tyr), which is aromatic, are distributed in the CDR1 helix exposed portion of the heavy chain, this portion has a relatively negative charge, and thus binds to lysine (K72) having a strong positive charge in the protein A56; and it was identified that arginine (R98) having a strong positive charge is located in CDR3. In addition, it was analyzed that the nucleophilic amino acid serine (S30) in CDR1 of the light chain binds to lysine (K91) having a strong positive charge in the protein A56, and lysine (K50) located between Sheet 5 (S5) and the loop binds to tyrosine (Y/Tyr, T71) in the protein A56 (FIG. 54).

### Example 4.4. Paratope analysis of protein A56 for anti-A56 antibody (Ab01)

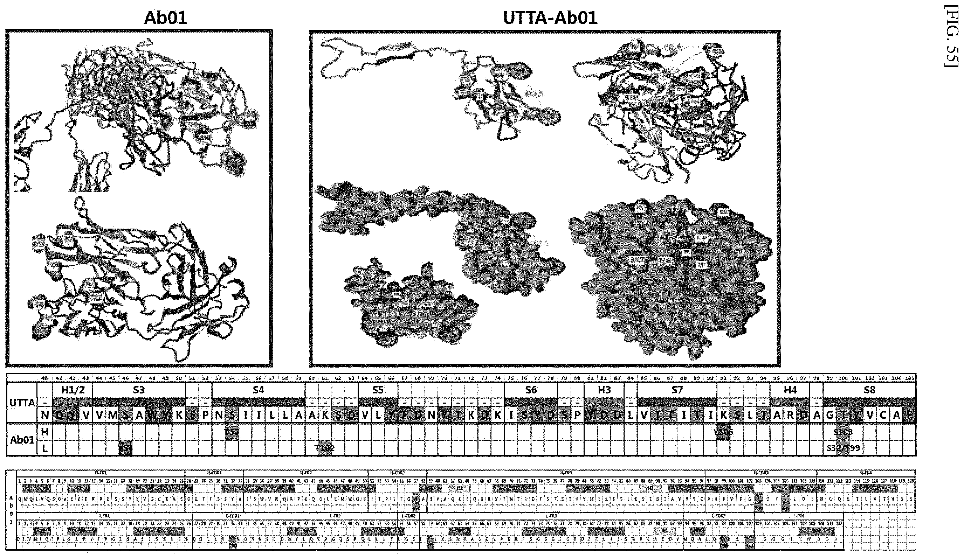

Ab01 was modeled for primary 3D protein structure with SWISS-MODEL software, and structural analysis of the amino acids that participate in binding was performed with iCn3D. As a result, it was analyzed that the amino acids serine (S/Ser) and threonine (T/Thr), which are nucleophilic, and tyrosine (Y106), which is aromatic, are distributed in the terminal exposed portion of the heavy chain, and thus have binding affinity with lysine (K91) having a strong positive charge in the protein A56; and the nucleophilic amino acids serine (S) and threonine (T102) are mainly located in CDRs of the light chain, and in particular, threonine (T102) has binding affinity with lysine (K61) having a strong positive charge in the protein A56 (FIG. 55).

### Example 4.5. Paratope analysis of protein A56 for anti-A56 antibody (Ab19)

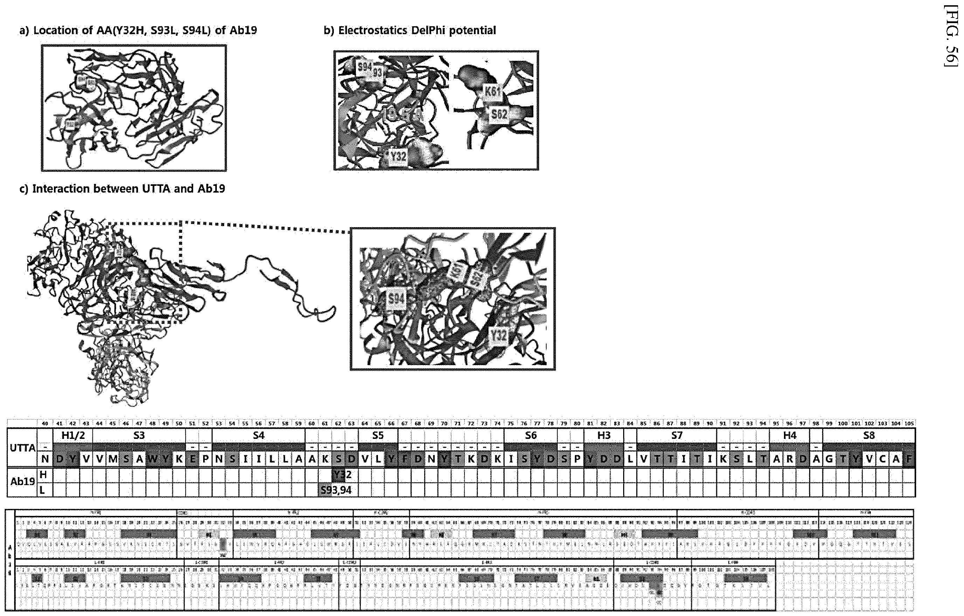

Ab19 was modeled for primary 3D protein structure with SWISS-MODEL software, and structural analysis of the amino acids that participate in binding was performed with iCn3D. As a result, it was analyzed that this antibody has relatively low binding affinity due to simple hydrogen bonding caused by serine (S93, S94), and due to the binding sites (K61, S62) present in the loop located between Sheet 4 (S4) and Sheet 5 (S5) in the protein A56, this antibody may not overlap, in terms of an epitope, with the other anti-56 antibodies (FIG. 56).

### Example 5. Analysis of binding between anti-A56 antibody and protein A56

The amino acid sequence numbering in A56 as shown in Examples 5.1.1 to 5.5.3 and the drawings mentioned therein is made based on a sequence obtained by excluding N-terminal amino acids 1-16 from the amino acid sequence represented by SEQ ID NO: 1 (for example, serine at position 30 corresponds to serine at position 46 in the amino acid sequence represented by SEQ ID NO: 1).

### Example 5.1.1. Analysis of electrostatic force of binding between anti-A56 antibody (Ab13) and protein A56

To analyze the electrostatic force of binding between the anti-A56 antibody (Ab13) and the protein A56, DelPhi potential, which is a scientific application that calculates the electrostatic potential and the corresponding electrostatic energy in and around macromolecules, was used. DelPhi potential is an analytical method commonly used to visualize electrostatic changes along the surface of proteins or other macromolecules and to calculate the electrostatic components of various energies, in which effects of ionic strength-mediated screening are incorporated by evaluating the Poisson-Boltzmann equation at a finite number of points in a three-dimensional lattice box. Here, blue and red indicate positive and negative potentials, respectively, and field lines indicate direction and strength of the electrostatic force surrounding the protein.

Specifically, the basic amino acid lysine (K91), which has a positive charge of strong DelPhi surface electrostatic potential, in the protein A56 excited a hydroxyl group of serine (S92), which is a residue right next thereto, so that an electrostatic force was generated and a relatively low level of DelPhi surface electrostatic potential was strongly positively charged. Then, for K26 and K57 of the light chain in Ab13, a relatively strongly positively charged DelPhi surface electrostatic potential was formed by negatively charged D25 and D55 therearound.

In addition, T52 and T58 of the heavy chain in Ab13 were highly exposed to the outside due to the nearby hydrophobic residue I51 (isoleucine), and F54 (phenylalanine) that is aromatic and free of a hydrophilic reactive group, and a high level of DelPhi surface electrostatic potential thereof was strongly negatively charged due to negatively charged D56.

As a result, the electrostatic properties as shown in Table 10 were identified.

**[Table 10]**

| | protein | Amino acid | DelPhi potential | →Ab13, →UTTA |
|---|---|---|---|---|
| Ab13 | UTTA | K91/S92 | Strong + | T52 H |
| | | R96 | Strong + | R32H |
| | | S62 | Mild - | K57L |
| | | Y66 | Mild - | K26L |
| | HC | R32 | Strong + | R96 |
| | | T52/T58 | Strong - | K91/S92 |
| | LC | K26 | Strong + + | Y66 |
| | | K57 | Strong + + | S62 |

In the table, the letter (H or L), which follows the amino acid position number indicated in the rightmost column, means heavy chain (H) or light chain (L).

### Example 5.1.2. Analysis of binding distance between anti-A56 antibody (Ab13) and protein A56

In order to analyze a binding distance between the anti-A56 antibody (Abl3) and the protein A56, structural alignment of the protein A56 with Ab13 was inferred using Swiss-PdbViewer (4.1.0), and active sites or other relevant portions were compared to predict H-bonds, angles, and distances between atoms.

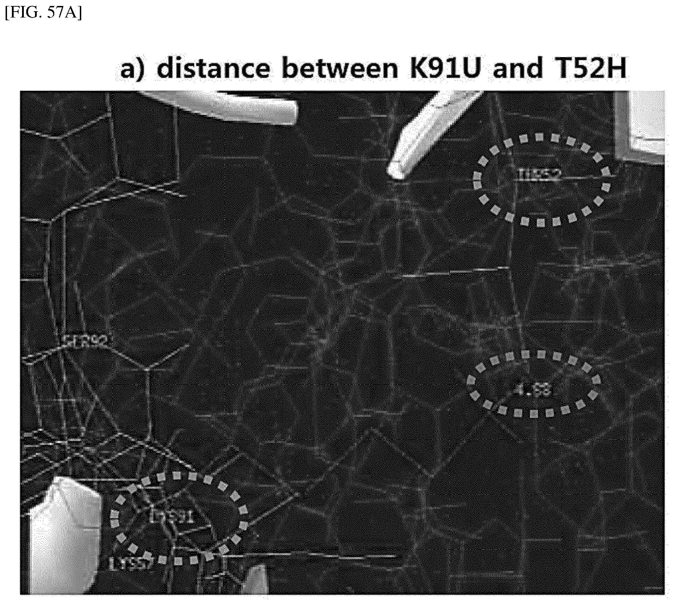

As a result, an NH residue, that is, a terminal functional group of K91, which becomes positive with a strong positive charge, in the protein A56 formed a hydrogen bond with O- of T52, which becomes negative with an appropriate level of negative charge, of the heavy chain in Ab13, and a distance therebetween was measured to be 4.88 Å (FIG. 57A). In addition, an NH residue, that is, a terminal functional group of K57, which becomes positive with a strong positive charge, of the heavy chain in Ab13 formed a hydrogen bond with O- of S62, which becomes negative with an appropriate level of negative charge, in the protein A56, and a distance therebetween was measured to be 3.73 Å (FIG. 57B).

It was predicted that as the two hydrogen bonds proceed, O- of Y66, which becomes negative, in the protein A56 would bind to K26, which becomes positive with a strong positive charge, of the light chain in Ab13. In addition, it was predicted that as the two hydrogen bonds proceed, the heavy chain and the light chain would undergo shrink folding so that the remaining bonds (S54 (A56)↔(heavy chain in antibody) and R96 (A56)↔(heavy chain in antibody)) are electrostatically formed.

### Example 5.1.3. Competition assay of binding between anti-A56 antibody (Ab13) and protein A56: binning test

To determine whether the ten anti-A56 antibodies (Ab18, Ab19, Ab01, Ab13, Ab14, Ab08, Ab03, Ab51, Ab55, Ab16) have the same epitope or different epitopes for the protein A56, analysis was performed by a surface plasmon resonance (SPR) method using Octet equipment.

Specifically, the A56-C-His antigen was immobilized on a biosensor (NTA), the primary antibodies (SA2038, Ab13, A56-02A02) were allowed to bind thereto to a saturated state, and the remaining nine antibodies were further allowed to bind thereto as secondary antibodies. Here, in a case where the antibodies have the same epitope, competition occurs and additional binding is difficult to occur; and in a case where the antibodies have different epitopes, additional binding will occur. In addition, the results were checked again by performing an additional experiment in which a binding order of the secondary antibody and the primary antibody to the A56-C-His antigen is reversed.

As a result, as illustrated in FIG. 58A, it was identified that no additional binding occurred, indicating that the antibodies have the same epitope.

In addition, Western blotting was performed to check the binding affinity in a two-dimensional structure using polyacrylamide gel electrophoresis (PAGE), and it was identified that binding did not occur on the protein A56 that is linear. From these results, it was identified that the anti-A56 antibody (Ab13) recognizes and binds to only a three-dimensional structure of the protein A56. In addition, the binding affinity (K_{D}) values calculated by OCTET (SPR) and ELISA are illustrated in FIG. 58B.

### Example 5.2.1. Analysis of electrostatic force of binding between anti-A56 antibody (Ab16) and protein A56

To analyze the electrostatic force of binding between the anti-A56 antibody (Ab16) and the protein A56, DelPhi potential was used. Specifically, the basic amino acid lysine (K61), which has a positive charge of strong DelPhi surface electrostatic potential, in the protein A56 excited a hydroxyl group of serine (S62), which is a residue right next thereto, so that an electrostatic force was generated and a relatively low level of DelPhi surface electrostatic potential was strongly positively charged. Here, for S59 and K60 of the light chain in Ab16, a positively charged DelPhi surface electrostatic potential was formed due to physicochemical properties of the protein A56.

In addition, it was predicted that arginine (R56) and lysine (K58) in CDR2 located at the loop between Sheet 5 (S5) and Sheet 6 (S6), which is a terminal exposed portion of the heavy chain in Ab16, would have a stronger positive charge due to the nucleophilic amino acid serine (S57) located between the two amino acids, so that binding to serine (S46, S54), which is a nucleophilic amino acid, in the protein A56 proceeds preferentially; and the electrostatic properties as shown in Table 11 were identified.

**[Table 11]**

| | protein | Amino acid | DelPhi potential | →Ab16, →UTTA |
|---|---|---|---|---|
| Ab16 | UTTA | K61/S62 | Strong +/mild- | S59L/K60L |
| | | S46/S54 | Strong - | R56H/K58H |
| | HC | R56/K58 | Strong + + | S46/S54 |
| | LC | S59/K60 | Strong +/mild- | K61/S62 |

In the table, the letter (H or L), which follows the amino acid position number indicated in the rightmost column, means heavy chain (H) or light chain (L).

### Example 5.2.2. Analysis of binding distance between anti-A56 antibody (Ab16) and protein A56

In order to analyze a binding distance between the anti-A56 antibody (Ab16) and the protein A56, structural alignment of the protein A56 with Ab16 was inferred using Swiss-PdbViewer (4.1.0), and active sites or other relevant portions were compared to predict H-bonds, angles, and distances between atoms.

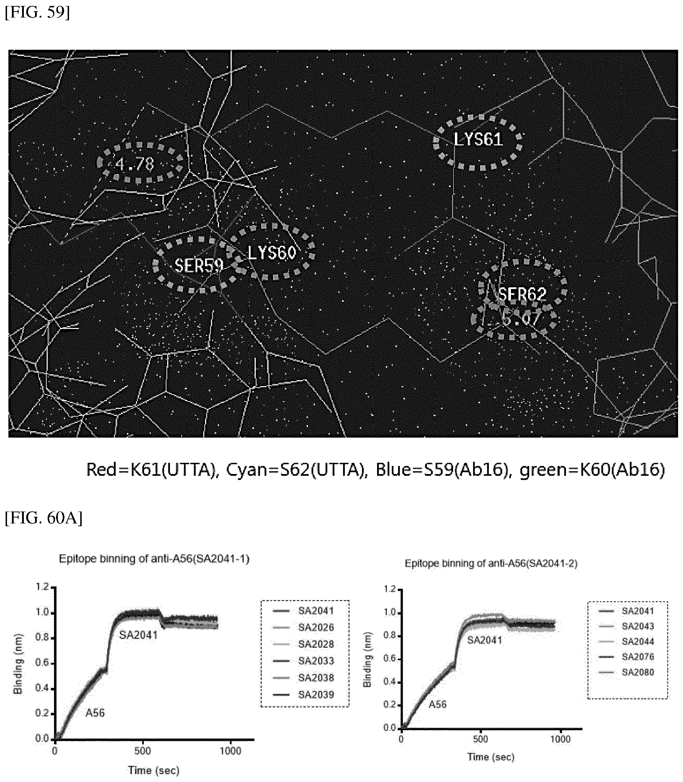

As a result, as illustrated in FIG. 59, an NH residue, that is, a terminal functional group of K61, which becomes positive with a strong positive charge, in the protein A56 formed a hydrogen bond with O- of S59, which becomes negative with an appropriate level of negative charge, of the light chain in Ab16, and a distance therebetween was measured to be 4.78 Å. At the same time, an NH residue, that is, a terminal functional group of K60, which becomes positive with a strong positive charge, of the light chain in Ab16 formed a hydrogen bond with O- of S62, which becomes negative with an appropriate level of negative charge, in the protein A56, and a distance therebetween was measured to be 5.07 Å.

It was predicted that as the two hydrogen bonds proceed, O- of S46/S54, which becomes negative, in the protein A56 would bind to R56/K58, which become positive with a strong positive charge, of the heavy chain in Ab16. In addition, it was predicted that as the binding proceeds, the heavy chain and the light chain would undergo shrink folding so that the remaining bonds are strongly electrostatically formed.

### Example 5.2.3. Competition assay of binding between anti-A56 antibody (Ab16) and protein A56: binning test

To determine whether the ten anti-A56 antibodies (Ab18, Ab19, Ab01, Ab13, Ab14, Ab08, Ab03, Ab51, Ab55, Ab16) have the same epitope or different epitopes for the protein A56, analysis was performed by a surface plasmon resonance (SPR) method using Octet equipment.

Specifically, the A56-C-His antigen was immobilized on a biosensor (NTA), the primary antibodies (SA2041, Ab16, A56-02B08) were allowed to bind thereto to a saturated state, and the remaining nine antibodies were further allowed to bind thereto as secondary antibodies. Here, in a case where the antibodies have the same epitope, competition occurs and additional binding is difficult to occur; and in a case where the antibodies have different epitopes, additional binding will occur. In addition, the results were checked again by performing an additional experiment in which a binding order of the secondary antibody and the primary antibody to the A56-C-His antigen is reversed.

As a result, as illustrated in FIG. 60A, it was identified that no additional binding occurred, indicating that the antibodies have the same epitope.

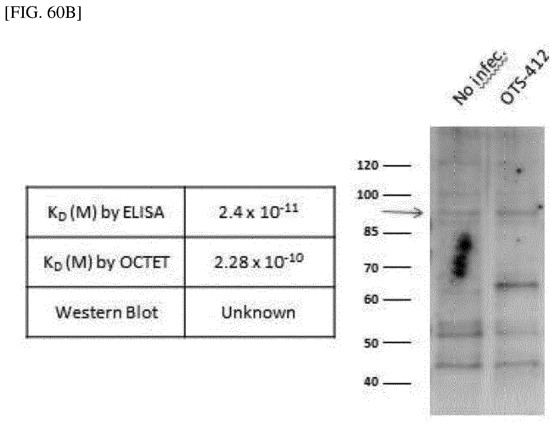

In addition, Western blotting was performed to check the binding affinity in a two-dimensional structure using PAGE, and linear multiple bands were identified. This indicates that there was no specific one-to-one binding for a simple linear amino acid sequence on the two-dimensional structure in the protein A56, and thus no discrimination was made. In addition, the binding affinity (K_{D}) values calculated by OCTET (SPR) and ELISA are illustrated in FIG. 60B.

### Example 5.3.1. Analysis of electrostatic force of binding between anti-A56 antibody (Ab18) and protein A56

To analyze the electrostatic force of binding between the anti-A56 antibody (Ab18) and the protein A56, DelPhi potential was used. Specifically, the basic amino acid lysine (K61), which has a positive charge of strong DelPhi surface electrostatic potential, in the protein A56 excited a hydroxyl group of serine (S62), which is a residue right next thereto, so that an electrostatic force was generated and a relatively low level of DelPhi surface electrostatic potential was strongly positively charged. Here, R98 of the heavy chain in Ab18 was highly exposed to the outside due to a methyl group (CH3), which is a short residue, of nearby A97, and W99 (tryptophan) that is aromatic and free of a hydrophilic reactive group, and a high level of DelPhi surface electrostatic potential thereof was strongly positively charged. In addition, for K50 of the light chain in Ab18, a relatively strongly positively charged DelPhi surface electrostatic potential was formed by negatively charged Y49 and S52 therearound.

As a result, the electrostatic properties as shown in Table 12 were identified.

**[Table 12]**

| protein | | Amino acid | DelPhi potential | →Ab16, →UTTA |
|---|---|---|---|---|
| UTTA | | K91 | Strong + + | Y32H, S30L |
| | | S62 | Mild - | R98H |
| | | T71 | Mild - | K50L |
| Ab18 | HC | R98 | Strong + + | S62U |
| | | Y32/S30/T31 | Strong - | K91U |
| | LC | S30 | Mid - | K91U |
| | | K50 | Strong + | T71U |

In the table, the letter (H, L, or U), which follows the amino acid position number indicated in the rightmost column, means heavy chain (H), light chain (L), or UTTA (or A56) (U).

### Example 5.3.2 Analysis of binding distance between anti-A56 antibody (Ab18) and protein A56

In order to analyze a binding distance between the anti-A56 antibody (Ab18) and the protein A56, structural alignment of the protein A56 with Ab18 was inferred using Swiss-PdbViewer (4.1.0), and active sites or other relevant portions were compared to predict H-bonds, angles, and distances between atoms.

As a result, an NH residue, that is, a terminal functional group of K91, which becomes positive with a strong positive charge, in the protein A56 formed a hydrogen bond with Y32L, which becomes negative with an appropriate level of negative charge, of the light chain in Ab18, and a distance therebetween was measured to be 3.46 Å (FIG. 61B). In addition, it was predicted that the structure would undergo folding by pulling, with an electrostatic force, S30, which becomes negative with an appropriate level of negative charge, of the light chain in Ab18. In addition, an NH residue, that is, a terminal functional group of R98, which becomes positive with a strong positive charge, of the heavy chain in Ab18 formed a hydrogen bond with O- of S62, which becomes negative with an appropriate level of negative charge, in the protein A56, and a distance therebetween was measured to be 3.77 Å (FIG. 61A).

It was predicted that as the two hydrogen bonds proceed, K50, which becomes positive with a strong positive charge, of the light chain in Ab18 would undergo folding and bind to O- of S30, which becomes negative, in the protein A56.

### Example 5.3.3. Competition assay of binding between anti-A56 antibody (Ab18) and protein A56: binning test

To determine whether the ten anti-A56 antibodies (Ab18, Ab19, Ab01, Ab13, Ab14, Ab08, Ab03, Ab51, Ab55, Ab16) have the same epitope or different epitopes for the protein A56, analysis was performed by an SPR method using Octet equipment.

Specifically, the A56-C-His antigen was immobilized on a biosensor (NTA), the primary antibodies (SA2043, Ab18, A56-02C06) were allowed to bind thereto to a saturated state, and the remaining nine antibodies were further allowed to bind thereto as secondary antibodies. Here, in a case where the antibodies have the same epitope, competition occurs and additional binding is difficult to occur; and in a case where the antibodies have different epitopes, additional binding will occur. In addition, the results were checked again by performing an additional experiment in which a binding order of the secondary antibody and the primary antibody to the A56-C-His antigen is reversed.

As a result, as illustrated in FIG. 62A, it was identified that no additional binding occurred, indicating that the antibodies have the same epitope.

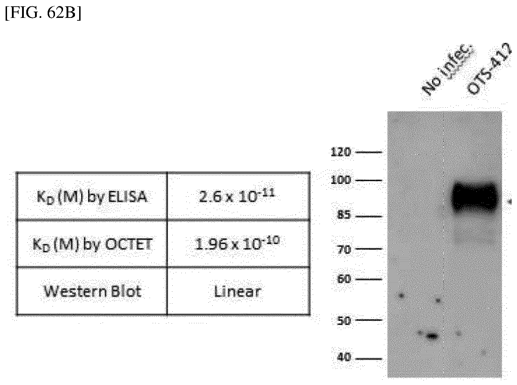

In addition, Western blotting was performed to check the binding affinity in a two-dimensional structure using PAGE, and linear multiple bands were identified. From these results, it was identified that this antibody recognized and bound to not only the amino acid sequence of a two-dimensional structure in the protein A56, but also a three-dimensional motif therein. In addition, the binding affinity (K_{D}) values calculated by OCTET (SPR) and ELISA are illustrated in FIG. 62B.

### Example 5.4.1. Analysis of electrostatic force of binding between anti-A56 antibody (Ab01) and protein A56

To analyze the electrostatic force of binding between the anti-A56 antibody (Ab01) and the protein A56, DelPhi potential was used. Specifically, the basic amino acid lysine (K61), which has a positive charge of strong DelPhi surface electrostatic potential, in the protein A56 excited a hydroxyl group of serine (S62), which is a residue right next thereto, so that an electrostatic force was generated and a relatively low level of DelPhi surface electrostatic potential was strongly positively charged. Then, for S32 and T102 of the light chain in Ab01, their DelPhi surface electrostatic potential was relatively negatively charged at an appropriate level.

In addition, S103 and Y106 of the heavy chain in Ab01 were highly exposed to the outside due to the nearby hydrophobic residue L107, and F101 that is aromatic and free of a hydrophilic reactive group, and a high level of DelPhi surface electrostatic potential thereof was strongly negatively charged due to the nucleophilic amino acid S103.

As a result, the electrostatic properties as shown in Table 13 were identified.

**[Table 13]**

| protein | | Amino acid | DelPhi potential | →Ab01, →UTTA |
|---|---|---|---|---|
| UTTA | | K61 | Strong + | T102L |
| | | K91 | Strong + | Y106H |
| | | 554 | Mid - | T57H |
| | | T100 | Mid - | S32L, S103H |
| Ab01 | HC | T57 | Strong - | S54 |
| | | S103 | Strong - | T100 |
| | | Y106 | Strong - | K91 |
| | LC | S32 | Mid - | T100 |
| | | T102 | Mid - | K61 |

In the table, the letter (H or L), which follows the amino acid position number indicated in the rightmost column, means heavy chain (H) or light chain (L).

### Example 5.4.2. Analysis of binding distance between anti-A56 antibody (Ab01) and protein A56

In order to analyze a binding distance between the anti-A56 antibody (Ab01) and the protein A56, structural alignment of the protein A56 with Ab01 was inferred using Swiss-PdbViewer (4.1.0), and active sites or other relevant portions were compared to predict H-bonds, angles, and distances between atoms.

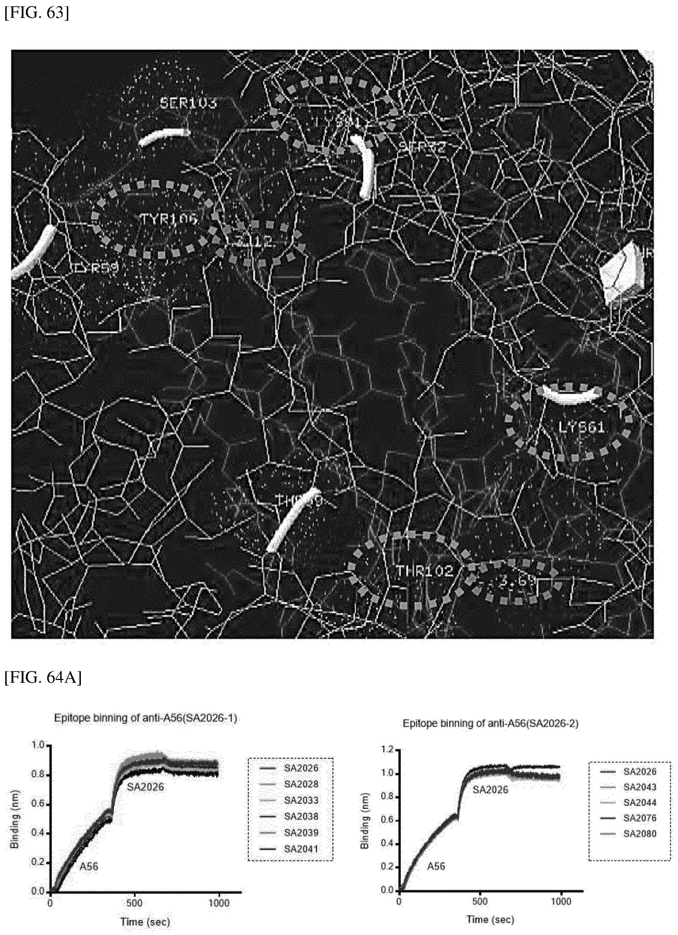

As a result, as illustrated in FIG. 63, an NH residue, that is, a terminal functional group of K61, which becomes positive with a strong positive charge, in the protein A56 formed a hydrogen bond with O- of T102, which becomes negative with an appropriate level of negative charge, of the light chain in Ab01, and a distance therebetween was measured to be 3.69 Å. In addition, an NH residue, that is, a terminal functional group of K91, which becomes positive with a strong positive charge, in the protein A56 formed a hydrogen bond with O- of Y106, which becomes negative with an appropriate level of negative charge, of the heavy chain in Ab01, and a distance therebetween was measured to be 3.12 Å.

It was predicted that as the two hydrogen bonds proceed, S54 in the protein A56 binds to T57, T100, S103 of the heavy chain and S32 of the light chain in Ab01. In addition, it was predicted that as the two hydrogen bonds proceed, the heavy chain and the light chain would undergo shrink folding so that the remaining bonds are electrostatically formed.

### Example 5.4.3. Competition assay of binding between anti-A56 antibody (Ab01) and protein A56: binning test

To determine whether the ten anti-A56 antibodies (Ab18, Ab19, Ab01, Ab13, Ab14, Ab08, Ab03, Ab51, Ab55, Ab16) have the same epitope or different epitopes for the protein A56, analysis was performed by an SPR method using Octet equipment.

Specifically, the A56-C-His antigen was immobilized on a biosensor (NTA), the primary antibodies (SA2026, Ab01, A56-01A02) were allowed to bind thereto to a saturated state, and the remaining nine antibodies were further allowed to bind thereto as secondary antibodies. Here, in a case where the antibodies have the same epitope, competition occurs and additional binding is difficult to occur; and in a case where the antibodies have different epitopes, additional binding will occur. In addition, the results were checked again by performing an additional experiment in which a binding order of the secondary antibody and the primary antibody to the A56-C-His antigen is reversed.

As a result, as illustrated in FIG. 64A, it was identified that no additional binding occurred, indicating that the antibodies have the same epitope.

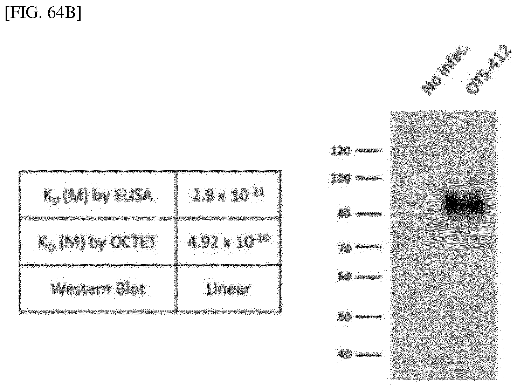

In addition, Western blotting was performed to check the binding affinity in a two-dimensional structure using PAGE, and a linear single band was identified. In addition, the binding affinity (K_{D}) values calculated by OCTET (SPR) and ELISA are illustrated in FIG. 64B.

### Example 5.5.1. Analysis of electrostatic force of binding between anti-A56 antibody (Ab19) and protein A56

To analyze the electrostatic force of binding between the anti-A56 antibody (Ab19) and the protein A56, DelPhi potential was used. Specifically, the basic amino acid lysine (K61), which has a positive charge of strong DelPhi surface electrostatic potential, in the protein A56 excited a hydroxyl group of serine (S62), which is a residue right next thereto, so that an electrostatic force was generated and a relatively low level of DelPhi surface electrostatic potential was strongly positively charged. Here, among the amino acids at positions 90 to 95 (DSSSD) of the light chain in Ab19, for serines at positions 93 and 94 (S93, S94), their DelPhi surface electrostatic potential was relatively strongly negatively charged due to the strong negative charge of aspartic acid residues (D90 and D95), this residue being an acidic amino acid with a negative charge.

In addition, Y32 of the heavy chain in Ab19 was highly exposed to the outside due to the nearby hydrophobic amino acids V (valine) and L (leucine), and F (phenylalanine) that is aromatic and free of a hydrophilic reactive group, and a low level of DelPhi surface electrostatic potential thereof was negatively charged at a low level due to the nucleophilic amino acid S (serine).

As a result, the electrostatic properties as shown in Table 14 were identified.

**[Table 14]**

| protein | | Amino acid | DelPhi potential |
|---|---|---|---|
| UTTA | | K 61 | Strong + |
| | | S 62 | Mild + |
| Ab19 | HC | Y 32 | Mid - |
| | LC | S93, S94 | Strong - |

### Example 5.5.2. Analysis of binding distance between anti-A56 antibody (Ab19) and protein A56

In order to analyze a binding distance between the anti-A56 antibody (Ab19) and the protein A56, structural alignment of the protein A56 with Ab19 was inferred using Swiss-PdbViewer (4.1.0), and active sites or other relevant portions were compared to predict H-bonds, angles, and distances between atoms.

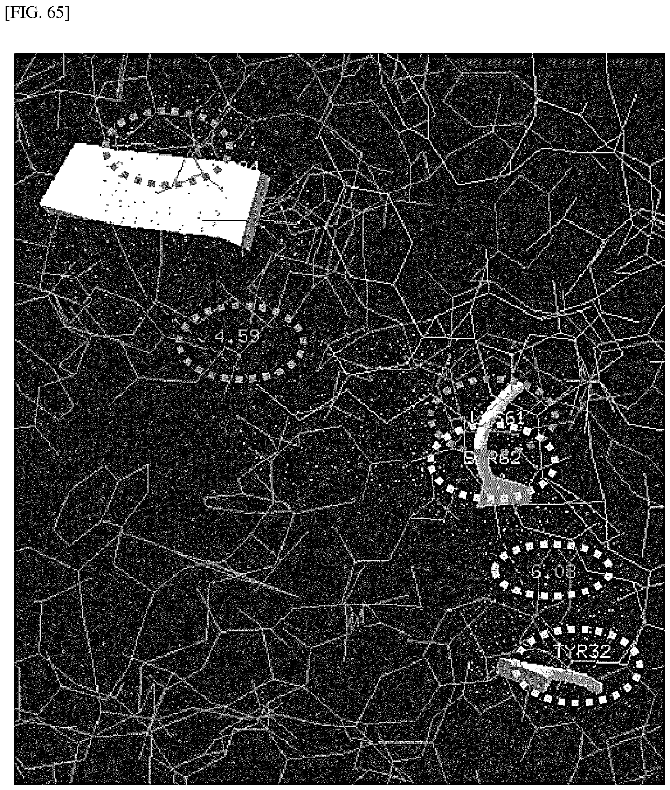

As a result, as illustrated in FIG. 65, it was predicted that K61 and S62, which are terminal functional groups of K61 and S62 in the protein A56, would be ionized to form NH+ (K61) and O- (S62), and S93 and S94 of the light chain in Ab19 would have an electrostatic surface DelPhi potential of O- (S93, S94) with a strong negative charge due to the nearby aspartic acid residues (D91 and D95), so that a bond is formed. NH2+ (K61) and O- (S62) spaced at 9.9 Å in the protein A56 are located between O-(S93) of the light chain and O- of Y32 of the heavy chain, which are spaced at 19.5 Å, in Ab19, so that K61 in the protein A56 binds to S93 in Ab19, in which the binding distance was measured to be 4.59 Å (indicated by a pink circle). S62 in the protein A56 bound to Y32 in Ab19, in which the binding distance was measured to be 6.08 Å (indicated by a white circle).

In addition, the binding between the protein A56 and Ab19 shows that the two molecules bind horizontally. Thus, it was predicted that in a case where another antibody with a specific paratope other than the binding site has a complementary electrostatic surface DelPhi potential, multi-binding would be possible due to presence of a different epitope on the protein A56.

### Example 5.5.3. Competition assay of binding between anti-A56 antibody (Ab19) and protein A56: binning test

To determine whether the ten anti-A56 antibodies (Ab18, Ab19, Ab01, Ab13, Ab14, Ab08, Ab03, Ab51, Ab55, Ab16) have the same epitope or different epitopes for the protein A56, analysis was performed by an SPR method using Octet equipment.

Specifically, the A56-C-His antigen was immobilized on a biosensor (NTA), the primary antibodies (SA2044, Ab19, A56-02C07) were allowed to bind thereto to a saturated state, and the remaining nine antibodies were further allowed to bind thereto as secondary antibodies. Here, in a case where the antibodies have the same epitope, competition occurs and additional binding is difficult to occur; and in a case where the antibodies have different epitopes, additional binding will occur. In addition, the results were checked again by performing an additional experiment in which a binding order of the secondary antibody and the primary antibody to the A56-C-His antigen is reversed.

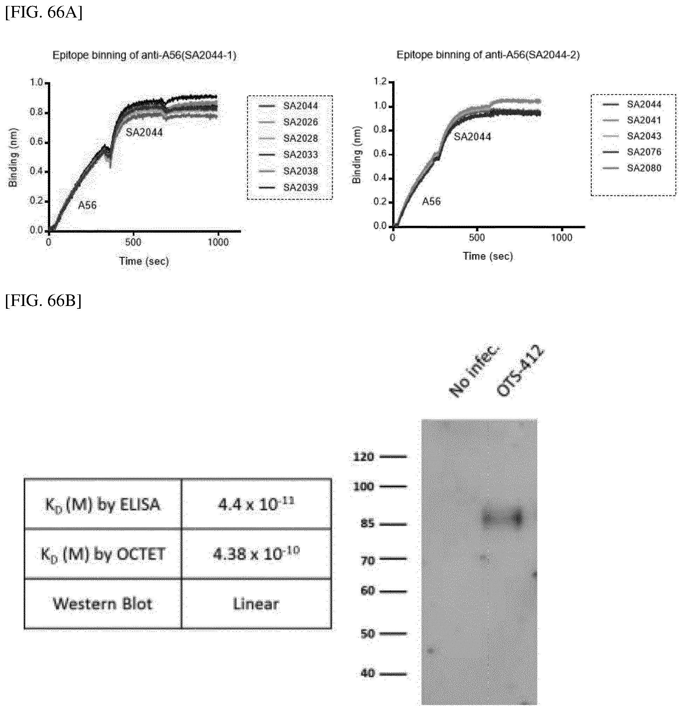

As a result, as illustrated in FIG. 66A, it was analyzed that although subtle, additional binding of two antibodies (SA2041 (Ab 16), SA2043 (Ab 18)) occurred in the step where the secondary antibody was bound. From these results, it was identified that the two antibodies (SA2041 (Ab16) and SA2043 (Ab18)) had epitopes other than that of Ab19.

In addition, Western blotting was performed to check the binding affinity in a two-dimensional structure using PAGE, and a linear single band was identified. In addition, the binding affinity (K_{D}) values calculated by OCTET (SPR) and ELISA are illustrated in FIG. 66B.

### Experimental Example 8. Production of CAR-T cells using antigen-binding region of anti-A56 antibody

### Experimental Example 8.1. Structural design of chimeric antigen receptor

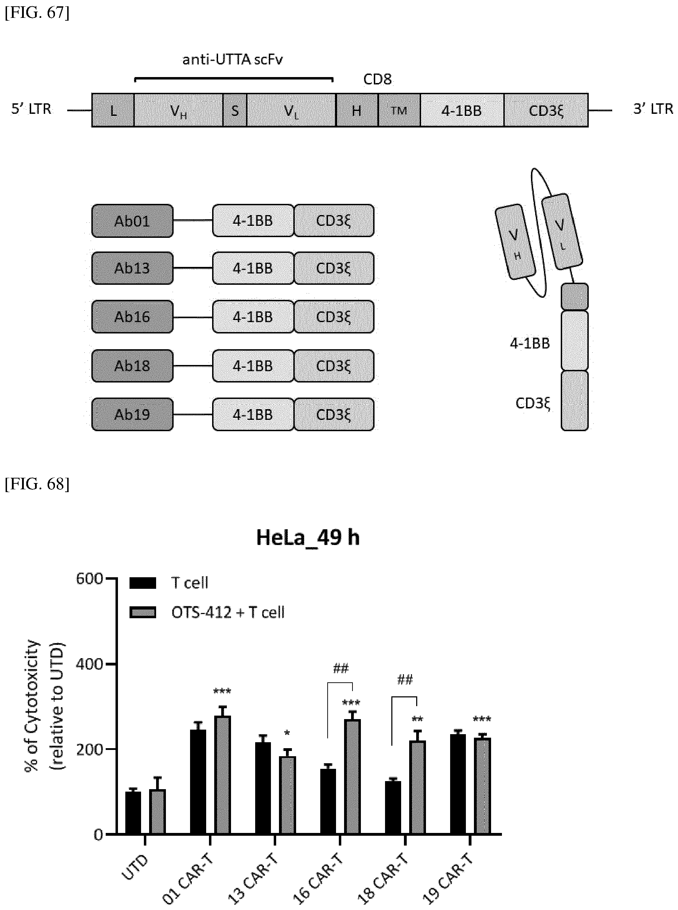

The structure of a chimeric antigen receptor was designed to include a signal peptide, an antigen-binding domain, a transmembrane domain, and an intracellular signaling domain. As an example, as illustrated in FIG. 67, it was designed to include an antigen-binding domain (anti-UTTA scFv), a CD8 transmembrane domain (H+TM), and an intracellular signaling domain (4-1BB and CD3Z).

### Experimental Example 8.2. Construction of vector encoding chimeric antigen receptor

As a vector, pLVX-EF1α-IRES-mCherry vector (Spel/Notl) was used. Into the vector was inserted a gene encoding a chimeric antigen receptor that includes a signal peptide (sp), a single chain variable fragment (anti-UTTA scFv) which specifically binds to A56, a transmembrane domain (H+TM) of human CD8, and an intracellular signaling domain (4-1BB and CD3ζ).

The amino acid sequence and nucleotide sequence of the chimeric antigen receptor used in the experiment are shown in Table 15.

**[Table 15]**

| UTTA-CAR | Domain | | Sequence information | SEQ ID NO |
|---|---|---|---|---|
| Amino Acid Sequence | CD8 signal sequence | | MALPVTALLLPLALLLHAARP | 92 |
| | anti-UTTA | VH | QMQLVESGAEVKKTGSSVKVSCKASGDTLTYRFL HWVRQAPGQAPEWMGWITPFNDNTNYAQKFQD | 93 |
| | scFv (Ab13) | | RVTITRDRSMSTAYMELSSLRSEDTAVYYCATGGG NNYYGMEVWGQGTPITVSS | |
| | | Spacer | GLGGLGGGGSGGGGSGGSSGVGS | |
| | | VL | SYELTQPLSLSVSPGQTASITCSGDKLGDKFTSWY QQQPGQSPVLVIYQDAKRPSGIPERFSGSNSGSTAT LTISGTQSMDEADYYCQAWDSSSVVFGGGTKLTV L | |
| | anti-UTTA scFv (Ab16) | VH | QVQLQQSGPGLVKPSQTLSLTCAISGDSVSSNSAA WNWIRQSPSRGLEWLGRTYYRSKWYNDYAVSVK SRITINPDTSKNQFSLQLNSVTAVDTAVYYCAKSY AGPISYYYYGLDVWGQGTTVTVSS | 94 |
| | | Spacer | GLGGLGGGGSGGGGSGGSSGVGS | |
| | | VL | QSALTQPASVSGSPGQSVTISCTGTSSDIGRYNLVS WYQQHPGKAPKLMIYGGSKRPSGVSNRFSGSNSD NTASLTISGLRAEDEADYYCSSYASSSTPYVFGTGT KVTVL | |
| | anti-UTTA scFv (Ab18) | VH | QVQLVESGGGLVQPGKSLRLSCAASGFTFSTYGM TWVRQAPGKGLEWVSGISGGGSSTNYADSVKGR FIISRDNSNNTLYLQMNSLRAEDTAVYYCARWGV MLSFDYWGQGTPVTVSS | 95 |
| | | Spacer | GLGGLGGGGSGGGGSGGSSGVGS | |
| | | VL | DIQMTQSPSTLPASVGDRVTITCRASYSVSPWLAW YQQKPGKAPKLLIYKASTLESGVPSRFSGSGSGTE FTLTISSLQPDDFATYYCQQFNSYMMYTFGQGTKL EIK | |
| | anti-UTTA scFv (Ab01) | VH | QMQLVQSGAEVKKPGSSVKVSCKASGGTFSSYAI SWVRQAPGQGLEWMGGIIPIFGTANYAQKFQGRV TMTRDTSTSTVYMELSSLKSEDTAVYYCARFVFG SGTYLDSWGQGTLVTVSS | 96 |
| | | Spacer | GLGGLGGGGSGGGGSGGSSGVGS | |
| | | VL | DIVMTQTPLSLPVTPGESASISCRSSQSLLYSNGNN YLDWYLQKPGQSPQLLIYLGSNRASGVPDRFSGS GSGTDFTLKISRVEAEDVGVYYCMQALQTPLTFG GGTKVDIK | |
| | anti-UTTA scFv (Ab19) | VH | QVQLVESGAEVRRPGSSVKVSCKTSGVTFSGYVL SWVRQAPGHGLEWMGRIIPLIDVENYAREFQGRM KITADKSTNTVYMELNNLRSEDTAVYYCAKSVVR GLDYYYYGFDVWGQGTTVTVSS | 97 |
| | | Spacer | GLGGLGGGGSGGGGSGGSSGVGS | |
| | | VL | SYELTQPPSLSVAPGKTARITCGGNNIGSKSVHWY QQKPGQAPRLVTYYDGNRPSGIPERFSGSNSGNTA TLIISRVEAGDEADYYCQVWDSSSDQGVFGTGTK VTVL | |
| | CD8 TM | Hinge(H) | TTTPAPRPPTPAPTIASQPLSLRPEACRPAAGGAVH TRGTTTPAPRPPTPAPTIASQPLSLRPEACRPAAGG AVHTRGLDFACD | 98 |
| | | TM | IYIWAPLAGTCGVLLLSLVITLYC | |
| | 4-IBB Signaling Domain | | KRGRKKLLYIFKQPFMRPVQTTQEEDGCSCRFPEE EEGGCEL | 99 |
| | CD3Z Signaling Domain | | RVKFSRSADAPAYKQGQNQLYNELNLGRREEYDV LDKRRGRDPEMGGKPRRKNPQEGLYNELQKDKM AEAYSEIGMKGERRRGKGHDGLYQGLSTATKDTY | 100 |
| | | | DALHMQALPPR | |
| Nucleotid e Sequence | CD8 signal sequence | | ATGGCTCTGCCTGTGACAGCTCTGCTGCTGCCTC TGGCTCTGCTTCTGCATGCTGCTAGACCT | 101 |
| | anti-UTTA scFv (Ab13) | VH | CAGATGCAGCTGGTGGAGTCTGGGGCTGAGGTG AAGAAGACTGGGTCCTCAGTGAAGGTTTCCTGC AAGGCTTCCGGAGACACCCTCACCTACCGCTTC CTGCACTGGGTGCGACAGGCCCCCGGACAAGC GCCTGAGTGGATGGGATGGATCACACCTTTCAAT GATAACACCAACTACGCACAGAAATTCCAGGAC AGAGTCACCATTACCAGGGACAGGTCTATGAGC ACAGCCTACATGGAGCTGAGCAGCCTGAGATCT GAGGACACGGCCGTGTATTACTGTGCGACTGGA GGTGGGAACAATTATTACGGCATGGAGGTCTGG GGCCAAGGGACCCCGATCACCGTCTCCTCA | 102 |
| | | Spacer | GGCCTCGGGGGCCTCGGAGGAGGAGGTAGTGG CGGAGGAGGCTCCGGTGGATCCAGCGGTGTGG GTTCC | |
| | | VL | TCCTATGAGCTGACTCAGCCACTCTCACTGTCCG TGTCCCCAGGACAGACAGCCAGCATCACCTGCT CTGGAGATAAATTGGGAGATAAATTTACTTCCTG GTATCAACAGCAGCCAGGCCAGTCCCCTGTACT GGTCATCTATCAAGATGCCAAGCGACCCTCAGG GATCCCTGAGCGATTCTCTGGCTCCAACTCTGGG AGCACAGCCACTCTGACCATCAGCGGGACCCAG TCTATGGATGAGGCTGACTATTACTGTCAGGCGT GGGACAGCAGCAGTGTGGTGTTCGGCGGAGGG ACCAAGCTGACCGTCCTA | |
| | anti-UTTA scFv (Ab16) | VH | CAGGTGCAGCTGCAGCAGTCAGGTCCAGGACT GGTGAAGCCCTCGCAGACCCTCTCACTCACCTG TGCCATCTCCGGGGACAGTGTCTCTAGCAACAG TGCTGCTTGGAACTGGATCAGGCAGTCCCCATC GAGAGGCCTTGAGTGGCTGGGAAGGACATACTA CAGGTCCAAGTGGTATAATGATTATGCAGTATCT GTGAAAAGTCGAATAACCATCAACCCAGACACA TCCAAGAACCAGTTCTCCCTGCAGCTGAACTCT GTGACCGCCGTGGACACGGCCGTGTATTACTGT GCGAAATCCTATGCGGGGCCTATCTCTTATTACTA CTACGGTCTGGACGTCTGGGGCCAAGGGACCAC GGTCACCGTCTCCTCA | 103 |
| | | Spacer | GGCCTCGGGGGCCTCGGAGGAGGAGGTAGTGG CGGAGGAGGCTCCGGTGGATCCAGCGGTGTGG GTTCC | |
| | | VL | CAGTCTGCCCTGACTCAGCCTGCCTCCGTGTCT GGGTCTCCTGGACAGTCGGTCACCATCTCCTGC ACTGGAACCAGCAGTGACATTGGTCGTTATAAC CTTGTCTCCTGGTACCAACAGCACCCAGGCAAA GCCCCCAAACTCATGATTTATGGGGGCAGTAAG CGGCCCTCAGGGGTTTCTAATCGCTTCTCTGGCT CCAACTCTGACAACACGGCCTCCCTGACAATCT CTGGGCTCCGGGCTGAGGACGAGGCTGATTATT ACTGCAGTTCATATGCAAGCAGCAGCACCCCTT ATGTCTTCGGAACTGGGACCAAGGTCACCGTCC TA | |
| | anti-UTTA scFv (Ab18) | VH | CAGGTGCAGCTGGTGGAGTCTGGGGGAGGCTT GGTACAGCCTGGGAAATCCCTGAGACTCTCCTG TGCAGCCTCTGGATTCACCTTCAGCACCTATGGC ATGACTTGGGTCCGCCAGGCTCCAGGGAAGGGG CTGGAGTGGGTCTCAGGGATTAGTGGTGGGGGT TCTAGCACAAACTACGCAGACTCCGTGAAGGGC CGCTTCATCATTTCTAGAGACAACTCTAACAACA CGCTGTATCTGCAAATGAACAGTCTGAGAGCCG | 104 |
| | | | AGGACACGGCCGTGTATTACTGTGCAAGGTGGG GAGTTATGCTTTCCTTTGACTACTGGGGCCAGGG AACCCCGGTCACCGTCTCC | |
| | | Spacer | TCAGGCCTCGGGGGCCTCGGAGGAGGAGGTAG TGGCGGAGGAGGCTCCGGTGGATCCAGCGGTGT GGGTTCC | |
| | | VL | GACATCCAGATGACCCAGTCTCCTTCCACCCTGC CTGCATCTGTAGGGGACAGAGTCACCATCACTT GCCGGGCCAGTTACAGTGTCAGTCCCTGGTTGG CCTGGTATCAGCAGAAACCAGGGAAAGCCCCTA AACTCCTGATCTATAAGGCATCTACTTTAGAAAG TGGGGTCCCATCAAGGTTCAGCGGCAGTGGATC TGGGACAGAATTCACTCTCACCATCAGCAGCCT GCAGCCTGATGATTTTGCAACTTATTACTGCCAA CAGTTTAATAGTTATATGATGTACACTTTTGGCCA GGGGACCAAGCTGGAAATCAAA | |
| | anti-UTTA scFv (Ab01) | VH | CAGATGCAGCTGGTGCAGTCTGGGGCTGAGGTG AAGAAGCCTGGGTCCTCGGTGAAGGTCTCCTGC AAGGCTTCTGGAGGCACCTTCAGCAGCTATGCT ATCAGCTGGGTGCGACAGGCCCCTGGACAAGG GCTTGAGTGGATGGGAGGGATCATCCCTATCTTT GGTACAGCAAACTACGCACAGAAGTTCCAGGGC AGAGTCACCATGACCAGGGACACGTCCACGAG CACAGTCTACATGGAGCTGAGCAGCCTGAAATC TGAGGACACGGCCGTGTATTACTGTGCGAGATT CGTCTTTGGTTCGGGGACTTATCTTGACTCCTGG GGCCAGGGAACCCTGGTCACCGTCTCCTCA | 105 |
| | | Spacer | GGCCTCGGGGGCCTCGGAGGAGGAGGTAGTGG CGGAGGAGGCTCCGGTGGATCCAGCGGTGTGG GTTCC | |
| | | VL | GATATTGTGATGACCCAGACTCCACTCTCCCTGC CCGTCACCCCTGGAGAGTCGGCCTCCATCTCCT GCAGGTCTAGTCAGAGCCTCCTGTATAGTAATGG GAACAACTATTTGGATTGGTACCTGCAGAAGCC AGGGCAGTCCCCACAGCTCCTGATCTATTTGGGT TCTAATCGGGCCTCCGGGGTCCCTGACAGGTTC AGTGGCAGTGGATCAGGCACAGATTTTACACTG AAAATCAGCAGAGTGGAGGCTGAGGATGTTGG GGTTTATTACTGCATGCAAGCTCTACAAACTCCT CTCACTTTCGGCGGAGGGACCAAGGTGGATATC AAA | |
| | anti-UTTA scFv (Ab19) | VH | CAGGTGCAGCTGGTGGAGTCTGGGGCTGAGGT GAGGAGGCCTGGGTCCTCGGTGAAGGTCTCCTG CAAGACTTCGGGAGTCACCTTCAGCGGCTATGT TCTGAGCTGGGTGCGACAGGCCCCTGGACACGG CCTTGAGTGGATGGGACGGATCATCCCTTTAATT GACGTGGAAAACTATGCACGGGAGTTCCAGGGT AGAATGAAGATCACCGCAGACAAGTCCACGAAT ACAGTCTACATGGAACTGAACAACCTGAGATCT GAGGACACGGCCGTGTATTACTGTGCGAAATCG GTCGTACGGGGTCTTGACTACTACTACTACGGTT TCGACGTCTGGGGCCAAGGGACCACGGTCACC GTCTCCTCA | 106 |
| | | Spacer | GGCCTCGGGGGCCTCGGAGGAGGAGGTAGTGG CGGAGGAGGCTCCGGTGGATCCAGCGGTGTGG GTTCC | |
| | | VL | TCCTATGAGCTGACACAACCACCCTCACTGTCA GTGGCCCCAGGAAAGACGGCCAGGATTACCTGT GGGGGAAACAACATTGGAAGTAAAAGTGTGCA CTGGTACCAGCAGAAGCCAGGCCAGGCCCCTAG ACTGGTCACTTATTATGATGGCAACCGGCCCTCA | |
| | | | GGGATCCCTGAGCGATTCTCTGGCTCCAACTCTG GAAACACGGCCACCCTAATCATCAGCAGGGTCG AAGCCGGGGATGAGGCCGACTATTACTGTCAGG TGTGGGATAGTAGTAGTGATCAGGGGGTCTTCG GAACTGGGACCAAGGTCACCGTCCTA | |
| | CD8 TM | Hinge(H) | ACCACAACACCCGCTCCTAGACCTCCAACACCA GCTCCAACAATCGCCAGCCAGCCTCTGTCTCTG AGGCCTGAAGCTTGTAGACCTGCTGCAGGCGGA GCCGTGCATACCAGAGGACTGGATTTCGCCTGC GAC | 107 |
| | | TM | ATCTACATCTGGGCCCCTCTGGCTGGAACATGTG GCGTTCTGCTGCTCAGCCTGGTCATCACCCTGTA CTGC | |
| | 4-IBB Signaling Domain | | AAGCGGGGCAGAAAGAAGCTGCTGTACATCTTC AAGCAGCCCTTCATGCGGCCCGTGCAGACAACC CAAGAGGAAGATGGCTGCTCCTGCAGATTCCCC GAGGAAGAAGAAGGCGGCTGCGAGCTG | 108 |
| | CD3Z Signaling Domain | | AGAGTGAAGTTCAGCAGATCCGCCGACGCTCCC GCCTATAAGCAGGGACAGAACCAGCTGTACAAC GAGCTGAACCTGGGGAGAAGAGAAGAGTACGA CGTGCTGGACAAGCGGAGAGGCAGAGATCCTG AGATGGGCGGCAAGCCCAGACGGAAGAATCCT CAAGAGGGCCTGTATAATGAGCTGCAGAAAGAC AAGATGGCCGAGGCCTACTCCGAGATCGGAATG AAGGGCGAGCGCAGAAGAGGCAAGGGACACG ATGGACTGTACCAGGGCCTGAGCACCGCCACCA AGGATACCTATGATGCCCTGCACATGCAGGCCCT GCCTCCAAGA | 109 |

### Experimental Example 8.3. Production of CAR-T cells

CAR-T cells were produced by separating CD4+/CD8+ cells from the blood provided by the Pusan National University Yangsan Hospital (Korea) with approval of IRB under the human-derived material research, using the MACS Cell Separation system. Then, CD3+ T cells (≥97%) were separated using MACS Pan T cell Ab. Subsequently, 1×10⁶ CD3+ T cells were cultured in medium containing 20 lU/ml of rhIL-2 and TransAct (CD3/CD28 agonist) for 24 hours to induce T cell activation.

The activated T cells were treated with each of two types of lentivirus, which was cloned into the vector constructed in Experimental Example 8.2, at an MOI of 50, and cultured for 48 hours. Then, the T cells were resuspended at 1×10⁶ cells/ml in medium containing 20 IU/ml of rhIL-2. The cells were counted every 2 or 3 days while replacing the medium with new medium containing rhIL-2 for cell proliferation. Here, the CAR-T cells produced using the lentivirus against the antigen-binding domain of Ab16 were designated as "16 CAR-T", and the CAR-T cells produced using the lentivirus against the antigen-binding domain of Ab18 were designated as "18 CAR-T". In addition, T cells (UTD, untransduced), which were not transduced with any CAR, were used as a control group.

### Experimental Example 9. Identification of anticancer effect of oncolytic vaccinia virus (OTS-412) and CAR-T cells

Anticancer effects of UTD cells and five types of CAR-T cells were analyzed by measuring cell death in real-time using a fluorescence-based cell imaging system (Incucyte^{®} Live-Cell Analysis System, Sartorius).

Specifically, the three cancer cell lines HeLa (3×10³ cells/well), NCI-H522 (6×10³ cells/well), and HCT-116 (9×10³ cells) were seeded in 96-well-plates. Then, while performing incubation at a temperature of 37°C, the cells were subjected to infection with OTS-412 (1.55×10⁸ pfu/ml) of Preparation Example 1.1 at an MOI of 0.05 so that the protein A56 was expressed on the cancer cell surface. After 2 hours, the medium was replaced with medium containing 2% FBS. After 4 hours, each of UTD cells and five types of CAR-T cells (Ab13 CAR-T, Ab16 CAR-T, Ab18 CAR-T, Ab01 CAR-T, Ab19 CAR-T) was administered so that a ratio of T cell: cancer cell was 3:1. The T cells were administered with medium (10% FBS) containing a red fluorescent staining reagent. Then, cell death image data were acquired at 30-minute intervals for 5 days using the Incucyte system and analyzed through software.

As a result, for the HeLa cell line infected with OTS-412, as compared with the group treated with the UTD cells, the groups, which were treated with the five types of CAR-T cells, respectively, showed a statistically significant specific cytotoxic effect (FIG. 68; * indicates p<0.033, ** p<0.002, and *** p<0.001, relative to the UTD group, and ## indicates p<0.002 relative to treatment with T cells alone in the same scFv Car-T group). In particular, among these, the Ab16 CAR-T and Ab18 CAR-T cells exhibited a statistically significant difference in protein A56-specific cytotoxicity as compared with the cytotoxicity of the group not infected with OTS-412.

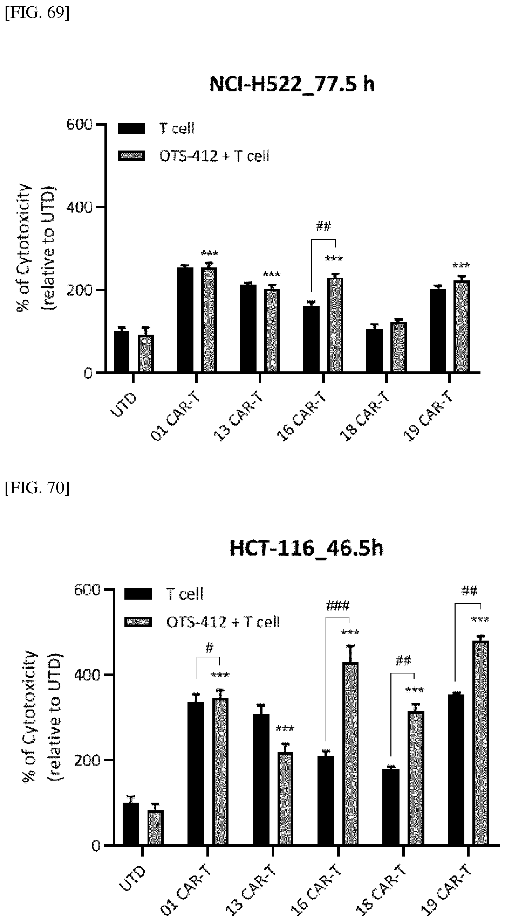

For the NCI-H522 cell line infected with OTS-412, as compared with the group treated with the UTD cells, the groups, which were treated with the Ab01 CAR-T, Ab13 CAR-T, Ab16 CAR-T, and Ab19 CAR-T cells, respectively, among the five types of CAR-T cells, showed a statistically significant specific cytotoxic effect (FIG. 69; * indicates p<0.033, ** p<0.002, and *** p<0.001, relative to the UTD group, and ## indicates p<0.002 relative to treatment with T cells alone in the same scFv Car-T group). In particular, among these, the Ab16 CAR-T cells exhibited a statistically significant difference in protein A56-specific cytotoxicity as compared with the cytotoxicity of the group not infected with OTS-412.

For the HCT-116 cell line infected with OTS-412, as compared with the group treated with the UTD cells, the groups, which were treated with the five types of CAR-T cells, respectively, showed a statistically significant CAR-T cell-specific cytotoxic effect (FIG. 70; * indicates p<0.033, ** p<0.002, and *** p<0.001, relative to the UTD group, and ## indicates p<0.002 relative to treatment with T cells alone in the same scFv Car-T group). In particular, among these, the Ab01 CAR-T, Ab16 CAR-T, Ab18 CAR-T, and Ab19 CAR-T cells exhibited a statistically significant difference in protein A56-specific cytotoxicity as compared with the cytotoxicity of the group not infected with OTS-412.

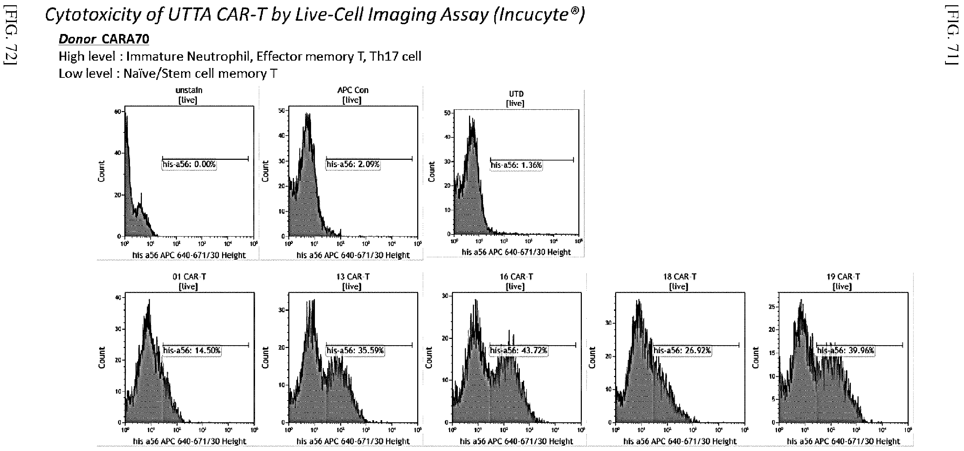

Furthermore, in a case where the transduction efficiency of the five types of CAR-T cells was measured with flow cytometry (FACS), for the Ab13 CAR-T, Ab16 CAR-T, and Ab19 CAR-T cells, distinct peaks were observed; and on the other hand, for the Ab01 CAR and Ab18 CAR-T cells, it appears that peaks distinct from the negative peak were not clearly observed due to low intensity of CAR expressed on the T cell surface, and the transduction efficiency was also low (FIG. 71). Differences in transduction efficiency between the five CAR-Ts were similar in the blood derived from the subjects of different ages and sexes. The transduction efficiency was consistently higher in Ab13 CAR-T, Ab16 CAR-T, and Ab19 CAR-T than Ab01 CAR-T and Ab18 CAR-T; and among these, the transduction efficiency of Ab16 CAR-T was consistently the highest.

### Experimental Example 10. Identification of anticancer effect of oncolytic vaccinia virus (OTS-412) and CAR-T cells

Anticancer effects of UTD cells and five types of CAR-T cells were analyzed by measuring cell death in real-time using a fluorescence-based cell imaging system (Incucyte^{®} Live-Cell Analysis System, Sartorius).

Specifically, the four cancer cell lines A549 (1×10⁴ cells) and HCT-116 (3×10⁴ cells) were seeded in 96-well-plates. Then, while performing incubation at a temperature of 37°C, the cells were subjected to infection with OTS-412 (1.55×10⁸ pfu/ml) of Preparation Example 1.1 at an MOI of 0.05 so that the protein A56 was expressed on the cancer cell surface. After 2 hours, the medium was replaced with medium containing 2% FBS. After 4 hours, each of UTD cells, Ab16 CAR-T cells, and Ab18 CAR-T cells was administered so that a ratio of T cell: cancer cell was 1:1. The T cells were administered with medium (10% FBS) containing a red fluorescent staining reagent. Then, cell death image data were acquired at 30-minute intervals for 5 days using the Incucyte system and analyzed through software.

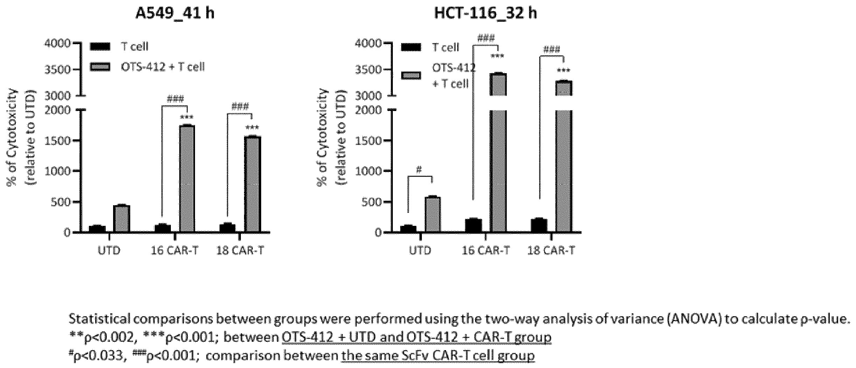

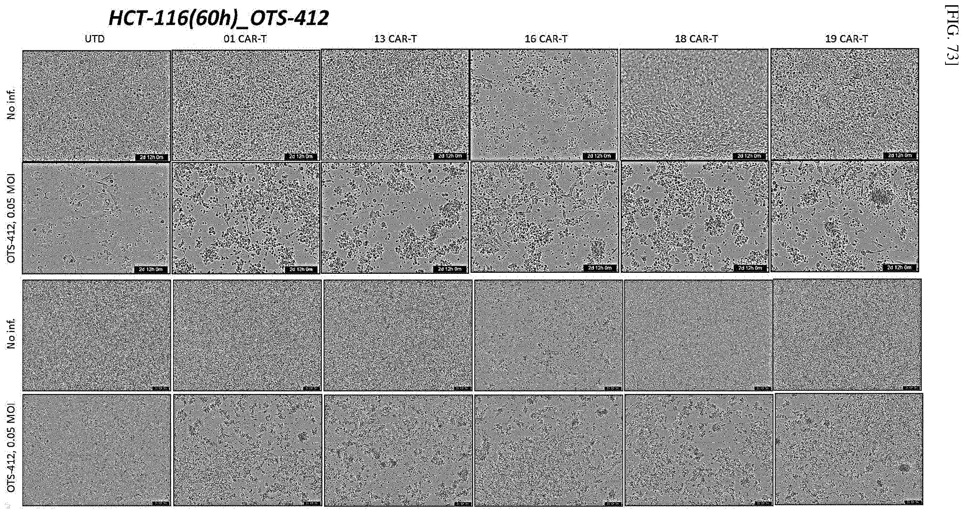

As a result, as illustrated in FIG. 73, it was identified that for the A549 and HCT-116 cell lines infected with OTS-412, more dead cells were stained in the groups, each of which was treated with the Ab16 CAR-T or Ab18 CAR-T cells, than the group treated with the UTD cells. In addition, for the A549 and HCT-116 cell lines infected with OTS-412, as compared with the group treated with the UTD cells, both groups, which were treated with the Ab16 CAR-T and Ab18 CAR-T cells, respectively, showed a statistically significant CAR-T cell-specific cytotoxic effect (FIG. 72).

### Experimental Example 11. Identification of anticancer effect of oncolytic vaccinia virus (OTS-412, WOTS-418) and CAR-T cells

Anticancer effects of CAR-T cells were analyzed by measuring cell death in real-time using a fluorescence-based cell imaging system (Incucyte^{®} Live-Cell Analysis System, Sartorius).

Specifically, cancer cell line HCT-116 (1×10⁴ cells) was seeded in 96-well-plates. Then, while performing incubation at a temperature of 37°C, the cells were subjected to infection with each of OTS-412 and WOTS-418 of Preparation Example 1.1 at an MOI of 0.05 so that the protein A56 was expressed on the cancer cell surface. After 2 hours, the medium was replaced with medium containing 2% FBS. After 4 hours, each of UTD cells, and the five types of CAR-T cells (Ab 13 CAR-T, Ab16 CAR-T, Ab18 CAR-T, Ab01 CAR-T, Ab19 CAR-T) was administered so that a ratio of T cell:cancer cell was 3:1. The T cells were administered with medium (10% FBS) containing a red fluorescent staining reagent. Then, cell death image data were acquired at 30-minute intervals for 5 days using the Incucyte system and analyzed through software.

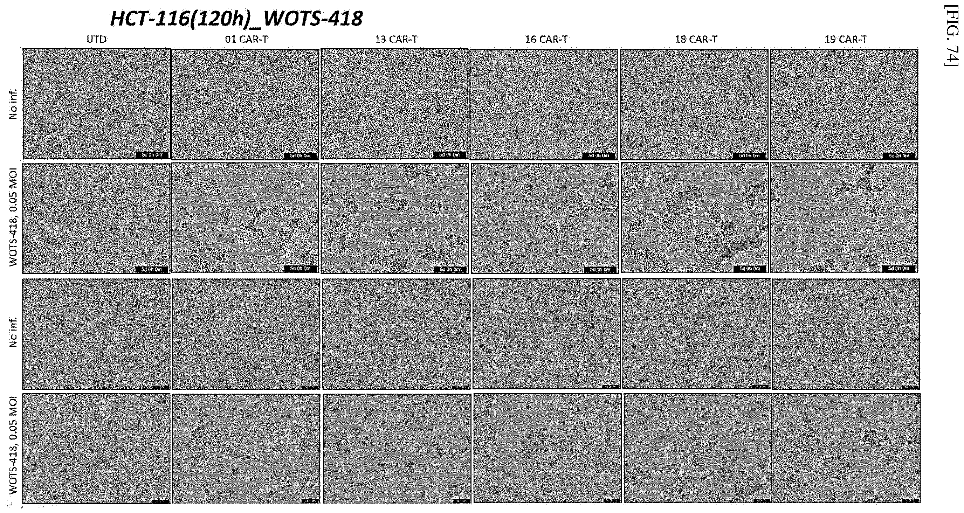

As a result, it was identified that for the HCT-116 cell line infected with OTS-412 or WOTS-418, more dead cells were stained in the groups which were treated with the five types of CAR-T cells (Ab13 CAR-T, Ab16 CAR-T, Ab18 CAR-T, Ab01 CAR-T, Ab19 CAR-T), respectively, than the group treated with the UTD cells (FIGS. 73 and 74).

## Claims

1. A binding molecule or a fragment thereof, which specifically binds to a conformational epitope of A56 or a fragment thereof, wherein the conformational epitope includes a basic or nucleophilic amino acid present in a region from amino acids at positions 60 to 63 in an amino acid sequence of protein A56 represented by SEQ ID NO: 1, and further includes:
(i) a nucleophilic amino acid present in a region from amino acids at positions 44 to 50 in the amino acid sequence of protein A56,
(ii) a nucleophilic amino acid present in a region from amino acids at positions 53 to 59 in the amino acid sequence of protein A56,
(iii) a nucleophilic amino acid present in a region from amino acids at positions 85 to 90 in the amino acid sequence of protein A56, or
(iv) a basic or nucleophilic amino acid present in a region from amino acids at positions 91 to 94 in the amino acid sequence of protein A56.

2. The binding molecule or a fragment thereof of claim 1, wherein the conformational epitope includes a basic or nucleophilic amino acid present in a region from amino acids at positions 60 to 63 in an amino acid sequence of protein A56 represented by SEQ ID NO: 1, and includes (i) a nucleophilic amino acid present in a region from amino acids at positions 44 to 50 in the amino acid sequence of protein A56 and (ii) a nucleophilic amino acid present in a region from amino acids at positions 53 to 59 in the amino acid sequence of protein A56.

3. The binding molecule or a fragment thereof of claim 1, wherein the binding molecule or a fragment thereof includes (iii) a nucleophilic amino acid present in a region from amino acids at positions 85 to 90 in the amino acid sequence of protein A56 and (iv) a basic or nucleophilic amino acid present in a region from amino acids at positions 91 to 94 in the amino acid sequence of protein A56.

4. The binding molecule or a fragment thereof of claim 1, wherein the binding molecule or a fragment thereof competes for binding to A56 with one or more of the following antibodies:
an antibody that includes a heavy chain CDR1 having the amino acid sequence of SEQ ID NO: 32, a heavy chain CDR2 having the amino acid sequence of SEQ ID NO: 33, a heavy chain CDR3 having the amino acid sequence of SEQ ID NO: 34, a light chain CDR1 having the amino acid sequence of SEQ ID NO: 35, a light chain CDR2 having the amino acid sequence of SEQ ID NO: 36, and a light chain CDR3 having the amino acid sequence of SEQ ID NO: 37;
an antibody that includes a heavy chain CDR1 having the amino acid sequence of SEQ ID NO: 38, a heavy chain CDR2 having the amino acid sequence of SEQ ID NO: 39, a heavy chain CDR3 having the amino acid sequence of SEQ ID NO: 40, a light chain CDR1 having the amino acid sequence of SEQ ID NO: 41, a light chain CDR2 having the amino acid sequence of SEQ ID NO: 42, and a light chain CDR3 having the amino acid sequence of SEQ ID NO: 43;
an antibody that includes a heavy chain CDR1 having the amino acid sequence of SEQ ID NO: 44, a heavy chain CDR2 having the amino acid sequence of SEQ ID NO: 45, a heavy chain CDR3 having the amino acid sequence of SEQ ID NO: 46, a light chain CDR1 having the amino acid sequence of SEQ ID NO: 47, a light chain CDR2 having the amino acid sequence of SEQ ID NO: 48, and a light chain CDR3 having the amino acid sequence of SEQ ID NO: 49;
an antibody that includes a heavy chain CDR1 having the amino acid sequence of SEQ ID NO: 50, a heavy chain CDR2 having the amino acid sequence of SEQ ID NO: 51, a heavy chain CDR3 having the amino acid sequence of SEQ ID NO: 52, a light chain CDR1 having the amino acid sequence of SEQ ID NO: 53, a light chain CDR2 having the amino acid sequence of SEQ ID NO: 54, and a light chain CDR3 having the amino acid sequence of SEQ ID NO: 55; and
an antibody that includes a heavy chain CDR1 having the amino acid sequence of SEQ ID NO: 56, a heavy chain CDR2 having the amino acid sequence of SEQ ID NO: 57, a heavy chain CDR3 having the amino acid sequence of SEQ ID NO: 58, a light chain CDR1 having the amino acid sequence of SEQ ID NO: 59, a light chain CDR2 having the amino acid sequence of SEQ ID NO: 60, and a light chain CDR3 having the amino acid sequence of SEQ ID NO: 61.

5. The binding molecule or a fragment thereof of claim 1, wherein the binding molecule or a fragment thereof binds to A56 with a KD value of less than about 1.0 nM as measured by surface plasmon resonance.

6. The binding molecule or a fragment thereof of any one of claims 1 to 5, wherein the binding molecule or a fragment thereof is used to decrease burden of cancer cells by being administered together with an oncolytic virus.

7. The binding molecule or a fragment thereof of claim 6, wherein the oncolytic virus is a vaccinia virus.

8. The binding molecule or a fragment thereof of claim 7, wherein the vaccinia virus is vaccinia virus Western Reserve (WR), New York vaccinia virus (NYVAC), Wyeth (The New York City Board of Health; NYCBOH), LC16m8, Lister, Copenhagen, Tian Tan, USSR, TashKent, Evans, International Health Division-J (IHD-J), or International Health Division-White (IHD-W).

9. A method for decreasing burden of cancer cells in a subject, comprising:
a step of administering, to the subject, the binding molecule or a fragment thereof of any one of claims 1 to 8.

10. The method of claim 9, wherein the cancer is any one selected from the group consisting of lung cancer, colorectal cancer, prostate cancer, thyroid cancer, breast cancer, brain cancer, head and neck cancer, esophageal cancer, skin cancer, thymus cancer, gastric cancer, colon cancer, liver cancer, ovarian cancer, uterine cancer, bladder cancer, rectal cancer, gallbladder cancer, biliary tract cancer, pancreatic cancer, lymphoma, acute leukemia, multiple myeloma, and combinations thereof.

11. A composition for preventing or treating cancer, comprising:
the binding molecule or a fragment thereof of any one of claims 1 to 8.

12. A kit for preventing or treating cancer, comprising:
the binding molecule or a fragment thereof of any one of claims 1 to 8; and
an oncolytic virus.

13. A use of the binding molecule or a fragment thereof of any one of claims 1 to 8 for the treatment of cancer.
